# EUROPEAN PATENT APPLICATION

(11) **EP 4 649 923 A1**
(43) Date of publication of application: **19.11.2025**
(21) Application number: 25176526.9
(22) Date of filing: 14.05.2025
(51) Int. Cl.: A61F 2/40, A61F 2/46

(54) **INFLATABLE IMPLANT FIXATION SYSTEMS AND METHODS**

(30) Priority: 14.05.2024 US 202463647348 P; 31.10.2024 US 202463714331 P
(71) Applicant: Stryker Corporation, Portage, MI 49002-9711 (US)
(72) Inventor: FLOW, James R., 49002 Portage Michigan (US); ELMALEK, Omar, 49002 Portage Michigan (US); MEIRAZ, Yoav, 49002 Portage Michigan (US); GROETHE, Jacob, 49002 Portage Michigan (US)
(74) Representative: V.O.

(57) **Abstract**

A method of delivering a balloon implant (140) into a patient includes slidably coupling one or more filaments (112) coupled to tissue in the patient to the balloon implant while the balloon implant is outside the patient, advancing the balloon implant along the one or more filaments into the patient, and securing the balloon implant to the tissue.

## Description

### CROSS-REFERENCE TO RELATED PATENT APPLICATIONS

This application claims the benefit of and priority to US Provisional Patent Application No. 63/647,348, filed May 14, 2024, and US Provisional Patent Application No. 63/714,331, filed October 31, 2024, both of which are incorporated by reference herein in their entireties. In addition, this application is related to US Patent Application No. 18/663,772, filed May 14, 2024, and entitled "Inflatable Prosthesis, Delivery Tools Therefor, Implantation Method Therefor, And Manufacturing Method Therefor," which is incorporated by reference herein in its entirety.

### BACKGROUND

Through repeated motion, particularly that incurred through regular physical activity, sensitive soft tissues may suffer wear and tear injuries due to repeated rubbing against one another and/or hard tissues, such as bone. Tears of rotator cuff tendons and articular capsule disintegration are examples of this type of injury. Tears of rotator cuff tendons and articular capsule disintegration may not only result from chronic deterioration but can also be caused or accelerated by acute injuries. In addition, these tissues may be adversely affected by inflammation, infection, disease and/or genetic predispositions which lead to degeneration of these tissues.

Severe or complete tears and deterioration of bodily joints, related tissues, such as tendons, ligaments, capsules, cartilage and bony parts, and other body parts, such as bursae, synovium and other membranes, may cause severe pain, hindered movement up to complete disability, joint parts dislocation, and possibly other physical impairments.

Some torn or otherwise deteriorated joints may be treated by filling voids and spaces between tissues with volumetric fillers, especially in scenarios where there is a need to create or revive unhindered relative motion between such tissues. Such volumetric fillers should be configured to, among other functions, occupy a three-dimensional volume of space between the tissues to provide the desired performance. Further, satisfactory performance of a volumetric filler also depends on the volumetric filler remaining in an intended implantation location between tissues in the joint region under repair.

Accordingly, it would be desirable to provide a repair for torn or deteriorated tissue in or near a joint that relies on an implant being placed between tissue at a desired location within a body such that it may be expected to remain at such location over time without becoming displaced in a manner that would diminish the effectiveness of the repair.

### SUMMARY OF THE INVENTION

The present disclosure provides improvements in the placement of balloon implants in torn or deteriorated joints to predictably restore function in such joints for an extended time period. In some aspects, a balloon is delivered to an implantation site within a joint region of a patient and secured to tissue of the patient so that the balloon remains in its initially implanted location at the implantation site.

In a first aspect, the present disclosure relates to an implant configured to be implanted into a joint of a patient to restore function in the joint. In a first example of the first aspect, an implant includes a balloon and a fitting. The balloon may be biocompatible and includes a body and an inlet, the inlet being in fluid communication with an internal cavity of the body. The fitting is attached to the balloon and has a lumen therethrough. A central longitudinal axis of the lumen is aligned with a central longitudinal axis of the inlet, and the fitting includes a connection portion configured to receive a filament. When the balloon is in a deployed configuration in a patient, the biocompatible balloon is fixable to a tissue of the patient by receipt of the filament on the connection portion and attachment of the filament to the tissue.

In a second example of the first aspect, the connection portion may include an opening offset from the central longitudinal axis of the lumen, and the opening is configured to receive the filament. In a third example, the connection portion may be a lateral projection extending from a portion of the fitting defining the lumen. In a fourth example, the connection portion may be a projection extending from the fitting at an angle transverse to the central longitudinal axis of the lumen, the projection having an opening therethrough such that a central longitudinal axis through the opening is at a non-zero angle relative to the central longitudinal axis of the lumen.

In a fifth example of the first aspect, the connection portion may be an outer clip that is slidably attached to a central portion of the fitting, the outer clip having a shape that partially encloses the central portion when attached to the central portion. In a sixth example, an opening may extend through the outer clip of the fifth example and may be configured to receive the filament. The opening may be offset from the central longitudinal axis of the lumen of the fitting. In a seventh example, the connection portion may be a cable tie configured to enclose a perimeter of a central portion of the fitting. In this arrangement, the opening may extend through the cable tie to allow the filament to pass therethrough when the cable tie encloses the perimeter of the central portion. In an eighth example, the central portion of the fitting includes a first circumferential projection and a second circumferential projection spaced apart from the first circumferential projection. And, the cable tie of the eighth example may be configured to be received between the respective first and second circumferential projections. One or both of the circumferential projections may have a ring-type shape.

In a ninth example of the first aspect, the fitting may include a first circumferential projection and a second circumferential projection spaced apart from the first circumferential projection. The connection portion of this example may be defined by a recessed region in between the first and second circumferential projections. In a tenth example, when the biocompatible balloon is in the deployed configuration in the patient, the balloon is fixable to the tissue of the patient by attaching the filament to the tissue with an anchor. In an eleventh example, the balloon may include a conduit and the fitting may be attached to the conduit so that the central longitudinal axis of the lumen is aligned with a central longitudinal axis of the conduit. In a twelfth example, the fitting may abut the conduit and the respective central longitudinal axes of the lumen and the conduit may be coincident. In a thirteenth example, the connection portion of the fitting may be a narrow central portion that flares outward toward opposite ends of the fitting.

In another example of the first aspect, an implant may include an inflatable balloon, a fitting, an anchor and a filament. The inflatable balloon is configured for implantation in a patient and includes an inflatable body. The fitting is attached to the inflatable balloon and includes an opening spaced apart from a lumen of the fitting. The anchor is configured to be secured to a tissue in the patient and the filament includes a first end portion configured for attachment to the anchor and a second end portion configured to pass through the opening and attach to the fitting. In another example of the first aspect, the opening of the fitting may be on a projection of the fitting extending transversely from a central body of the fitting, the central body defining the lumen of the fitting.

In a second aspect, the present disclosure relates to a system for repairing tissue in a patient. In a first example of the second aspect, an implant system includes an implant and an implant delivery instrument. The implant includes a balloon and a fitting attached to the balloon, the fitting including an outer part and an inner part. The implant delivery instrument includes a shaft and a sheath slidably disposed over the shaft. Further, the implant is configured to be received on a distal portion of the implant delivery instrument and the implant system is adjustable between a pre-deployment configuration and a deployment configuration. In the pre-deployment configuration, the implant is within the sheath, while in the deployment configuration, the implant is external to the sheath. The outer part of the fitting is slidable along the shaft from a location proximal to the sheath, through the sheath and onto the inner part while the system is in the pre-deployment configuration. The fitting is configured to receive a filament configured to secure the implant to tissue.

In a second example of the second aspect, the outer part of the fitting may include an opening therethrough, the opening being configured to receive the filament. In a third example, the implant system may include a supplemental guide attachable to the shaft of the implant delivery instrument, the supplemental guide configured to push the outer part of the fitting onto the inner part of the fitting.

In another example of the second aspect, a system for repairing tissue in a patient includes an implant delivery instrument and an implant attachable to the implant delivery instrument. The implant delivery instrument includes a shaft and a sheath slidably disposed over the shaft, where the implant is attachable to a distal end of the shaft. The implant includes an expandable body, a conduit attached to the expandable body and a sleeve attached to the conduit. The sleeve includes a portion configured to receive a filament, such as a suture. And, the portion of the sleeve is configured to receive the filament while the portion of the sleeve is within the sheath. Further, when the implant is attached to the distal end of the shaft, the implant delivery instrument is adjustable between a pre-deployment configuration and a deployment configuration, the implant being within the sheath and the portion of the sleeve configured to receive the filament in the pre-deployment configuration and being external to the sheath in the deployment configuration.

In a third aspect, the present disclosure relates to a method of delivering an implant into a patient to repair a joint. In a first example of the third aspect, a method of delivering a balloon implant into a patient using an implant delivery instrument includes: advancing the implant delivery instrument into the patient with the balloon implant attached to the implant delivery instrument, a first end of a filament passing through an opening in a peripheral portion of the balloon implant, the implant delivery instrument including: a shaft and a sheath slidably disposed over the shaft and the balloon implant; causing the sheath to be retracted toward a proximal end of the implant delivery instrument such that the balloon implant is exposed from the sheath, wherein when the sheath is retracted, at least a portion of the sheath passes over the peripheral portion with the filament loaded therethrough as the sheath moves toward the proximal end of the implant delivery instrument; releasing the balloon implant from the implant delivery instrument; and affixing the filament to the peripheral portion of the balloon implant.

In a second example of the third aspect, the method may include loading the first end of the filament through the opening in the peripheral portion of the balloon implant prior to advancing the implant delivery instrument into the patient. In a third example, loading the first end of the filament may include passing the filament through the opening in the peripheral portion where the peripheral portion is a fitting attached to an inflatable body of the balloon implant. In a fourth example, a portion of the filament may be positioned through an elongate slit of the sheath that extends along a portion of a length of the sheath as the sheath is retracted toward the proximal end of the implant delivery instrument, the position of the portion of the filament being partially within the sheath and partially outside of the sheath. In a fifth example, the method may include attaching a guide to the shaft and using the guide to push a slidable part of the peripheral portion along the shaft and onto an inner part of the peripheral portion prior to causing the sheath to be retracted, the inner part being adjacent to an inlet of the balloon implant that is in fluid communication with an inflatable portion of the balloon implant.

In a sixth example of the third aspect, the filament is a first filament, the opening is a first opening, and the peripheral portion is a first peripheral portion, and the method includes loading a second filament through a second opening in a second peripheral portion of the balloon implant, the second peripheral portion being separate from the first peripheral portion. In a seventh example, the first peripheral portion and the second peripheral portion of the sixth example may be on opposite ends of a length of the balloon implant and may be centrally disposed such that each is aligned with an inlet of the balloon implant in fluid communication with an internal cavity of the balloon implant. In an eighth example, the first peripheral portion may be on a first lateral side of the balloon implant and the second peripheral portion may be on a second lateral side of the balloon implant opposite the first lateral side. In a ninth example, the first peripheral portion and the second peripheral portion may be on a first side of the balloon implant. In a tenth example, the filament may include a second end opposite the first end, the second end being attached to a tissue in the patient. In an eleventh example, the second end of the filament may be attached to the tissue via an anchor.

In another example of the third aspect, a method of delivering a balloon implant into a patient includes the following steps: delivering a tissue augment to a tissue location within the patient; securing the tissue augment to tissue when the tissue augment is at the tissue location; and delivering a balloon implant to the tissue location such that movement of the balloon implant is restrained by a position of the balloon implant with respect to the tissue augment. In another example, the balloon implant may be restrained by a pair of filaments, each filament including ends attached to the tissue augment.

In a fourth aspect, the present disclosure relates to a method of delivering an implant into a patient. In a first example of the fourth aspect, the method includes: delivering an implant to a tissue location within the patient, the implant including a tissue augment and a balloon; and securing the tissue augment to tissue when the tissue augment is at the tissue location. In some examples, the method may also include operatively engaging the balloon to the tissue augment. In some examples, operatively engaging the balloon implant to the tissue augment may occur before delivering the tissue augment to the tissue location. In some examples, delivering the implant to the tissue location may include utilizing an opening in the implant to guide the implant to the tissue location. In a subset of these examples, the method may include delivering the implant to the tissue location by guiding the implant along a filament passing through the opening, the opening being defined by the tissue augment and the filament being attached to a fixation anchor on a tissue of the patient. In a subset of these examples, delivering the implant to the tissue location may include guiding the implant along a filament passing through the opening, the opening being defined by the balloon and the filament being attached to a fixation anchor on a tissue of the patient.

In another aspect, the present disclosure relates to a method of delivering a balloon implant into a patient, the method including slidably coupling one or more filaments coupled to tissue in the patient to the balloon implant while the balloon implant is outside the patient, advancing the balloon implant along the one or more filaments into the patient, and securing the balloon implant to the tissue. In some examples, the balloon implant is advanced into the patient while in a collapsed configuration using an implant delivery instrument and transitions from the collapsed configuration to an expanded configuration in the patient. In some examples, the implant delivery instrument includes a sheath, wherein the balloon implant is positioned in the sheath when the balloon implant is advanced into the patient. In some examples, the method further includes retracting the sheath to release the balloon implant from the sheath inside the patient, releasing the balloon implant causing the balloon implant to transition from the collapsed configuration to the expanded configuration. In some examples, the method further includes inflating a balloon of the balloon implant inside the patient using the implant delivery instrument.

In some examples, securing the balloon implant to the tissue includes at least one of tying one of the one or more filaments to the balloon implant, tying one of the one or more filaments to an anchor coupled to the tissue, tying a first filament of the one or more filaments to a second filament of the one or more filaments, tying the first filament to a third filament coupled to the anchor, tying a first end of the first filament to a second end of the first filament, the first filament passing through the anchor, tying a first end of a fourth filament to at least one of an anchor, the balloon implant, a fifth filament, or a second end of the fourth filament, or pressing a fixation element through material of the balloon implant.

In some examples, securing the balloon implant to the tissue includes at least one of securing a first filament of the one or more filaments to a second filament using a knotless fixation feature, securing the first filament to an anchor coupled to the tissue using a knotless fixation feature, securing a third filament to the implant using a knotless fixation feature, or securing a first portion of the first filament to a second portion of the first filament using a knotless fixation feature, the first filament passing through the anchor.

In some examples, each of the one or more filaments is coupled to the tissue by one of a plurality of anchors, wherein the anchors have been coupled together during a previously completed joint repair by the one or more filaments or by one or more other filaments, wherein the balloon implant is secured to the tissue at a location of the previously completed joint repair.

In some examples, slidably coupling the one or more filaments to the balloon implant includes passing a first end of a first filament of the one or more filaments through a first opening in the balloon implant. In some examples, the method further includes passing the first end of the first filament through a second opening in the balloon implant. In some examples, the first opening extends through a peripheral portion of the balloon implant.

In some examples, the balloon implant includes one or more tabs forming peripheral portions of the balloon implant, and wherein slidably coupling the one or more filaments to the balloon implant includes passing a first end of each of the one or more filaments directly through material of one of the one or more tabs.

In some examples, the method further includes tensioning the one or more filaments prior to securing the balloon implant to the tissue.

In some examples, after securing the balloon implant to the tissue using the one or more filaments, the balloon implant is free to move between 2.0 millimeters and 10.0 millimeters in a lateral direction relative to the tissue.

In some examples, a first filament of the one or more filaments is coupled to a first anchor coupled to the tissue and is slidably coupled to the balloon implant in a first two locations, wherein securing the balloon implant to the tissue includes securing the first filament to a second anchor coupled to the tissue. In some examples, a second filament of the one or more filaments is coupled to a third anchor coupled to the tissue and is slidably coupled to the balloon implant in a second two locations, wherein securing the balloon implant to the tissue includes securing the second filament to a fourth anchor coupled to the tissue.

In another aspect, the present disclosure relates to an implant system including an implant including a balloon including a body and an inlet being in fluid communication with an internal cavity of the body, a fitting attached to the inlet of the balloon and configured to receive a fluid for inflating the body of the balloon, and a connection feature configured to receive a filament therethrough, and an implant delivery instrument configured to receive the implant in a collapsed configuration. In some examples, the implant delivery instrument includes a sheath configured to receive the implant in the collapsed configuration and a shaft configured to be coupled to the fitting, the shaft including a conduit configured to provide the fluid for inflating the body of the balloon. In some examples, the sheath is retractable relative to the shaft to release the implant from the sheath. In some examples, the sheath includes a slot configured to allow a filament coupled to the connection feature extend out of a side of the sheath, the slot extending to a distal end of the sheath. In some examples, the slot includes a widened portion configured to be positioned adjacent to the connection feature when the conduit interfaces with the fitting.

In some examples, the connection feature includes an opening extending through a portion of the fitting.

In some examples, the connection feature includes an opening extending through a peripheral portion of the implant.

### BRIEF DESCRIPTION OF THE DRAWINGS

A more complete appreciation of the subject matter of the present disclosure and of the various advantages thereof can be realized by reference to the following detailed description in which reference is made to the accompanying drawings in which:
FIG. 1 is a perspective view of an implant system according to one aspect of the present disclosure
FIG. 2 is a cross-sectional view of the implant system of FIG. 1;
FIG. 3 is a cross-sectional view of the implant system of FIG. 1;
FIG. 4 is a close-up partial view of an implant system according to one aspect of the present disclosure;
FIG. 5 is a close-up partial perspective view of the implant system of FIG. 4;
FIG. 6 is a perspective view of the implant system of FIG. 4;
FIG. 7 is a perspective view of an implant of the implant system of FIG. 4;
FIGS. 8-12 are perspective partial views of steps in a method of deploying an implant of an implant system according to one aspect of the present disclosure;
FIG. 13 is a perspective view of a component of an implant according to one aspect of the present disclosure;
FIG. 14 is a perspective view of a component of the implant of FIG. 13;
FIG. 15 is a perspective view of the implant of FIG. 13;
FIG. 16 is a top view of an implant according to one aspect of the present disclosure;
FIGS. 17A-B are top views of implants according to aspects of the present disclosure;
FIG. 18 is a top view of an implant according to one aspect of the present disclosure;
FIG. 19A is a partial perspective view of a step in a method of using an implant system according to one aspect of the present disclosure;
FIG. 19B is a partial perspective view of a step in a method of using an implant system according to one aspect of the present disclosure;
FIGS. 19C-19I illustrate a method of securing an implant using the implant system of FIG. 19B;
FIGS. 19J-19P illustrate a method of securing an implant using the implant system of FIG. 19B;
FIGS. 20A-20B are top views of steps in the method of using the implant system of FIG. 19A;
FIGS. 21-24 are top views of steps in a method of using an implant according to one aspect of the present disclosure;
FIGS. 25A-25D are top views of steps in a method of using an implant according to one aspect of the present disclosure;
FIGS. 25E is a top view illustrating a method of securing an implant according to one aspect of the present disclosure;
FIG. 26A is a perspective view of an implant according to one aspect of the present disclosure;
FIG. 26B is a top view of the implant of FIG. 26A;
FIGS. 27-29 are top views of steps in a method of using an implant according to one aspect of the present disclosure;
FIGS. 30A-B are respective end and top views of steps in a method of using an implant according to one aspect of the present disclosure;
FIG. 31A is a top view of an implant according to one aspect of the present disclosure;
FIG. 31B is a side view of a tab of the implant of FIG. 31A, according to one aspect of the present disclosure;
FIG. 32A is a top view of an implant according to one aspect of the present disclosure;
FIG. 32B is a side view of a tab of the implant of FIG. 32A, according to one aspect of the present disclosure;
FIGS. 33-34 are top views of steps in a method of using an implant according to one aspect of the present disclosure;
FIGS. 35A-35C are side views of steps in the method of the aspect shown in FIGS. 33-34;
FIGS. 36A-36D are top views of steps in a method of using the implant of FIGS 33-34, according to one aspect of the present disclosure;
FIGS. 37A-37B are top view of implants according to aspects of the present disclosure;
FIGS. 38A-38B are top views of steps in a method of using an implant according to one aspect of the present disclosure;
FIGS. 39-41 are top views of steps in a method of using an implant according to one aspect of the present disclosure;
FIG. 42 is a perspective view of an implant according to one aspect of the present disclosure;
FIGS. 43-46 are top views of steps in a method of using an implant according to one aspect of the present disclosure;
FIGS. 47-50 are top views of steps in a method of using an implant according to one aspect of the present disclosure;
FIGS. 51-52 are top views of steps in a method of using an implant system according to an aspect of the disclosure;
FIGS. 53-54 are top views of steps in a method of using an implant system according to an aspect of the disclosure;
FIG. 55 is a top view of a step in a method of using an implant system according to an aspect of the disclosure; and
FIG. 56 is a top view of a step in a method of using an implant system according to an aspect of the disclosure.
FIG. 57 is a side view of an implant according to one aspect of the present disclosure;
FIG. 58 is a perspective view of an augment according to one aspect of the present disclosure;
FIG. 59 is a perspective view of an augment according to one aspect of the present disclosure;
FIG. 60 is a perspective view of an augment according to one aspect of the present disclosure;
FIG. 61A is a side view of an implant according to one aspect of the present disclosure;
FIG. 61B is a side view of an implant according to one aspect of the present disclosure;
FIG. 61C is a top view of the implant of FIG. 61A according to one aspect of the present disclosure;
FIG. 61D is a top view of an implant according to one aspect of the present disclosure;
FIG. 62A is a perspective view of an implant according to one aspect of the present disclosure;
FIG. 62B is a side view of the implant of FIG. 62A;
FIG. 62C is a perspective view of an implant according to one aspect of the present disclosure;
FIG. 62D is a side view of the implant of FIG. 62C;
FIGS. 62E-62F are top views of steps in a method of using an implant according to one aspect of the present disclosure; and
FIGS. 63-67 are flowcharts illustrating methods of delivering implants to an implantation site according to various aspects of the present disclosure.

### DETAILED DESCRIPTION

As used herein, the term "distal" refers to that portion of an instrument or component thereof which is farther from the user while the term "proximal" refers to that portion of the instrument or component thereof which is closer to the user. The term "medial" means closer to or toward the midline of the body, and the term "lateral" means further from or away from the midline of the body. The term "inferior" means closer to or toward the feet, and the term "superior" means closer to or toward the crown of the head. As used herein, the terms "about," "approximately," "generally," and "substantially" are intended to mean that slight deviations from absolute are included within the scope of the term so modified.

In one aspect, the present disclosure relates to an implant for use in restoring anatomic function in a joint space of a patient. Throughout the present disclosure, examples of implants may include balloon implants. In one application, the implant may be configured for placement in a shoulder joint. In some specific examples, the implant may be configured for placement in a subacromial space in the shoulder joint. In other examples, the implant may be configured for placement under the rotator cuff. It should be appreciated, however, that the present disclosure is not limited to shoulder applications and may be used between any two or more tissues in a mammalian body where the placement would function to restore anatomic function in a joint space. In some examples, the implant includes an augment that may function to promote healing in the joint space.

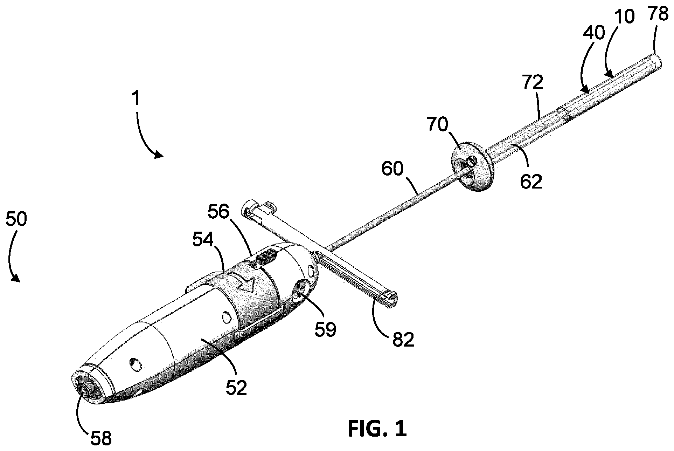

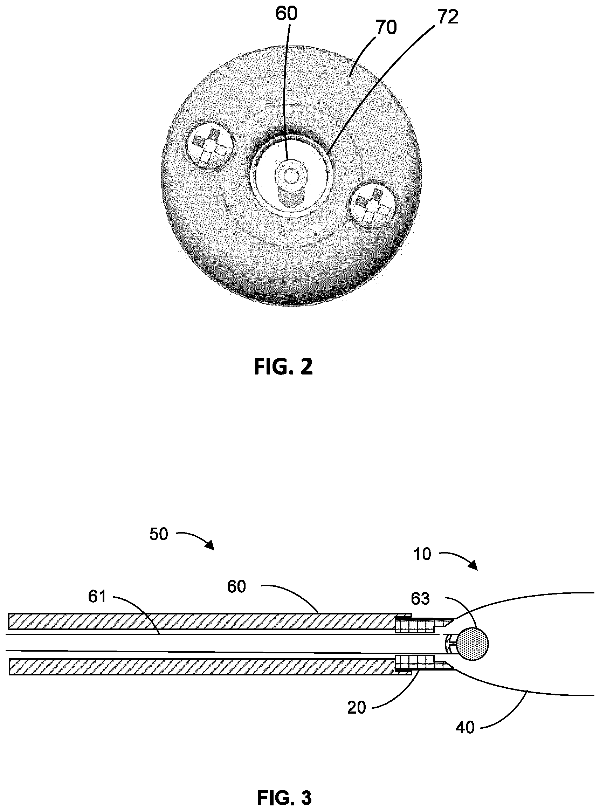

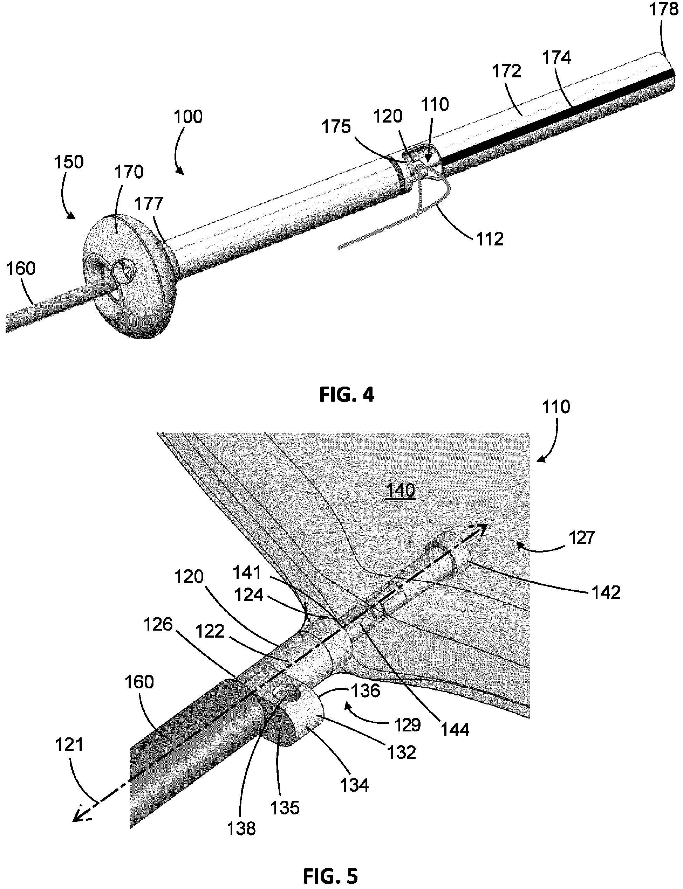

In one aspect, an implant 110 is as shown in FIGS. 5-7, and in part in FIG. 4. The implant 110 may be positionable at an implantation site between tissues, for example, in a joint (e.g., a shoulder joint) in part using an implant delivery instrument 50 as shown in FIGS. 1-3 or implant delivery instrument 150 shown in FIGS. 3-5. The implant delivery instruments 50, 150 are described in further detail below. Implant 110 includes a fitting 120 and a balloon 140. The balloon 140 includes a body defining an internal cavity 127 and an inlet 129 allowing fluid communication between the outside of balloon 140 and the internal cavity.

Implant 110 may also include, and in the depicted example includes, a conduit 144 operatively connected to the balloon 140. Conduit 144 may be configured to receive fitting 120, as shown in FIG. 5, and provide the inlet 129 to deliver fluid or gas to the inner cavity of balloon 140 via the fitting 120 to fill the balloon 140. Thus, the fitting 120 may be attached to the inlet 129 of the balloon 140 and configured to receive a fluid for inflating the body of the balloon 140. In some examples, the conduit 144 may be a component of the fitting 120. The fluid may be or may include, for example, water, saline solution, and/or a therapeutic fluid such as hyaluronic acid, platelet-rich plasma, or bone marrow aspirate concentrate. The gas may be, for example, carbon dioxide or air. The fluid or gas may be provided by a pump fluidly coupled to the fitting 120 through the conduit 144 into to the balloon 140. In some examples, conduit 144 may also include a valve for sealing an internal volume of balloon 140. In other examples, an implant delivery instrument may be used to seal the internal volume of balloon 140, as described elsewhere in the present application. Further, when employed in a surgical procedure, implant 110 may also include a filament 112 that is attached to fitting 120, as shown in FIG. 3. In some examples, the filament 112 may be a suture. It should be appreciated that references to a filament in any one of the aspects of the present disclosure may also be in reference to a strand or a suture comprised of a plurality of filaments. Further, it should also be appreciated that while implant 110 is depicted with a filament 112, such filament need not be provided with the implant 110. Put another way, implant 110 may be provided without a filament 112.

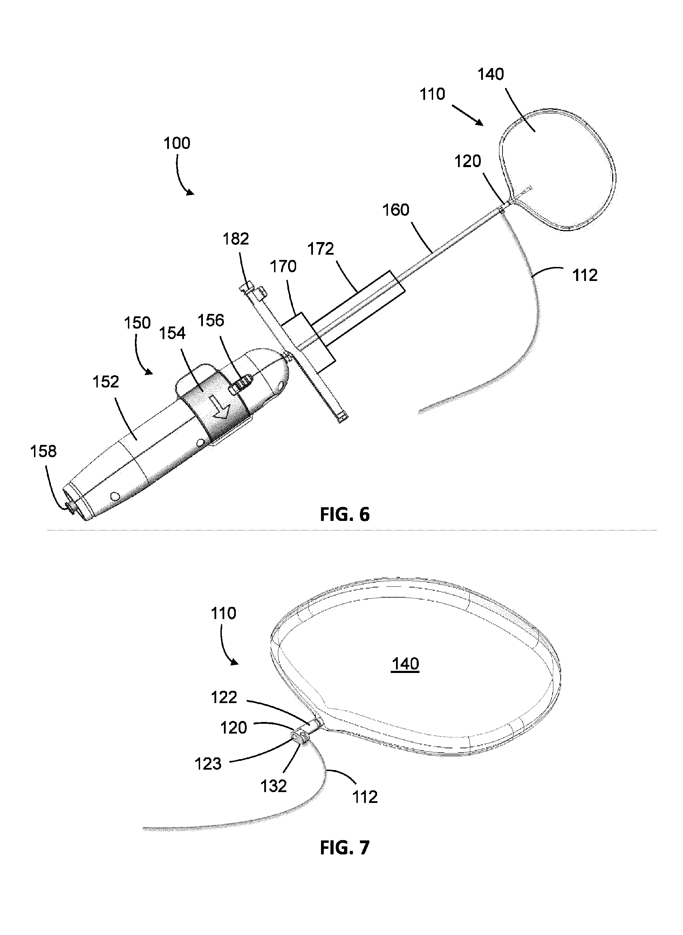

Balloon 140 of implant 110 may be a biocompatible balloon adapted for placement in a body of a patient in surgery, including within or proximate to a joint in the body. Balloon 140 has a body that is inflatable and is configured for use such that it is inflatable within the patient. Balloon 140 may also be configured to receive fluids in some applications. In some examples, and as shown in FIGS. 6-7, balloon 140 may be adapted to reach, at a maximal or over a predetermined partial inflation volume, a disc-like shape. As shown, the disc-like shape generally includes two at least partially parallel and substantially flat, oval surfaces, which may be separated from each other by a relatively small width, and a peripheral, optionally rounded, wall connecting the surfaces while allowing a single inlet for inflation and deflation. In some variations, at least one of the flat oval surfaces is smooth enough to allow or facilitate a continuous unhindered sliding of a tissue over the balloon surface, e.g., where the tissue may be a ligament, a tendon, a cartilage, or a bone. While FIGS. 6-7 illustrate a particular example of a balloon 140 of the present disclosure, it will be appreciated that this disclosure contemplates a balloon 140 in any shape adapted to be received within a joint space. For example, in some examples, the balloon 140 may be circular, square, rectangular, triangular, or any other simple or complex shape. Further, in some examples, the opposing sides of the balloon 140 may not be parallel or substantially flat. In some examples, the balloon 140 may not include peripheral walls as shown in FIGS. 6-7, and the opposing surfaces (e.g., the oval surfaces in FIGS. 6-7) may be directly coupled together to form the entirety or substantially the entirety of the periphery of the balloon 140. The corners of the balloon 140 may be rounded as shown in FIGS. 6-7 or may be substantially squared. As discussed above, it should be noted that the present disclosure is not limited to shoulder applications and may be used between any two or more tissues in a mammalian body where the placement would function to promote healing and/or restore anatomic function in a joint space. Thus, the size, shape, fluid pressure, etc. of the balloon 140 may vary based on for example, the size and species of the mammal, the particular joint, and the type and severity of injury to the joint, among other considerations. In some variations, balloon 140 is manufactured as a single piece, optionally seamless. In some variations, balloon 140 consists essentially of a polymeric material, which may be a degradable (e.g., biodegradable) material or non-degradable material, and may be a homogeneous material. In some examples, the balloon 140 may be made of or may include materials such as polycaprolactone, polyglycolide, polyhydroxybutyrate, plastarch material, polyetheretherketone, zein, polylactic acid, polydioxanone, poly(lactic-co-glycolic acid), poly(lactic acid-co-epsilon caprolactone), poly(l-lactide-co-ε-caprolactone), collagen, and/or methyl cellulose. Filaments 112 may be made of a similar (or different) polymeric material, which may be a degradable (e.g., biodegradable) material or a non-degradable material. For example, a balloon 140 and the filaments 112 that secure balloon 140 to an implantation site may be made from the same or similar biodegradable materials, such that the balloon 140 and filament 112 degrade at substantially the same rate.

Fitting 120 of implant 110 includes central body 122 and connection portion 132 attached to central body 122. In some examples, the fitting 120 further includes the conduit 144, which may be integrally or monolithically formed with or coupled (e.g., fixedly or detachably) to the central body 122. Central body 122 may have a convex curved outer surface, and in some examples may be cylindrical as shown in FIG. 5. Central body 122 is cannulated with a lumen 123 extending therethrough, the lumen 123 being aligned with a length dimension of central body 122, as shown in FIG. 7. Connection portion 132 may be formed monolithically with central body 122 or may be physically attached to central body 122 through means known to persons of ordinary skill, such as welding (e.g., ultrasonic welding), mechanical coupling (e.g., screws, pins, rivets, bolts, press fitting, a threaded connection, etc.), solvent bonding, an adhesive, etc. Connection portion 132 extends outward away from a central longitudinal axis 121 of central body 122, as shown in FIG. 5. In one example, and as depicted, connection portion 132 extends orthogonally relative to the central longitudinal axis 121 of central body 122. Connection portion 132 includes a first surface 134, second surface 135 and a third surface 136, the first surface 134 separating the second and third surfaces 135, 136. A portion of first surface 134 remote from central body 122 may be curved to reduce the number of edges on connection portion 132, and in this way, minimize any potential risk that tissue may be torn with the use of fitting 120. As depicted, such curved surface is a convex surface that wraps around a free end of connection portion 132 to bridge opposing surface portions of first surface 134. Connection portion 132 also includes opening 138 therethrough that extends between opposing sides of first surface 134, as shown in FIG. 5. The opening 138 may be configured to receive a filament therethrough. The filament may be used to manipulate balloon 140, for example, to guide the implant 110 into a patient to an implantation site and secure the fitting 120 to tissue at the implantation site. The fitting 120 may be made from or may include a biodegradable material or biodegradable materials. In some examples, the fitting 120 and the balloon 140 may be made from or may include the same biodegradable material (e.g., the biodegradable materials listed above). In other examples, the fitting 120 and the balloon 140 may be made of from or may include different non-degradable or degradable (e.g., biodegradable) materials.

Continuing with FIG. 5, assembled implant 110 includes balloon 140 with a conduit 144 defining an interior inlet 142 into the balloon 140 and an exterior inlet 141 at a periphery of balloon 140. Fitting 120 is attached to the exterior inlet 141 via a leading end 124 of central body 122. In other examples, fitting 120 may be arranged so that trailing end 126 is attached to the exterior inlet 141 of conduit 144. In some examples, fitting 120 may be attached to the inlet of balloon 140 via an adhesive. As discussed above, according to other aspects, the fitting 120 and the conduit 144 may be a single integral component. Implant 110 may also include a filament 112 secured thereto, as shown in FIGS. 6-7. Filament 112 may be used to secure implant 110 to a tissue within a patient. Securement of the filament 112 to a tissue of the patient may be accomplished through various non-limiting means. For example, an anchor (not shown) may be disposed proximate a joint to be repaired within the patient, and the filament 112 may be attached to the anchor. In other examples, the filament 112 may be secured directly to the tissue. In further examples, other fixation approaches may be used. It should further be appreciated that references to anchors within tissue throughout the present disclosure may be substituted with other fixation means in variations of such described aspects.

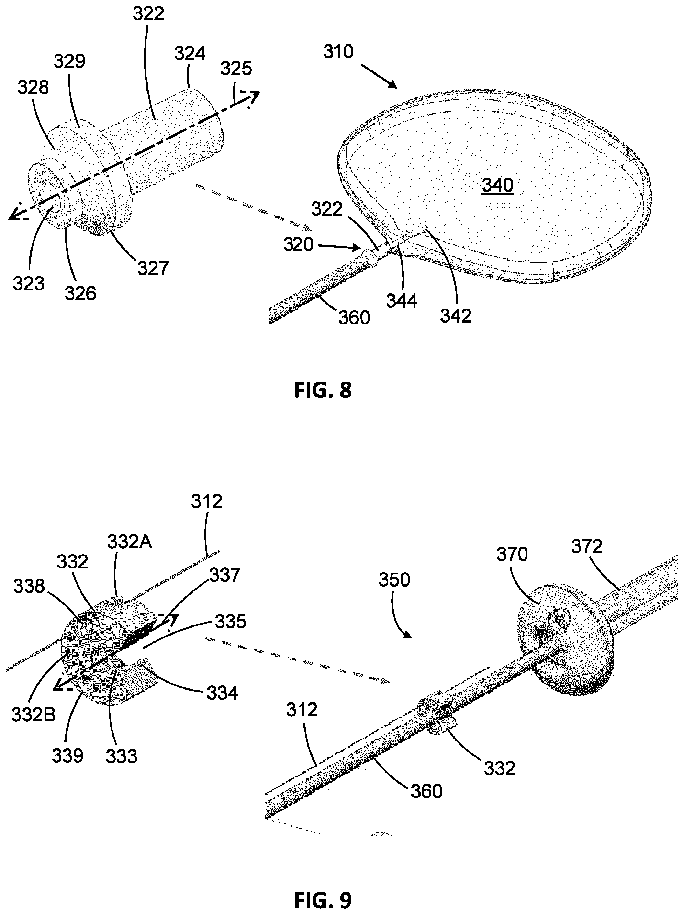

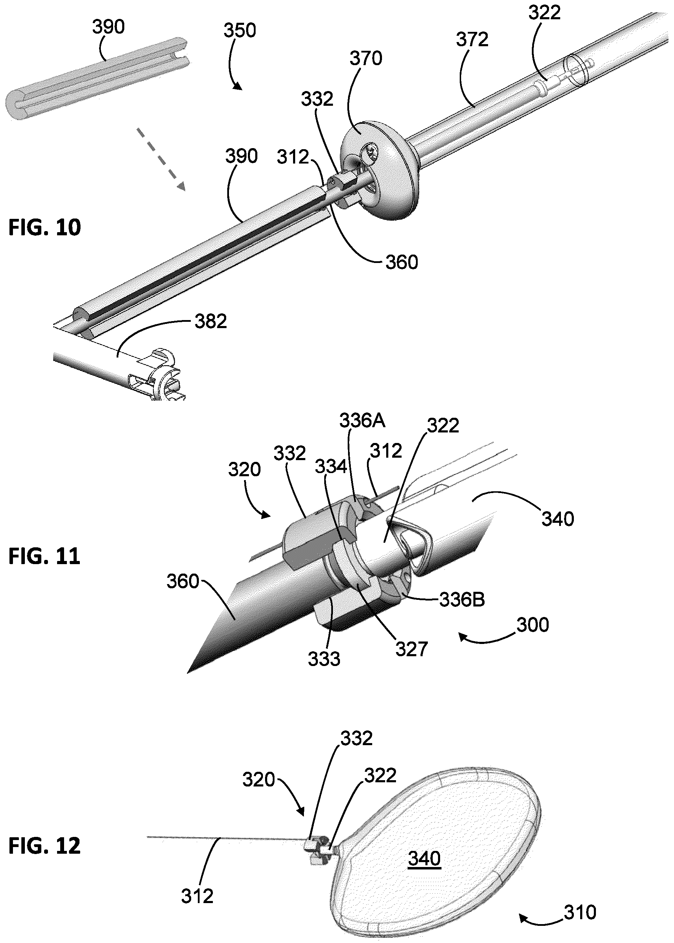

In one aspect, components of an implant 310 are shown in FIGS. 8-10. An example of implant 310 in an assembled state is shown in FIGS. 11 and 12. Unless otherwise indicated, like reference numerals refer to like elements of implant 110 shown in FIGS. 5-7, but within the 300-series of numerals. Implant 310 includes a balloon 340 and may include an inner body 322 and an outer body 332. Like balloon 340, inner body 322 and outer body 332 may be may of a degradable (e.g., biodegradable) material that degrades over time. In some examples, inner body 322 and/or outer body 332 may be considered part of an implant delivery system. Implant 310 may also receive a filament 312, for example, through an opening 338 in outer body 332 and/or wrapped around inner body 322. Implant 310 may be provided without a filament 312. Inner body 322 is attached to a conduit 344 of balloon 340 as shown in FIG. 8. In other examples, inner body 322 may be attached directly to an inlet of balloon 340. Outer body 332 is slidably attached onto inner body 322, as shown in FIG. 12. The process of slidable attachment of these components is described in greater detail elsewhere in the present application.

Turning to the details of the components of implant 310, inner body 322, shown in isolation in FIG. 8, includes a lumen 323 therethrough, the lumen 323 aligned with a central longitudinal axis 325 of inner body 322 extending from leading end 324 to trailing end 326. As depicted, inner body 322 has a generally cylindrical shape such that a cross-sectional shape of inner body 322 is circular. In alternative examples, a shape of inner body may vary from that shown. Spaced apart from trailing end 326, while still proximate trailing end 326, inner body 322 includes a circumferential projection 327 that extends radially outward from a base outer surface of inner body 322. Circumferential projection 327 includes a ridge surface 329 having a generally uniform radius around a circumference of inner body 322. On a side of ridge surface 329 closest to leading end 324, circumferential projection 327 may have a flat surface (not shown) perpendicular to the central longitudinal axis 325 of inner body 322. On a side of ridge surface 329 closest to trailing end 326, circumferential projection 327 includes a tapered surface 328 such that circumferential projection 327 gradually recedes toward the base surface of inner body 322 toward trailing end 326, as shown in FIG. 8. Tapered surface 328 may have a frustoconical shape.

Outer body 332, shown in FIG. 9, extends from a leading end 332A to a trailing end 332B and has a central longitudinal axis 337 along a direction between such ends. In a central part of outer body 332 and extending to one side is a central channel 335. The presence of such central channel 335 through outer body 332 renders a cross-sectional shape of outer body 332 c-shaped. As depicted, an overall shape of outer body 332 is partially cylindrical but for the side opening of central channel 335. An outward facing surface of outer body 332 is smooth thereby minimizing any potential risk that tissue may be torn with the use of outer body 332. In other examples, an outer surface contour of outer body 332 may vary from that shown in FIG. 9. Central channel 335 is closed on one lateral side and opens out of outer body 332 on an opposite lateral side, as shown in FIG. 9. An inner surface 333 of outer body 332 defines a shape of central channel 335. Inner surface 333 includes a circumferential groove 334 recessed relative to a remainder of inner surface 333. A length of circumferential groove 334 is oriented orthogonally relative to the central longitudinal axis 337 of outer body 332 and in this way is generally parallel to a leading end surface of outer body 332 at leading end 332A. Outer body 332 may also include one or more recessed portions extending inward from leading end 332A. Such recessed portions may provide outer body 332 with added flexibility for attachment to inner body 322. In implant 310, outer body 332 includes two such recesses 336A, 336B, as shown in FIG. 11. Outer body 332 also includes openings 338, 339. Each opening extends internally through outer body 332 from leading end 332A to trailing end 332B, and the openings 338, 339 are spaced apart from each other. Each opening 338, 339 is sized to receive a filament therethrough. In some examples, a filament is passed through one or both of the openings 338, 339 as part of the implant 310. In variations of outer body 332, outer body 332 may include a single enclosed opening extending internally therethrough or may include three or more enclosed openings extending internally therethrough.

As mentioned above, implant 310 is shown in an assembled condition in FIGS. 11 and 12. Leading end 324 of inner body 322 is attached to balloon 340, and outer body 332 is attached to inner body 322. Specifically, outer body 332 is disposed external to and around circumferential projection 327 of inner body 322 so that ridge surface 329 is within circumferential groove 334, as shown in FIG. 11. Further, inner body 322 may be attached to balloon 340 via an adhesive. In this arrangement, a filament 312 passed through an opening 338, 339 in outer body 332 will be usable to secure balloon 340 to a tissue within a patient, as described in greater detail elsewhere in the present disclosure.

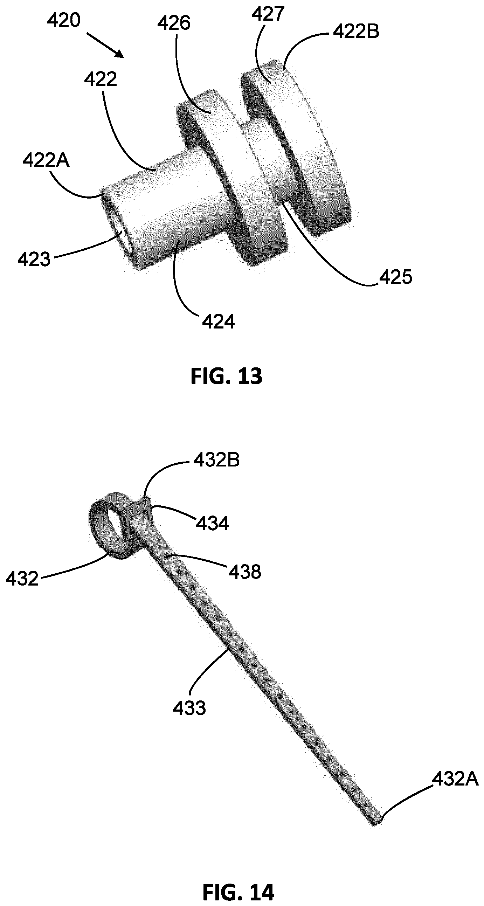

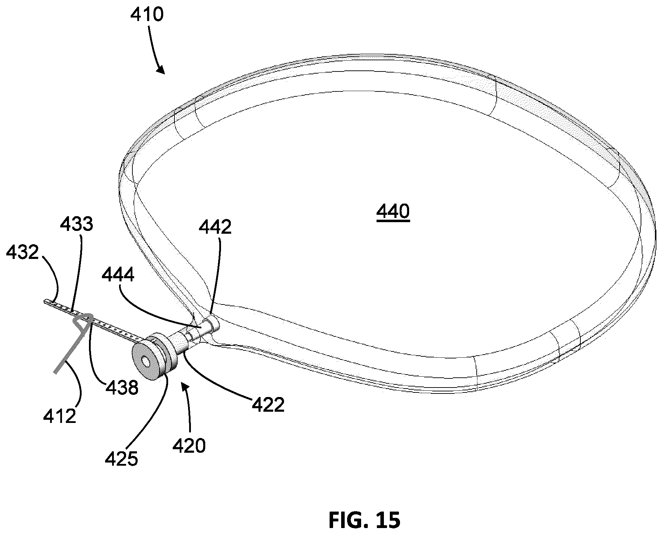

In one aspect, an implant 410 in an assembled state is shown in FIG. 15. Components of the implant 410 are also shown in FIGS. 13-14. Unless otherwise indicated, like reference numerals refer to like elements of implant 110 shown in FIGS. 4-6, but within the 400-series of numerals. Implant 410 includes a fitting 420 and a balloon 440 attached to the fitting 420. Implant 410 may also receive a filament 412. As with the preceding aspects, implant 410 may be provided without a filament 412.

In some examples, and as shown in FIG. 15, fitting 420 includes a central body 422 and a connection portion 432. Central body 422, shown in isolation in FIG 13, has a length extending from leading end 422A to trailing end 422B. Along its length from leading end 422A, central body 422 includes a leading portion 424, a first circumferential projection 426, a receiving portion 425 and a second circumferential projection 427. In this manner, the respective first and second circumferential projections 426, 427 are separated by receiving portion 425, which is recessed therebetween. Further, central body 422 includes a lumen 423 therethrough that is aligned along its length from leading end 422A to trailing end 422B. As depicted, each portion of central body 422 is cylindrical in shape, though it is contemplated that the shape of one or more portions of central body 422 may vary from that shown in FIG. 13. Turning to connection portion 432, connection portion 432 is shown in isolation in FIG. 14. In the depicted aspect, connection portion 432 is a cable tie. Connection portion 432 has a length extending from a first end 432A to a second end 432B. A strap 433 extends from first end 432A to a base 434 at second end 432B, as shown in FIG. 14. Strap 433 optionally includes, and in FIG. 14 includes, a plurality of openings 438 spaced apart along the length of connection portion 432. Such plurality of openings 438 may begin proximate first end 432A and terminate at a location along the length of strap 433 remote from second end 432B. It is contemplated that the strap 433 may include any number of openings at any desired spacing. Base 434 has a frame shape defining an opening therethrough, where a width of the frame is wider than a width of strap 433 passable through the frame opening, as shown in FIG. 14. Further, strap 433 and base 434 may include complementary features to form a ratcheted connection between the elements to tighten a loop formed by connection portion 432. For example, this may be a series of teeth (not shown) on the strap complemented by a receiving feature on the frame of base 434. One example of a strap mechanism that may be used is that described in U.S. Pat. No. 3,022,557, the disclosure of which is hereby incorporated by reference herein in its entirety.

In an assembled condition, fitting 420 is attached to balloon 440. In one example depicted in FIG. 15, such assembly is illustrated with fitting 420 including both central body 422 and connection portion 432. Central body 422 is attached to a conduit 444 extending from balloon 440, allowing for fluid communication between lumen 423 and an interior of balloon 440, and connection portion 432 is coupled to receiving portion 425 of central body 422. Strap 433 of connection portion 432 may be tightened so that a loop formed by strap 433 is snug against a surface of receiving portion 425. When a filament 412 is used to secure implant 410 to internal tissue of a patient, filament 412 may be passed through one or more openings of the plurality of openings 438, as shown in FIG. 15. With an opposite end of the filament fastened to the internal tissue, securement of the filament 412 to fitting 420 may provide secure fixation of implant 410 to the internal tissue. In some examples, an end of the filament attached to the internal tissue may be attached via an anchor secured to the internal tissue. As an example of the above, filament 412 may be secured to an opening 438 with strap 433 after implant 410 has been positioned at the implantation site. Filament 412 may be secured to an anchor coupled to tissue at the implantation site to secure implant 410 to the tissue. Filament 412 may secure implant 410 to the anchor with, for example, either a knotted or knotless technique. In another example, filament 412 may first be secured to an anchor coupled to the tissue at the implantation site. According to some aspects, the filament may come pre-loaded to the anchor or pre-attached to the anchor. The free end of filament 412 may be passed through an opening 438 in strap 433 while implant 410 is outside the patient, and implant 410 may be guided along filament 412 to the implantation site. Once implant 410 is positioned at the implantation site, filament 412 may be tensioned and secured to strap 433 (e.g., by tying a knot with filament 412). It should be appreciated that in various aspects disclosed herein, a first filament including a knotless structure such as a finger trap, a self-locking knot, or another one-way tensioning mechanism may be used to secure a second filament to another structure, such as a fixation anchor.

In another example, fitting 420 of implant 410 may include central body 422 without connection portion 432 (not shown). In this arrangement, implant 410 may also be secured to internal tissue of a patient. Specifically, a filament (not shown) already fastened to the anatomy may be looped around receiving portion 425 of central body 422, then tightened and fastened at receiving portion 425 to provide fixation.

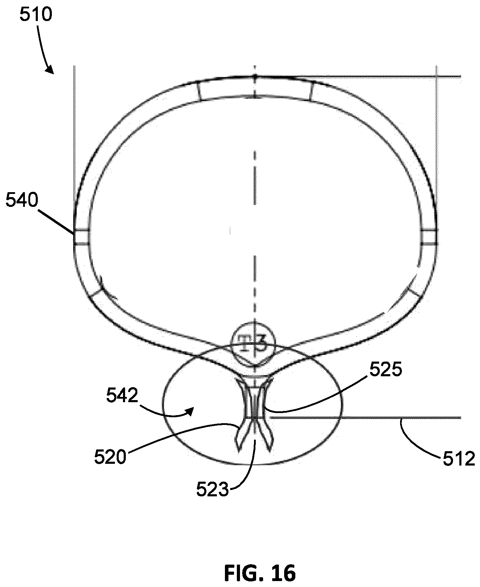

In one aspect, an implant 510 is as shown in FIG. 16. Unless otherwise indicated, like reference numerals refer to like elements of implant 110 shown in FIGS. 4-6, but within the 500-series of numerals. Implant 510 includes fitting 520 and balloon 540, with fitting 520 attached to balloon 540. Fitting 520 is attachable onto an inlet 542 of ballon 540 and includes a narrow central portion 525 or neck that flares outward at opposing ends. Fitting 520 is cannulated along its length via lumen 523 to facilitate fluid communication between fitting 520 and balloon 540. In arrangements where implant 510 includes a filament 512 for fixation of implant 510 to internal anatomy of a patient, such filament may be wrapped around narrow central portion 525. An opposite end of the filament may be secured to internal tissue of a patient either before or after fixation to fitting 520. As with the preceding aspects, implant 510 may be provided without a filament 512. In some examples, filament 512 may be secured to narrow central portion 525 at one end while the other end of Filament 512 may be passed through an opening in an anchor in tissue at the implantation site. Filament 512 may be pulled through the opening in the anchor to guide implant 510 to the implantation site. In other examples, implant 510 may be positioned at the implantation site before filament 512 is secured to narrow central portion 525 and an anchor. Balloon 540 of implant 510 may be inflated after implant 510 has been inserted into the implantation site, either before or after securing narrow central portion 525 to an anchor using filament 512.

In various aspects described herein, balloon implants (e.g., implants 110, 310, 410 510, 610, 710, 810, 910, 1010, 1110, 1210, 1310, 1410, 1510, 1610, 1710, 1810, 1910, 2010, 2310, 2710, 2710', 2710'', 2810, 3310, etc.) may be inserted through a relatively narrow portal into a patient in a folded, rolled, or otherwise collapsed configuration with the balloon (e.g., balloons 140, 340, 440, 540, 640, 740, 840, 940, 1040, 1140, 1240, 1340, 1440, 1540, 1640, 1740, 1840, 1940, 2040, 2340, 2740, 2740', 2740'', 2840, 3340) deflated. For example, the balloon implant may be inserted into a sheath of an instrument (e.g., sheath 72 of implant delivery instrument 50, described below) in a deflated condition, and the sheath may be inserted into the patient and retracted to expose the balloon implant. The balloon implant may then be unrolled or unfolded, the balloon may be inflated, and the balloon implant may be secured to tissue (e.g., directly to tissue, to anchors coupled to tissue, etc.) at an implantation site in the patient. In any of these aspects, the balloon implant may be secured before or after the balloon is inflated. In some examples, the balloon may be partially inflated before the balloon implant is secured, and then fully inflated after the balloon implant has been secured. Partial inflation of the balloon may aid in the unrolling or unfolding of the balloon implant. In some examples, the balloon may be partially or completely inflated after the filaments used to secure the balloon implant have been partially tensioned. For example, the filaments may be partially tensioned, then the balloon may be partially inflated, then the filaments may be fully tensioned, then the balloon may be fully inflated. Other examples may include further intermediate tensioning and inflation steps.

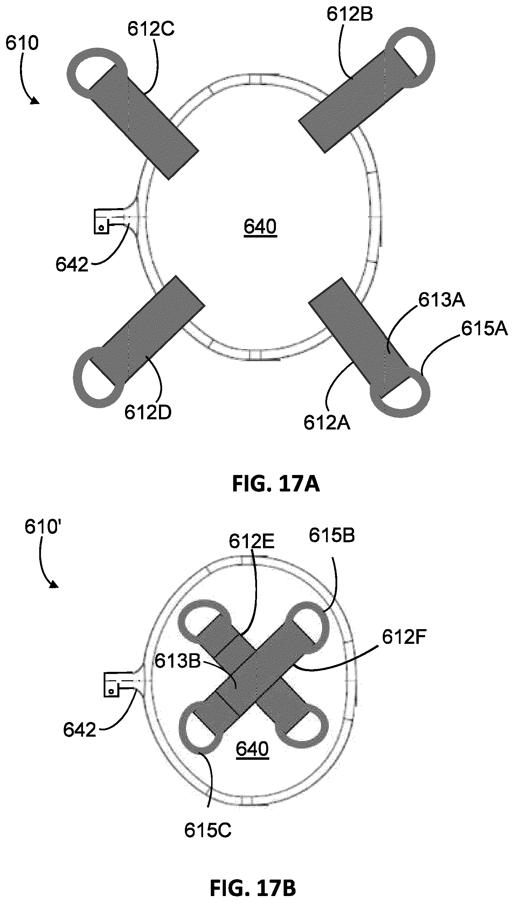

In one aspect, an implant 610 is as shown in FIG. 17A. Unless otherwise indicated, like reference numerals refer to like elements of implant 110 shown in FIGS. 4-6, but within the 600-series of numerals. As depicted in FIG. 17A, implant 610 includes balloon 640 and a peripheral portion including four fixation members 612A-D. A "peripheral portion" as used herein may include portions of an implant extending from or forming the top, bottom, edge, etc. of an implant (e.g., extending from a balloon or an augment of an implant). For example, the peripheral portion may be a portion of the implant other than the portion of the implant configured to receive the gas or liquid used to inflate the balloon. Thus, the peripheral portion may extend from the body of the balloon and may be configured to receive a filament or fixation member for coupling the implant to tissue without the filament or fixation member passing through the body of the balloon. The peripheral portion may include tabs, channels, flaps, straps, a flange or protuberance surrounding all, substantially all, or a portion of the implant, or other material through which a filament may be passed for securing the implant to tissue at an implantation site. A peripheral portion may be made from the same material as a balloon or augment or from different materials. A peripheral portion may be integrally formed with a balloon or augment, or may be formed separately and coupled to the balloon or augment (e.g., mechanically or adhesively). Fixation member 612A may be representative of each fixation member 612A-D and may include a strap 613A and a loop member 615A attached at a distal end of strap 613A. Each fixation member 612A-D may be adhered, fused, or otherwise connected to balloon 640. Adhesion may be accomplished through solvent bonding. Other forms of attachment of the strap 613A to the balloon 640 include stitching, heat staking, adhesives, and the use of a strap to envelope the balloon. In another example, the strap 613A may be incorporated into the wall of the balloon 640 during the balloon molding process, during which a mold is dipped at least once into molten material (e.g., polymer) to coat the mold and form the balloon. For example, a strap 613A may be attached to the mold with the loop member 615A extending beyond the portion of the implant 610 that is dipped in the molten material. In another example, after at least one dip of the mold into the molten material, a strap 613A may be held in place (e.g., using dowels or pins perpendicular to the balloon mold) against the partially formed balloon, and the partially formed balloon may be dipped additional times to complete the balloon 640, thus integrating the strap 613A into the balloon 640. In other examples, a strap 613A may be placed on the balloon before the molten material cures and may adhere to the balloon 640 as the material cures. Subsequent dips of the balloon mold after placing the strap 613A on the uncured material may further secure the strap 613A to the balloon 640. In some examples, the straps 613A may be separately formed of the same material as the balloon 640. In some examples., a portion of a strap 613A may be positioned on a balloon mold and covered with material in an electrospinning process used to form the balloon 640, thus adhering the strap 613A to the balloon 640.

In some examples, a strap 613A may be relatively short, with the loop member 615A relatively close to the balloon 640 (as shown in FIG. 17A), while in other examples, the strap 613A may be relatively long, with the loop members 615A more remote from balloon 640. In some examples, the loop member 615A may be a part of (e.g., integrally formed with) the strap 613A. For example, the strap 613A and the loop member 615A may be molded as a single component. In other examples, the loop member 615A may be formed by stitching or adhering the end of strap 613A to form a loop. In other examples, the loop member 615A may be formed separately from the strap 613A and coupled to the strap 613A. In some examples, loop member 615A-D may be a polymeric material (e.g., a polymeric D-ring). In some examples, the strap 613A and the loop member 615A may be made from or include biodegradable material (e.g., biodegradable polymeric materials), similar to the materials of the balloon 640. In some examples, implant 610 may be provided without any attachment means, such as filaments and/or anchors. Loop members 615A-D may be referred to as connection means and are configured and sized to receive a filament therethrough. The filaments may be passed through loop members 615A-D and used to guide the implant 610 into a patient to an implantation site and secure the loop members 615A-D to tissue at the implantation site. An inlet 642 of balloon may be spaced apart from each of the fixation members 612A-D. In other examples, implant 610 may include a lesser or greater number of fixation members 612, such as one fixation member, two fixation members, three fixation members, five fixation members, and so on. In some examples, one or more fixation members 612 may be positioned on implant 610 in different orientations (e.g., aligned with the axis of the inlet 642). Further, the fixation members 612 may be positioned with varying angular spacings therebetween (e.g., not evenly spaced). In still further examples, loop members on the fixation members may be replaced with other components adapted to receive a filament for attachment of implant 610 to tissue located internally within a body of a patient.

FIG. 17B illustrates another implant 610' according to an example aspect. Implant 610' may be substantially similar to implant 610 except as shown and described herein. As shown in FIG. 17B, implant 610' includes fixation members 612E-F. Each fixation member 612E-F includes a strap 613B with a loop member 615B-C coupled to each end of strap 613B. Straps 613B are arranged in a crisscross orientation over one side of balloon 640. As discussed above with respect to FIG. 17A, straps 613B may be adhered, fused, or otherwise connected to balloon 640 through solvent bonding, stitching, heat staking, adhesives, etc. Unlike the implant 610 of FIG. 17A, the loop member 615B-C of the implant 610 of FIG. 17B are positioned within the outer perimeter of the balloon 640, which may reduce the overall footprint of the implant 610 and the space required for implantation of the implant 610 inside the implantation site.

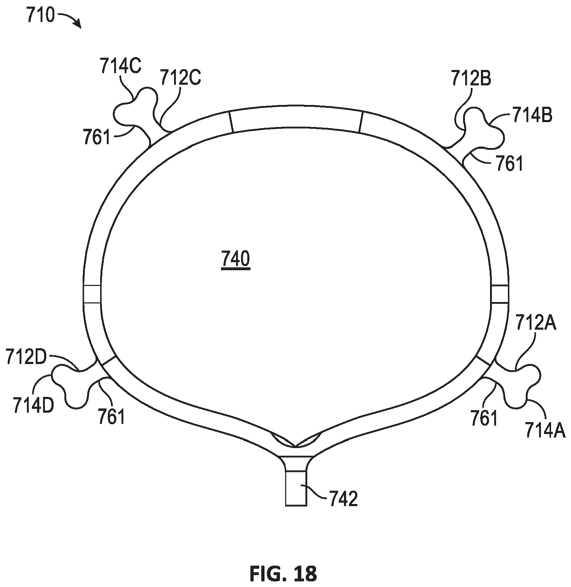

In one aspect, an implant 710 is as shown in FIG. 18. Unless otherwise indicated, like reference numerals refer to like elements of implant 110 shown in FIGS. 5-7, but within the 700-series of numerals. Implant 710 includes a balloon 740 and four fixation members 712A-D, each attached at separate locations on balloon 740. Each fixation member 712A-D includes a respective flared out end portion 714A-D and in this manner, a filament (not shown) may be secured to one or more of fixation members 712A-D along a neck portion 761 located between an attachment end abutting balloon 740 and end portion 714A-D. Fixation members 712A-D may be made of the same or different material as the balloon 740. In some examples, a filament or suture may be secured (e.g., knotted onto or looped around) a fixation member 712A-D. In other examples, secondary components may be attached to the fixation members 712A-D, where such secondary components, such as material shaped in a loop, may be used to attach a filament. For example, a loop may be attached or formed at one end of a filament or suture, and the other end of the filament or suture may be wrapped around the fixation member 712A-D, fed through the loop, and pulled taut to tighten the filament or suture around the fixation member 712A-D (e.g., like a luggage tag). In some examples, implant 710 may include a lesser or greater number of fixation members 712A-D, such as one fixation member, two fixation members, three fixation members, five fixation members, and so on. In some examples, materials for the fixation member may be one of the materials described for the balloon above. The material of the fixation member may be the same as the balloon or different from the balloon.

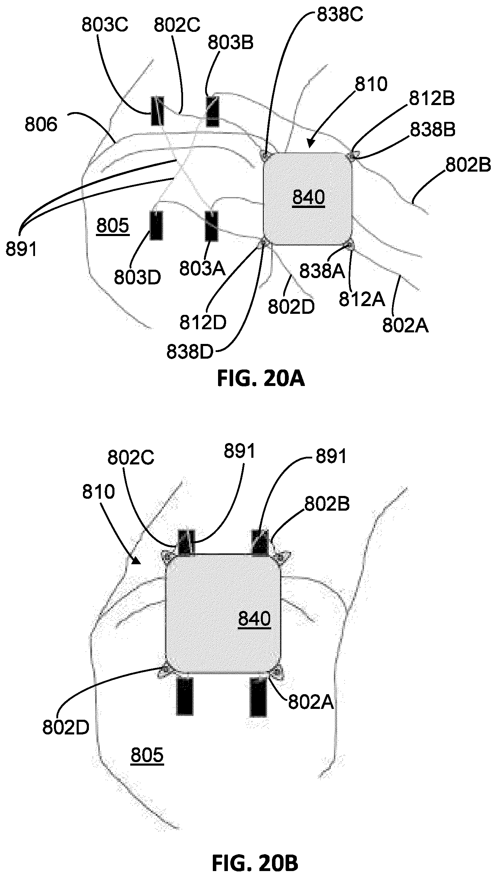

In one aspect, an implant 810 is as shown in FIGS. 20A-20B. Implant 810 includes balloon 840 and four fixation members in the form of tabs 812A-D. A "tab" as used herein is a broad term referring to a feature extending from a wall of a balloon (e.g., balloon 840) and may include, for example, a flange, a grommet, a projection, a protuberance, etc. The tabs 812A-D may be integrally formed with balloon 840 (e.g., in a mold), or in other examples may be formed separately, then attached to balloon 840. As shown in FIGS. 20A-20B, top and bottom surfaces of balloon 840 may be generally square or rectangularly shaped, though it should be understood as discussed above with respect to FIGS. 5-6 that the shape of the balloon 840 is not limited to any particular shape. As shown in FIGS. 20A-20B each corner of the four corners having one tab of the respective tabs 812A-D extending therefrom. Each tab 812A-D includes a respective opening 838A-D therethrough. Openings 838A-D may be configured and sized to receive a filament therethrough. The filaments may be used to guide the implant 810 into a patient to an implantation site and secure the tabs 812A-D to tissue at the implantation site. Balloon 840 may also include an inlet (not shown) for fluid communication between an external environment and an internal cavity of the balloon 840. The inlet may be as described with respect to any one of the aspects contemplated by the present disclosure. Filaments (e.g. sutures) may be used to secure implant 810 to tissue. For example, as depicted in FIGS. 20A and 20B, implant 810 may be secured by filaments 802A-D passing through respective openings 838A-D. It should be understood that while the tabs 812A-D and similar tabs disclosed in various aspects herein may be shown to include openings (e.g., opening 838A-D) extending therethrough, in some examples, the tabs may not include openings, and the filaments may instead be passed directly through the material of the tabs (e.g., using a surgical suture needle). In such examples, the tabs may be configured for guiding an implant into the patient and securing the implant to tissue.

In some examples, implant 810 may be secured to one or more fixation anchors coupled to tissue at the implantation site, such as fixation anchors 803A-D shown in FIGS. 20A-20B. In some examples, the filaments 802A-D and anchors 803A-D may be described herein as part of an implant (e.g., implant 810), but it should be understood that these may be provided separately from the implant. In some examples, the anchors 803A-D may have been coupled to tissue at the implantation site earlier in the surgical procedure and used for other purposes (e.g., as suture anchors in a double row repair). In some examples filaments 802A-D may be directly coupled to tissue 805 without the use of fixation anchors 803A-D. For example, filaments 802A-D may pierce tissue 805 at the implantation site and be tied in a knot to secure filaments 802A-D to tissue 805 or may be secured with knotless techniques. It will be understood that in other aspects discussed herein in which filaments coupled to implants are secured to fixation anchors in tissue at an implantation site, the filaments may instead be directly coupled to the tissue as discussed above. In some examples, the implant 810 may include more or fewer than four tabs 812A-D. For example, the implant 810 may include only the tabs 812A, 812B on one side of the balloon 840, may include only the tabs 812A, 812C on opposite corners of the balloon 840 or may include only one of the tabs 812A-D. In some examples, the implant 810 may include all of the tabs 812A-D, but less than all of the tabs 812A-D may be used to secure the implant 810 to tissue at the implantation site.

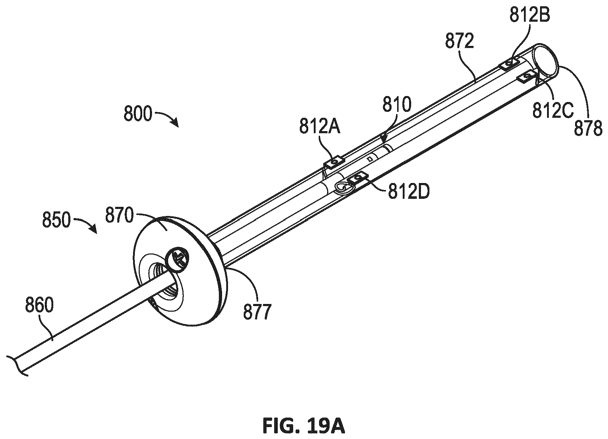

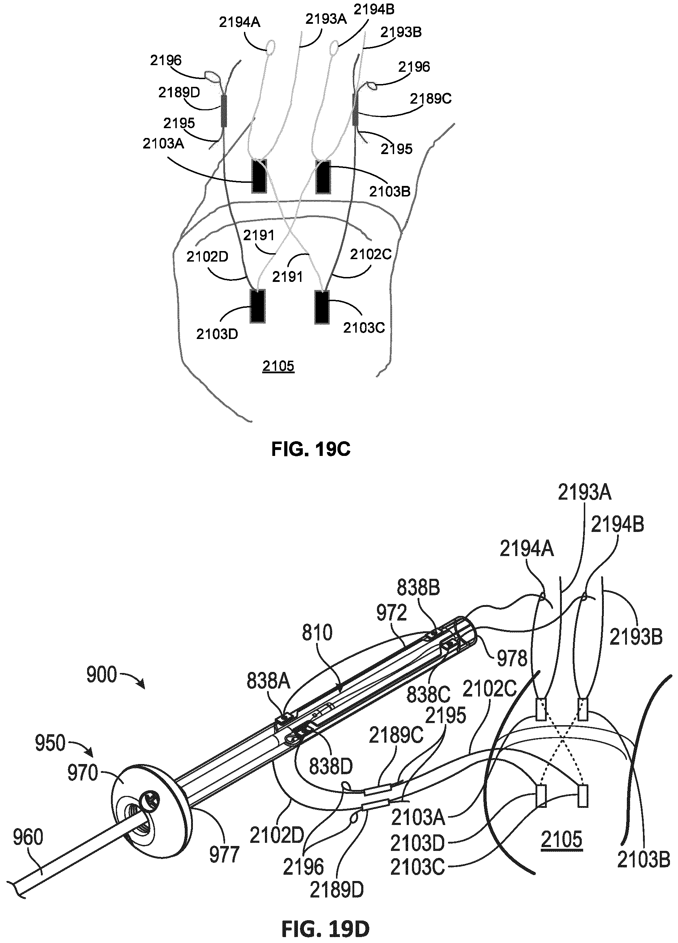

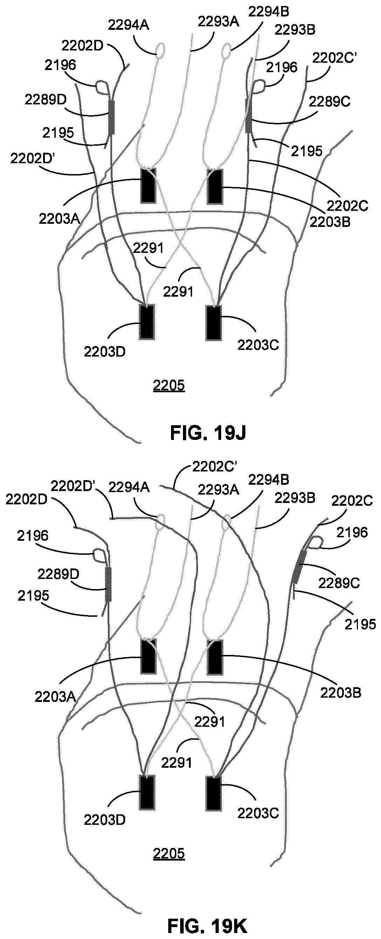

In the various embodiments disclosed herein, filaments may be secured to anchors or portions of the implant. Filaments may be secured to anchors in various ways. In some examples, a filament may be secured to an anchor by tying the filament to the anchor. In some examples, a first filament may be secured to an anchor by tying the first filament to a second filament coupled to or passing through an opening (e.g., an eyelet) in the anchor, or by coupling the first filament to the second filament using knotless fixation techniques such as finger traps. For example, as discussed herein with respect to FIGS. 19J-19O, filament 2202D and 2202D' may be separate filaments coupled together after filament 2202D' is passed through an eyelet of anchor 2203A and openings 838A and 838D, thus securing filaments 2202D and 2202D" to anchor 2203A and implant 810. In some examples, a first filament may be secured to an anchor by passing a filament through an opening in the anchor and coupling (e.g., using knotted or knotless fixation techniques) a first portion (e.g., a first end) of the filament to a second portion (e.g., a second end) of the filament, with the filament looped through the anchor. For example, as discussed herein with respect to FIGS. 19C-19I, a first portion of filament 2102D is passed through an eyelet of anchor 2103A and openings 838A and 838D before being secured to a second end of filament 2102D using finger trap 2189D, thus securing filament 2102D to anchor 2103A and implant 810. In some examples, a filament maybe attached to the anchor by the manufacturer. For example, the filament may be permanently or removably attached to the anchor by the manufacturer. The anchor may additionally include a knotless fixation element that may be used to secure a filament. Thus, in some examples, securing a filament to an anchor may include fastening the filament directly to the anchor, fastening the filament to another filament coupled to or passing through the anchor, or passing the filament through the anchor and fastening the filament to another filament or to itself.

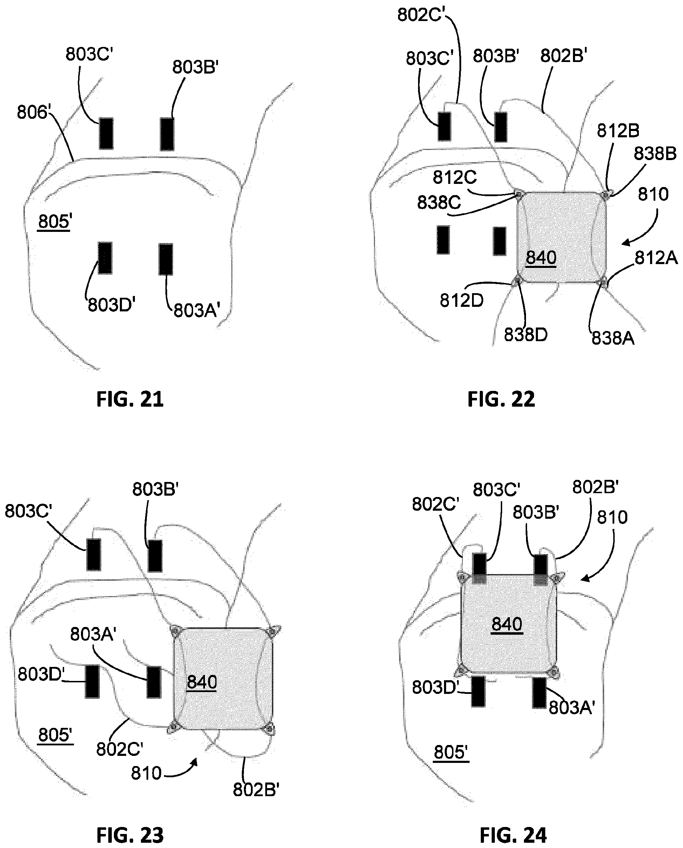

Filaments may similarly be secured to implants in various ways. In some examples, a filament may be secured to an implant by passing the filament through an opening in the implant or directly through material of the implant and tying the filament to the implant. For example, as discussed herein with respect to FIGS. 20A-20B, filaments 802A-D may be respectively passed through openings 838A-D and tied to tabs 812A-D. In some examples, a first filament may be secured to an implant by passing the first filament through an opening in the implant or directly through material of the implant and coupling (e.g., using knotted or knotless fixation techniques) the first filament to a second filament coupled to or passing through an opening in an anchor or coupling the first filament directly to an anchor. For example, as discussed herein with respect to FIGS. 21-24, filament 802C', which is coupled to anchor 803C, is passed through openings 812C, 812D, and secured to (e.g., tied to) anchor 803D', thus securing filament 802C' to implant 810. Alternatively, filament 802C' may be secured to a second filament coupled to anchor 803D', for example, using knotted or knotless fixation techniques. In some examples, a first filament may be secured to an implant by passing a filament through an opening in the implant or directly through material of the implant and coupling (e.g., using knotted or knotless fixation techniques) a first portion (e.g., a first end) of the filament to a second portion (e.g., a second end) of the filament, with the filament looped through the opening in or material of the implant (e.g., as discussed above with respect to FIGS. 19C-19I). In some examples, in order to secure the filament to the implant, a filament attached to an anchor or passing through an anchor may be secured to the same anchor, a different anchor, or a knotless fixation element coupled to the same filament or a different filament after the filament passes through an opening in the implant or through the material of the implant. Thus, securing a filament to the implant may include, for example, fastening the filament directly to the implant, fastening the filament to another filament coupled to or passing through an anchor after passing the filament through the implant, or fastening a first end of the filament to a second end of the filament with the filament passing through the implant and an anchor.

As one example of the above, a first portion (e.g., a first end) of a filament may be passed through an eyelet of an anchor, passed through an opening of an implant, and coupled to (e.g., using knotted or knotless fixation techniques) to a second portion (e.g., a second end) of the filament. By coupling the first portion of the first filament to the second portion of the filament with the filament passing through the eyelet of the anchor and the opening in the implant, the filament is secured to the anchor and the implant. Filaments may also pass through multiple anchors and through the implant in multiple locations. For example, a first portion of a filament may be passed through eyelets of a first anchor and a second anchor, passed through first and second openings in an implant, and coupled to a second portion of the filament. By coupling the first portion of the first filament to the second portion of the filament with the filament passing through the eyelets of the first and second anchors and the openings in the implant, the filament is secured to the first and second anchors and to the implant. A filament may be a single strand that, for example, is coupled at a first end and has a single free end. In other examples, a filament may pass through or be coupled to one or more anchors, with both ends of the filament being free for use in securing an implant. As discussed above, the two free ends may be used to form a loop between the anchor or anchors and the implant. At least one of the free ends of the filament may include a feature for receiving and securing the other end (e.g., a finger trap, a sliding knot, etc.). in some examples, a filament may include two strands coupled together. For example, the length of a filament may be extended by attaching a second strand to the end of the first strand using, for example, a finger trap.

In the example shown in FIGS. 20A-20B, each anchor 803A-D is first coupled to tissue at the implantation site (e.g., inserted into bone at the implantation site). Each filament 802A-D (e.g., sutures) may be coupled respectively to one of the anchors 803A-D either before or after the anchors 803A-D are coupled to the implantation site. An end of each filament 802A-D may then be passed through the opening 838A-D in a respective tab 812A-D (e.g., outside of the implantation site, outside the body of the mammal receiving the implant 810, etc.). The implant 810 may then be moved along the filaments 802A-D to the implantation site. The filaments 802A-D may then be tensioned and used to secure the implant 810 to the anchors 803A-D at the implantation site.

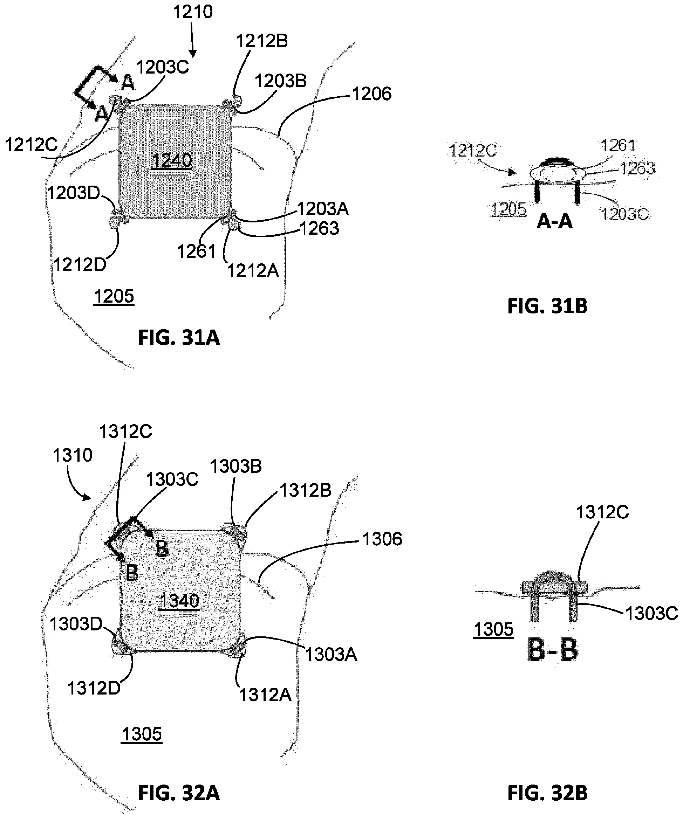

In some examples, filaments 802A-D may be only partially tensioned such that implant 810 is allowed to move relative to anchors 803A-D (or relative to locations that filaments 802A-D are directly coupled to tissue 805 without the use of anchors 803A-D). Thus, securing the implant 810 to tissue 805 using filaments 802A-D may not completely or substantially completely restrict movement of the balloon implant. Some amount of movability may allow implant 810 to adjust its position in response to different motions of the joint in which implant 810 is implanted. The distance from anchors 803A-D that implant 810 is allowed to move may be determined by the slack in filaments 802A-D. In some examples, filaments 802A-D (and the other filaments discussed herein) may be made from a more flexible material than traditional sutures, such as FLEXBAND^{®} biomaterial, made from a co-polymer of polycaprolactone and polyurethane-urea, or other flexible filaments. This may further allow some motion of implant 810 relative to anchors 803A-D. It should be understood that the other methods or structures for securing an implant to tissue disclosed herein may similarly be performed or modified to allow for motion of the implant relative to the tissue. For example, tabs 1212A-D of implant 1210, shown in FIGS. 31A-31B secured to tissue by fixation elements 1203A-D (shown as U-shaped bars or staples), may be configured to enable some relative movement with respect to the fixation elements 1203A-D (e.g., based on tab length, fixation location, shape of tab, etc. In some examples, implants disclosed herein may be secured such that movement of the implant is allowed in one or more lateral directions, or in all lateral directions. In some examples, the implant may be free to move at least at least 2.0 millimeters in a lateral direction (e.g., between 2.0 millimeters and 10.0 millimeters in a lateral direction, between 2.0 and 6.0 millimeters in a lateral direction, between 2.0 and 12.0 millimeters in a lateral direction, etc.), or at least 3.0 millimeters in a lateral direction, or at least 4 millimeters in a lateral direction, which may be a larger distance than a tightly secured implant may move due to the flexibility of the implant materials. A "lateral direction" as used herein refers to a direction along the tissue to which the implant is secured (e.g., left, right, up, or down as shown in FIG. 31A), as opposed to a direction towards or away from the tissue.

In another aspect, the present disclosure relates to an implant system that includes an implant and an implant delivery instrument configured to receive the implant thereon. The implant system may be arranged such that the implant is preloaded on the instrument when delivered to a user. The implant system is usable to deliver the implant to a surgical site and to otherwise facilitate the deployment and readiness of the implant at the surgical site. While certain of the described implant systems include an implant preloaded on the instrument, it should be appreciated that in other aspects, the implant system may be provided to a user such that the implant and implant delivery system are separate. In such cases, the user may load the implant on the instrument (e.g., prior to or during a procedure).

In one aspect, an implant system 1 is as shown in FIGS. 1-3 and includes implant 10 and implant delivery instrument 50. Implant 10 may include a balloon 40 as described above and may further include an inlet and other accompanying features as described above with respect to implant 110, implant 210, etc. Implant delivery instrument 50 includes a handle 52, a shaft 60 extending from the handle 52, a sheath 72 slidably disposed over the shaft 60, and a lock bar 82 movably attached to the shaft 60. In an assembled condition, implant 10 may be attached to an end of a distal portion 62 of shaft 60 and sheath 72 may be slid along shaft 60 toward and away from handle 52 to control exposure of implant 10 from within sheath 72. Implant delivery instrument 50 may be configured such that handle 52 includes physical features manipulable to control shaft 60 and in turn, implant 10 attached to shaft 60.

As shown in FIG. 3, in some aspects, implant 10 is mounted on or interfaces with an inner shaft 61, such as a needle, disposed within a lumen of shaft 60. In some examples, the inner shaft 61 may be loaded with a plug 63 detachably connected to a distal end of the inner shaft 61. The implant 10 may include a fitting 20 configured to interface with the shaft 60 and/or the inner shaft 61. Inner shaft 61 may be inserted through an opening (e.g., conduit, inlet, etc.) in fitting 20 into an inner cavity of balloon 40. Plug 63, which may be larger than the opening, may deform as plug 63 is pushed through the opening. After balloon 40 is inflated, inner shaft 61 may be pulled out of fitting 20, which may cause the plug 63 to dislodge from the inner shaft 61 at the inner surface of the fitting 20 to seal the balloon 40. In other examples, the plug may be pre-loaded inside a conduit or other inlet (e.g., in fitting 20) of implant 10 and may be engaged by the inner shaft during attachment of the instrument 50 to the implant 10, and then pulled against the inner surface of the conduit or inlet as described above. In other examples, a valve may be used instead of a plug. Further details of exemplary detachable connection means are provided in U.S. Pat. No. 9,289,307 (the "'307 Patent), the disclosure of which is hereby incorporated by reference herein in its entirety.

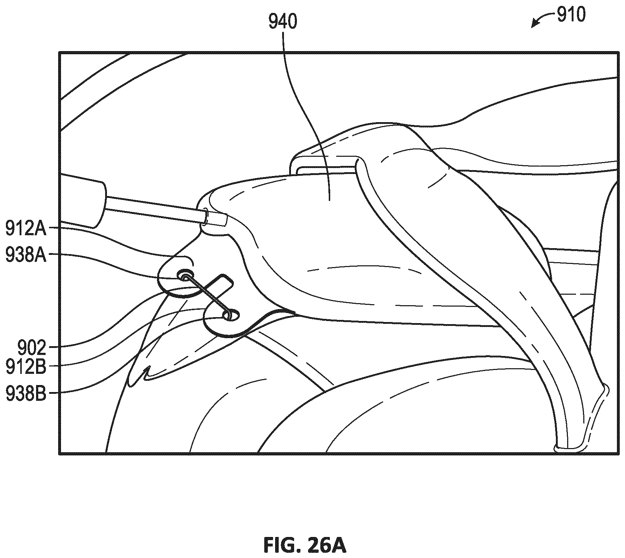

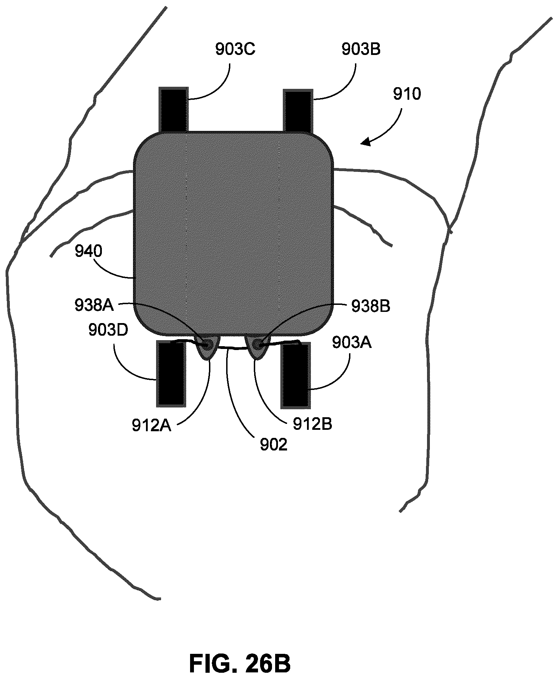

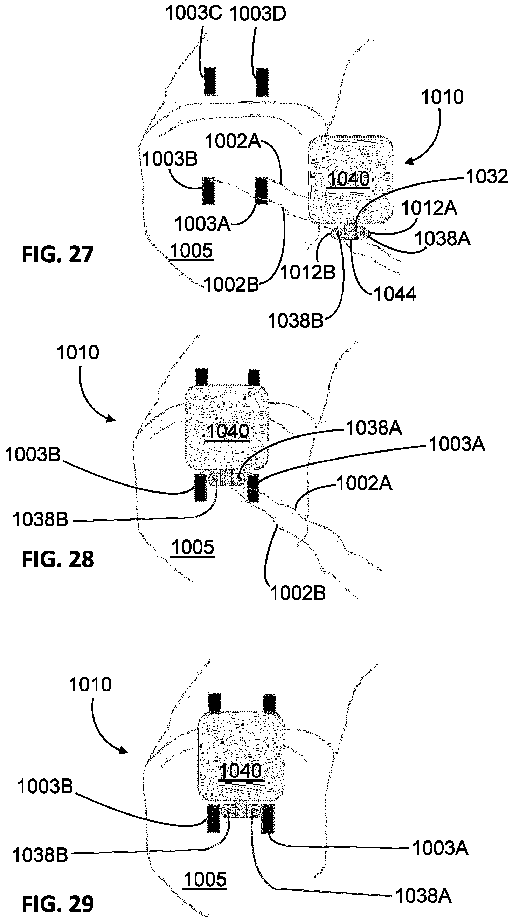

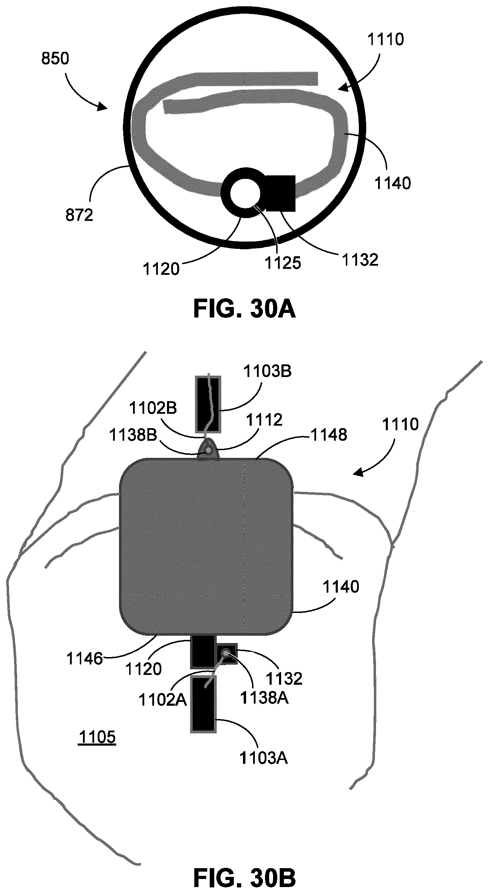

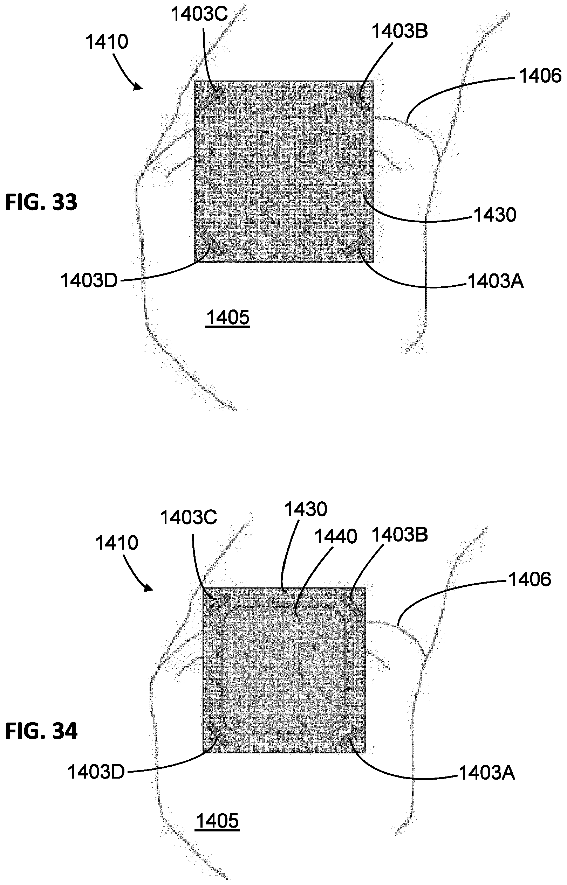

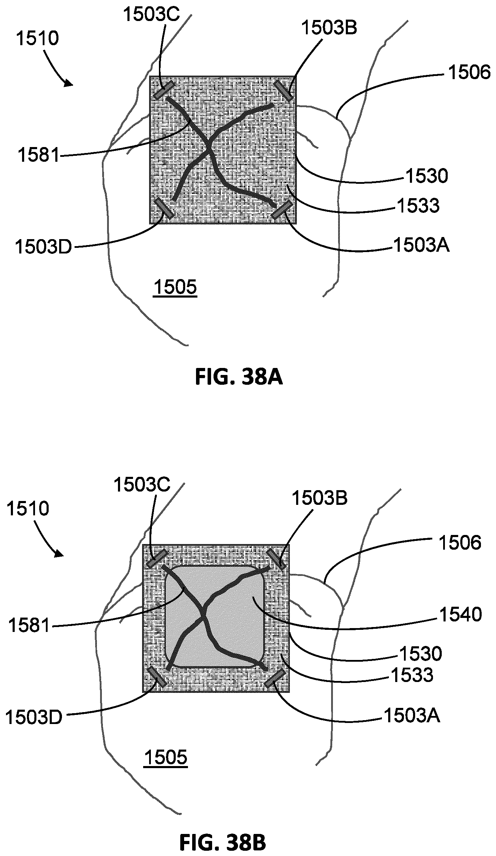

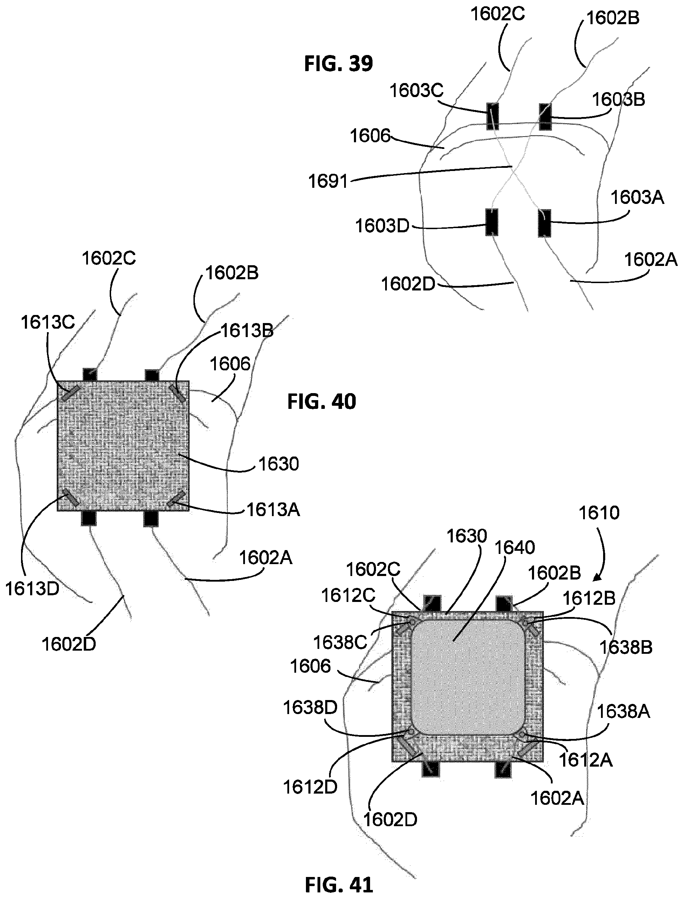

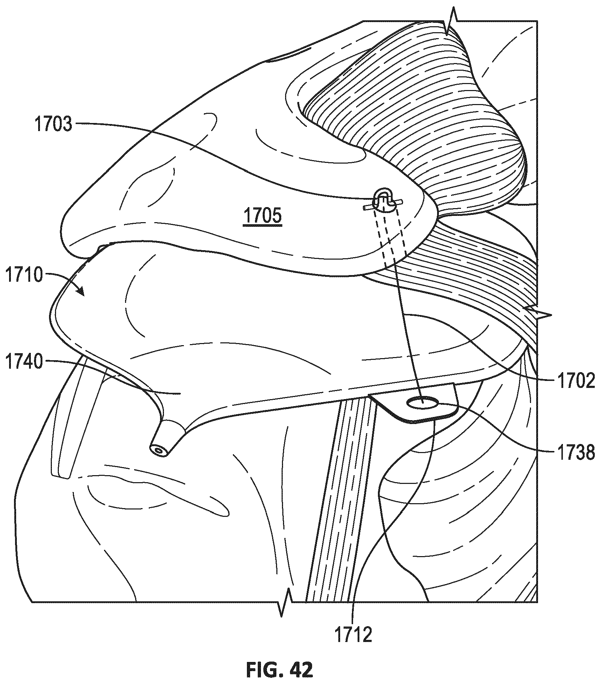

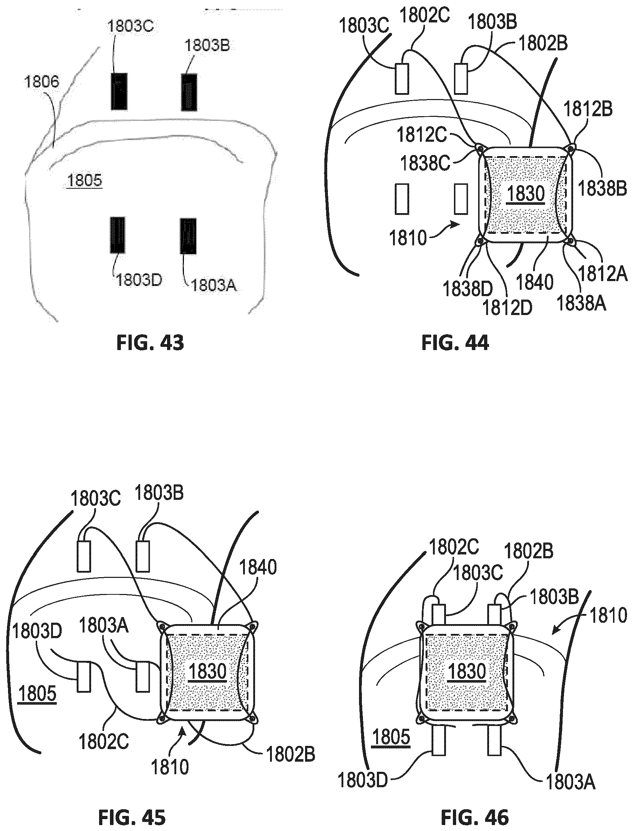

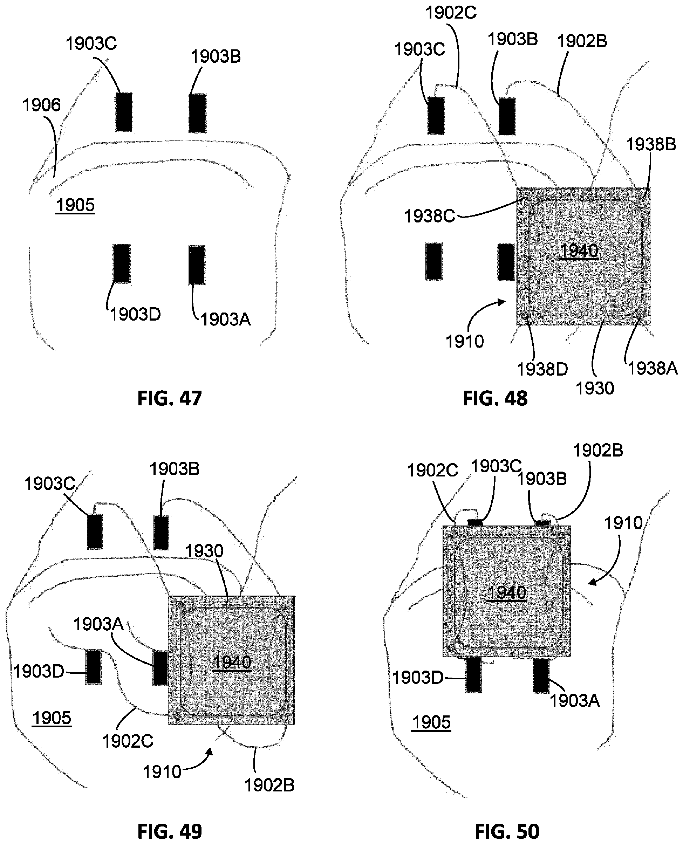

Implant 10 may be provided completely deflated and rolled, folded in various ways (e.g., in an accordion fold), or otherwise collapsed to a small volume for a minimally invasive delivery, while covered, protected and maintained in the collapsed configuration by sheath 72. Prior to use, lock bar 82 may be positioned coaxially with shaft 60 (not shown) such that lock bar 82 resists or prevents retraction of sheath 72. When lock bar 82 is so positioned, the locked configuration may be released by rotating lock bar 82 relative to shaft 60, e.g., to position lock bar 82 perpendicular to shaft 60, and then sliding lock bar 82 to abut handle 52, as shown in FIG. 1. In this way, the configuration shown in FIG. 1 may be considered an unlocked position that allows for sliding movement of sheath 72 relative to shaft 60. In some examples, mechanisms other than the lock bar 82 may be provided for restricting or preventing the retraction of the sheath 72 (e.g., a component fastened to shaft 60 adjacent base 70 that restricts retraction of 72 until the component is removed from shaft 60 or slid along shaft 60 toward handle 52). When implant delivery instrument 50 is positioned at a repair site within a patient and ready for deployment of implant 10, and before inflation of implant 10, sheath 72 is withdrawn thereby allowing implant 10 to unroll and expand. In some aspects, during inflation, implant 10 first unrolls, and only during or after complete unrolling, implant 10 begins to expand in thickness until reaching a fully or predetermined inflated shape or size. While not shown in the figures for implant 10, the inflated shape for implant 10 may be similar to that shown for implant 110 shown in FIGS. 5-6, implant 310 shown in FIG. 12, implant 410 shown in FIG. 15, implant 510 shown in FIG. 16, implant 610 shown in FIGS. 17A-17B, implant 710 shown in FIG. 18, implant 810 shown in FIGS. 20A-25D, implant 910 shown in FIGS. 26A-26B, implant 1010 shown in FIGS. 27-29, implant 1110 shown in FIGS. 30A-B, implant 1210 shown in FIGS. 31A-32B, implant 1410 shown in FIGS. 33-34, implant 1510 shown in FIG. 38B, implant 1610 shown in FIG. 41, implant 1710 shown in FIG. 42, implant 1810 shown in FIG. 44, implant 1910 shown in FIG. 48, or any combination thereof. Further, the implant 10 may be in configurations not explicitly shown in the Figures. For example, the balloon may not be limited to any particular shape, and where the implant 10 includes an augment, the augment may not be limited to any particular shape.

Handle 52 includes a knob 54 that is rotatable from a first closed position to a fully opened position, where such rotation simultaneously rotates the shaft 60 connected thereto over a proximal portion of inner shaft 61. In some examples, the closed position may be as shown in FIG. 1, and the open position may result from sliding the partially-cylindrically shaped knob 54 in a circumferential direction along a surface of the handle so that one side, i.e., where the arrow points in FIG. 1, enters the handle. In some aspects, inner shaft 61 includes locking features to secure inner shaft 61 to implant 10 (e.g., to fitting 20), and the rotation of shaft 60 to a partially or fully opened position inner shaft 61 may cause the locking features to release to allow withdrawal of inner shaft 61 from fitting 20. Alternatively or additionally, rotation of knob 54 causes axial movement of the inner shaft 61 within shaft 60 away from implant 10 (e.g., in a proximal direction toward handle 52) until the inner shaft 61 detaches from fitting 20. As discussed above, the withdrawal of inner shaft 61 from fitting 20 may cause plug 63 to detach from inner shaft 61 and seal fitting 20. In some aspects, rotation of knob 54 is selectively allowed or prevented using safety switch 56.

An operator port 58 located optionally at a proximal end of handle 52 is connectable to an external inflation medium reservoir and/or pressurizing device, such as a pump or a syringe (not shown). The inflation medium may be a fluid (e.g., saline) which is transferable from the external reservoir through a lumen extending through handle 52, shaft 60 and into the implant 10. In other aspect, the inflation medium may be a gas (e.g., air, carbon dioxide, etc.). In some aspects, handle 52 includes connecting means 59 to auxiliary devices or instruments, such as a pressure meter, a temperature meter and/or a flow rate meter. The inflation medium may flow through shaft 60 or inner shaft 61 and into balloon 40 through fitting 20.

The inner shaft 61 and/or shaft 60 can be made of any biocompatible rigid or semi-rigid material, such as but not limited to, metals (e.g., stainless steel). Handle 52 and other parts affixed thereto can be made of plastic or other polymers such as polycarbonate. Any of the disclosed parts and elements may be disposable or non-disposable and may be configured for single use or repeated use.

In one aspect, an implant system 100 is as shown in FIGS. 4-6 and includes implant 110 and implant delivery instrument 150. Details of implant 110 are as described above. As to implant delivery instrument 150, unless otherwise indicated, like reference numerals refer to like elements of implant delivery instrument 50 shown in FIG. 1, but within the 100-series of numerals. Implant delivery instrument 150 may be removably attached to implant 110 in the same manner as implant delivery instrument 50 is removably attached to implant 10. Implant delivery instrument 150 includes a sheath 172 extending from a base 170, the combined structure being slidably received over shaft 160, which in turn is attached to handle 152. Sheath 172 defines a lumen therethrough sized to receive shaft 160 and a folded implant 110, as shown in FIG. 3. Between proximal and distal ends 177, 178 along a length direction of sheath 172 is a trailing end window 175 through one side of sheath 172. And, from trailing end window 175 to distal end 178, sheath 172 further includes a slit 174. Along a portion of sheath 172 including slit 174, sheath 172 does not define a full enclosure due to the slit 174 on one side. In this manner, sheath 172 may have a generally c-shaped cross-section from trailing end window 175 to distal end 178. Slit 174 may be narrower in size than trailing end window 175.

In an initially prepared condition with implant 110 loaded within sheath 172, trailing end window 175 exposes fitting 120 of implant 110 therein, as shown in FIG. 3. In this way, a filament 112 may be physically attached to fitting 120, e.g., by being passed through opening 138, while implant 110 remains enclosed within sheath 172. Implant system 100 may be delivered to an implantation site within a patient with or without filament 112 preloaded on implant 110. It should be appreciated that such possibilities are contemplated for any of the implant system aspects contemplated by the present disclosure. Further, the inclusion of slit 174 allows for sheath 172 to be retracted along shaft 160 without causing filament 112 to be drawn into the lumen of sheath 172 or to otherwise interrupt the retraction process, since such filament 112 may extend through and out of slit 174 as sheath 172 is retracted. In some examples, implant delivery instrument 150 may be configured so that sheath 172 is rotationally fixed relative to shaft 160. In some examples, sheath may be tubular and in a subset of such examples may be cylindrical. In some examples, a cross-sectional shape of sheath 172 at the trailing end window 175 and/or at the slit 174 may be c-shaped. In some examples, central body 122 of fitting may have a diameter between 1.5 mm and 3.5 mm, a width dimension of connection portion 132 may be approximately 3.0 mm to 5.0 mm, and an inner diameter of sheath 172 may be 4.0 mm to 11.0 mm. In one specific example of implant system 100, central body 122 of fitting 120 may have a diameter of approximately 2.5 mm, a fitting at connection portion 132 may have a width of approximately 4.1 mm and sheath 172 may have a diameter of approximately 7.0 mm. Sheath 172 may be made of a polymeric material and may include additives and/or coatings. Such material ensures sheath 172 has strength to withstand forces experienced during use without susceptibility to breakage. In some examples, sheath 172 may be made of nylon. In a subset of these examples, the sheath may include barium sulphate and/or EverGlide^{®}.

In one aspect, an implant system 300 is as shown in FIGS. 8-12 and includes implant 310 and implant delivery instrument 350. Details of implant 310 are described above. And, to avoid ambiguity, implant 310 may include a balloon 340 and a fitting 320 with inner body 322 and outer body 332. Implant 310 may be coupled to filament 312 (e.g., filament 312 may pass through an opening in implant 310). As to implant delivery instrument 350, unless otherwise indicated, like reference numerals refer to like elements of implant delivery instrument 50 shown in FIG. 1, but within the 300-series of numerals. Implant delivery instrument 350 may be removably attached to implant 310 in the same manner as implant delivery instrument 50 is removably attached to implant 10. Implant system 300 may also include guide 390 shown in FIG. 10. Guide 390 may include a side-facing groove sized to be received on shaft 360. In some examples, guide 390 may have a c-shaped cross-section. Further, guide 390 may be sized such that when guide 390 is attached to shaft 360 as shown in FIG. 10, guide 390 is slidable over shaft 360 through a portion of shaft 360 disposed in sheath 372. In this manner, guide 390 is usable to push outer body 332 through sheath 372 in a distal direction toward inner body 322. As described further in the methods of the present disclosure, such functionality of attaching outer body 332 to inner body 322 after an initial loading of implant 310 onto instrument 350 may be advantageous in that a filament 312 may be loaded onto outer body 332 proximal to sheath 372, as shown in FIG. 9, and may then be slid onto and attached to inner body 322, as shown in FIGS. 11-12, all prior to advancement of implant delivery instrument 350 into a patient. In this manner, a filament 312 may be attached to the implant delivery instrument 350 with balloon 340 thereon without difficulty. Further, filament 312 that is passed through an opening 338, 339 in outer body 332 may be secured to an anchor in the patient before or after such loading of filament 312 onto implant delivery instrument 350.

As shown in FIG. 8, inner body 322 may be coupled at one end to balloon 340, and at the other end to shaft 360, thus coupling balloon 340 to shaft 360. In some examples, inner body 322 may have been previously coupled to balloon 340, for example, during the manufacture of balloon 340. Balloon 340 may be rolled up and positioned within sheath 372 of implant delivery instrument 350 (as shown in FIG. 11). As shown in FIG. 9, filament 312 is passed through opening 338 in outer body 332. In some examples, filament 312 may be secured (e.g., tied) to outer body 332. Outer body 332 is then slidably coupled to shaft 360, for example, by snapping outer body 332 onto shaft 360 through the side opening of central channel 335. As shown in FIG. 10, guide 390 may then be snapped onto shaft 360 in a similar manner and used to push outer body 332 in a distal direction along shaft 360 toward inner body 322 until outer body 332 snaps onto inner body 322, thus securing filament 312 to balloon 340. FIG. 11 shows outer body 332 after it has snapped over inner body 322. As shown in FIG. 12, after implant delivery instrument 350 has been inserted into an implantation site, balloon 340 may be released from sheath 372 unfolded and inflated, with filament 312 coupled to balloon 340 via outer body 332 and inner body 322. Filament 312 may then be used to secure balloon 340 to tissue at the implantation site.

In one aspect, an implant system may include implant 410 and an implant delivery instrument, such as implant delivery instrument 50. Details of implant 410 are described above. Implant delivery instrument 50 may be removably attached to implant 410 in the same manner as implant delivery instrument 50 is removably attached to implant 10.

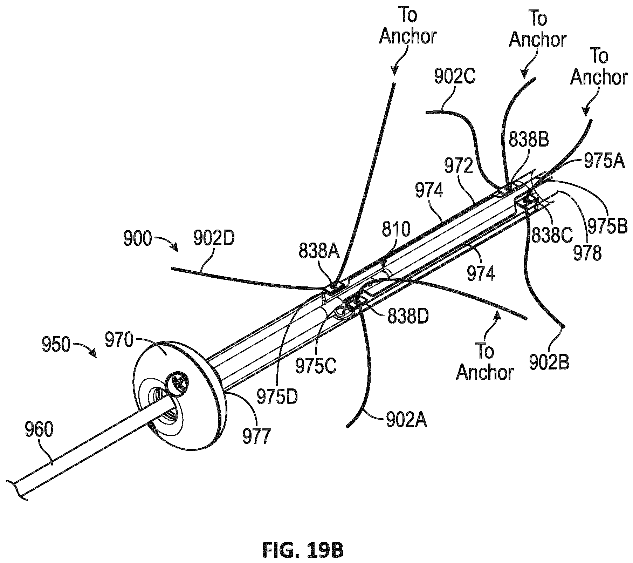

In one aspect, an implant system 800 as shown in FIG. 19A may include implant 810 and an implant delivery instrument 850, which may be substantially similar to or the same as implant delivery instrument 50. Details of implant 810 are described above. In a variation, implant 1810 shown in FIGS. 44-46, or implant 1910 shown in FIGS. 48-50 may similarly be included in this system in place of implant 810. Implant delivery instrument 850 may be used to deliver various implants disclosed herein, including, for example, implants 810, 1810, 1910. Implant delivery instrument 850 may be removably attached to implant 810, 1810, 1910 or other implants in the same manner as implant delivery instrument 50 is removably attached to implant 10. Implant 810 may be loaded into sheath 872 of implant delivery instrument 850, for example, by coupling implant 810 to shaft 860, rolling or folding implant 810, and moving sheath 872 distally along shaft 860 to envelop implant 810. As discussed above with respect to implant delivery instrument 150 and implant 110, shaft 860 may be configured to interface with and be coupled to a fitting (e.g., similar to 120). Shaft 860 may include a conduit configured to provide fluid to the fitting for inflating the body of balloon 840. Implant delivery instrument 850 may then be inserted into a patient and positioned adjacent an implantation site. Sheath 872 may then be retracted by moving sheath 872 proximally along shaft 860 until Implant 810 is exposed. Implant 810 may then be unrolled or unfolded, for example, by inflating balloon 840 or due to the stiffness of implant 810. Sheath 872 may also include various cutouts, openings, channels or the like to accommodate one or more filaments which can be or are connected to the implant 810. One such example of such a sheath is sheath 972 of FIG. 19B.

In further aspects, an implant system may include any one of implant 210, 310, 410 510, 610, 710, 810, 810', 910, 1010, 1110, 1210, 1310, 1410, 1510, 1610, 1710, 1810, 1910, 2010, 2310,2710, 2810 and an implant delivery instrument, such as implant delivery instrument 50, 150, 350, 850, 950, 1050. It should be understood that in any example disclosed herein in which an implant is not explicitly described as being delivered to an implantation site using an implant delivery instrument, such implant may be delivered using one or more implant delivery instruments (e.g., instrument 50, 150, 350, 850, 950, 1050 or similar) by rolling or folding the implant or components of the implant, inserting the implant into a sheath, advancing the sheath into an implantation site and removing the implant from the sheath at the implantation site (e.g., by retracting the sheath. In various examples, an augment and a balloon of an implant may be loaded into separate delivery instruments, which may be used to separately deliver the augment and the balloon to the implantation site. It should also be appreciated that any implant system may optionally include one or more filaments, which may be preloaded on an implant delivery instrument, and/or one or more anchors for securement of filament to internal tissue of a patient at a repair site.

In another aspect, the present disclosure relates to a kit that may include an implant with attachment means, two or more implants, two or more implant delivery instruments, or a combination of implants and implant delivery instruments. In some embodiments, a kit may include any implant as contemplated by the present disclosure and one or more filaments and anchors. For example, a kit may include an implant, a filament, and an anchor. In other examples, a kit may include an implant and a filament. In yet another example, a kit may include an implant and an anchor. In some aspects, a kit may include two or more implants from among the implants contemplated by the present disclosure. In some aspects, a kit may include one or more augments that may be provided separate from or coupled to a balloon. In some aspects, a kit may include two or more implant delivery instruments from among the implant delivery instruments contemplated by the present disclosure. In some aspects, a kit may include a first delivery instrument preloaded with an augment and a second delivery instrument preloaded with a balloon implant. In some aspects, a kit may include an implant delivery instrument and two or more implants. In any one of the contemplated kit aspects, a kit may include one or more filaments. In any one of the contemplated kit aspects, a kit may include one or more anchors. In any aspect with an implant delivery instrument, a kit may include a guide for slidable attachment to a shaft of the implant delivery instrument.

The kit may be varied in many ways. For example, the various combinations of elements of any kit contemplated herein may be included in a single package or distributed among multiple packages. In other examples, the kit contemplated herein may be accompanied by an instruction manual on how to perform one or more of the methods of using the contents of the kit.

In another aspect, the present disclosure relates to methods of implanting an implant within a patient. These methods include methods of assembling an implant and an implant delivery instrument, methods of delivering and securing the implant to a target site within a patient, and methods that encompass assembly, delivery, and securement. In any one of the contemplated aspects, the method may be performed with attachment of the implant to a rotator cuff to facilitate a rotator cuff repair, with attachment of the implant to the acromion bone, or with attachment of the implant to the humeral head. Further, it should be appreciated that these are non-limiting examples of implant placement.

In one aspect, a method of assembly in preparation for implantation of an implant is performed by combining implant 10 and implant delivery instrument 50 to make implant system 1. Prior to using implant delivery instrument 50 in surgery, implant 10 is loaded onto implant delivery instrument 50. In some examples, the implant 10 may be preloaded into the implant delivery instrument 50 (e.g., during manufacture) or may be loaded in advance of or during a surgical procedure (e.g., a by a member of the surgical team). To do so, lock bar 82 is rotated and slid into the unlocked position on shaft 60 as shown in FIG. 1, and sheath 72 is retracted toward handle 52, thereby exposing a distal portion 62 of shaft 60. Implant 10 is then attached to a tip of implant delivery instrument 50 (e.g., to a distal tip inner shaft 61). Engagement between the implant 10 and the implant delivery instrument 50 may be made using means similar to one of the means described with respect to the systems of the present disclosure. Implant 10 is then folded or rolled. To fold implant 10, respective lateral sides of implant 10 may be folded about one or more axes generally parallel to a length direction of implant delivery instrument 50 to form a curled or rolled shape. One example of a folded implant folding is also illustrated in part in FIG. 11 by balloon 340. According to various aspects, the implant 10 may be folded, rolled, or otherwise collapsed prior to attachment to the implant delivery instrument 50. Next, sheath 72 is slid away from handle 52 and advanced over distal portion 62 of shaft 60 and folded implant 10, as shown in FIG. 1. In some examples, assembly of implant system 1 also includes loading one or more filaments onto implant 10 prior to advancement of sheath 72 over implant 10. In such cases, the filament may pass through one or both ends of sheath 72 when implant system 1 is assembled. In some examples, the implant delivery instrument 50 may be preassembled with the implant 10 pre-positioned within the sheath 72. In some examples, filaments may be coupled to an implant after the implant has been loaded into a sheath of an implant delivery instrument, for example, as shown in FIG. 19B.

In one aspect, a method includes implantation of implant 110 of implant system 100 into a patient. Optionally, the method may include the initial assembly of implant system 100. To assemble implant system 100, sheath 172 of implant delivery instrument 150 may be retracted, or is already retracted, and implant 110 may be prepared (e.g., folded, rolled, etc.) and coupled to implant delivery instrument 150 in the same manner as described above for implant system 1 and as shown in FIG. 1. Assembly of implant system 100 is then complete. In some examples, implant system 100 is initially provided in an assembled condition. To prepare for placement of implant 110 in the patient, an anchor (not shown) may be secured to tissue of the patient at a location proximate to a target site intended for receipt of the implant. The anchor may be any biocompatible anchor capable of receiving and holding a filament and capable of being secured to soft or hard tissue. For example, anchors may be secured to hard tissue with knotted-type anchors such as Iconix or AlphaVent by Stryker^{®}. Further, in other examples, anchors may be secured to hard tissue with knotless type anchors such as Iconix Knotless and Omega by Stryker^{®}. Other anchors that may be used in the various aspects disclosed herein include PEEK suture anchors, all-suture anchors, biocomposite suture anchors, buttons, interference screws, or any other type of anchor commonly used to secure sutures or other filament to tissue. For soft tissue fixation, anchors may include, for example, tacks, staples, nails, retention screws and/or any other mechanical fastener suitable for securing to soft tissue. A filament 112 may be secured to the anchor before or after the anchor is secured to the tissue of the patient. In some examples, filament 112 may be secured directly to the tissue. In other examples, an anchor is provided with a filament pre-assembled to the anchor. Either before or after securement of the anchor to the tissue, filament 112 may also be passed through opening 138 of fitting 120, as shown in FIG. 7. With these steps completed, filament 112 is secured to the anchor, which is in turn secured to the tissue, and filament 112 is passes through opening 138 of fitting 120.

Implant delivery instrument 150 with implant 110 attached is then directed through a prepared access, i.e., portal into the patient to position a distal end 178 of instrument 150 at the target site within the patient, i.e., the location where the implant 110 is to be deployed within the patient. In some examples, this may be in a subacromial space within the shoulder. Then, the base 170 of sheath 172 is retracted to expose implant 110, as shown in FIG. 5. Although filament 112 extends from within sheath 172 to a space outside of sheath 172 as shown in FIG. 3, filament 112 does not interfere with retraction of sheath 172 as slit 174 (as shown in FIG. 4) provides space for filament 112 to extend laterally out of sheath 172 while sheath 172 is retracted. Balloon 140 of implant 110 is then inflated via input of air or fluid through operator port 158.

While filament 112 continues to pass through opening 138, a free end of filament 112 may then be pulled and tensioned and then secured to fitting 120 (e.g., by tying a knot using filament 112) so that implant 110 is fixed relative to the anchor. Securement of balloon 140 to the tissue via filament 112 may be completed before or after inflation of balloon 140. Implant delivery instrument 150 may then be decoupled from implant 110 and removed from the patient at any time after inflation. Decoupling of implant delivery instrument 150 from implant 110 (implant 110 being shown in isolation in FIG. 7), may cause a seal to engage within a pathway extending from fitting 120 to interior inlet 142, thereby preserving the inflated condition of the balloon 140. The features of the implant delivery instrument 150 and implant 110 that cause the balloon 140 to be sealed may be as described elsewhere in the present disclosure. In some examples, rotation of knob 154 from a closed position to an open position may cause withdrawal of an inner shaft (e.g., similar to inner shaft 61) of implant delivery instrument 150 and release of a locking feature between implant delivery instrument 150 and implant 110. When the inner shaft is withdrawn within shaft 160, a plug (e.g., similar to plug 63) positioned at the end of the inner shaft may be withdrawn in conjunction with the inner shaft until it is prevented from further withdrawal by an inner surface of implant 110 (e.g., a surface of interior inlet 142) causing the plug to dislodge from the inner shaft. As a result, the plug then seals off the internal volume of balloon 140. The procedure is completed with the closure of the portal into the patient.

In one aspect, a method includes implantation of implant 210 into a patient. Such method may be performed through performance of the same steps as described above for implantation of implant 110 used with implant delivery instrument 150.

In one aspect, a method includes implantation of implant 310 of implant system 300 into a patient. Optionally, the method may include the initial assembly of implant system 300. In some examples of the method of assembly of implant system 300, there may be an initial step of attachment of the inner body 322 onto the balloon 340. In other examples, inner body 322 is assembled with balloon 340 prior to delivery to a surgical site. With inner body 322 of implant 310 attached to balloon 340, sheath 372 of implant delivery instrument 350 may be retracted, or is already retracted, and implant 310 may be prepared (e.g., folded, rolled, etc.) and coupled to implant delivery instrument 350 in the same manner as described above with respect to implant system 1. This includes, when implant 310 is attached to implant delivery instrument 350, advancement of sheath 372 toward the distal end of shaft 360 over the folded implant 310 to enclose the implant 310 within sheath 372. Assembly of implant system 300 may also include loading a filament 312 onto fitting 320 of implant 310. In such cases, the method may continue with attachment of outer body 332 of implant 310 onto shaft 360 as shown in FIG. 9. Outer body 332 may be snapped into place on shaft 360 from one side, as shown, where central channel 335 snaps over shaft 360. Then, a filament 312, such as a filament already secured in vivo onto tissue within a patient, may be passed through an opening in outer body 332, such as one of openings 338, 339, as shown in FIG. 9. In some examples, a free end of filament 312 is passed into a lumen of sheath 372 through distal end of sheath 372 and out of the lumen through base 370, and then through an opening in outer body 332. Guide 390 is then snapped onto shaft 360 between handle (not shown) and outer body 332 as shown in FIG. 10. Guide 390 is then pushed along shaft 360 until in contact with outer body 332. Guide 390 thereafter pushes outer body 332 through base 370 into sheath 372 until outer body 332 snaps onto inner body 322, as shown in FIG. 11. At this juncture, filament 312 extends from an anchor affixed to tissue within the patient, out of the body of the patient, into sheath 372, through opening 338, 339 and through base 370 to a free end, thereby accessible to a user. Alternatively, filament 312 may be positioned through implant delivery instrument 350 as described but secured or otherwise attached to the tissue after loading through implant delivery instrument 350. Implant system 300 is then ready for use in placement of implant 310 into an implantation location in the patient.

In some examples of the method of implantation of implant 310, the method includes the assembly steps described above. In other examples, the method begins with implant system 300 in an assembled condition, i.e., preassembled. To prepare for placement of implant 310 in the patient, an anchor (not shown) is secured to tissue of the patient at a location proximate to a target site for receipt of the implant 310. The anchor may be any biocompatible anchor capable of receiving and holding a filament and capable of being secured to soft or hard tissue. Filament 312 may be secured to the anchor before or after the anchor is secured to the tissue of the patient. With these steps completed, filament is secured to the anchor on the tissue and also passes through an opening 338, 339 of fitting 320, where fitting includes the combined inner body 322 and outer body 332 as shown in FIG. 11. Implant delivery instrument 350 with implant 310 attached is then directed through a prepared access, i.e., portal into the patient to position a distal end of instrument 350 at the target site within the patient, i.e., the location where the implant 310 is to be deployed within the patient. In some examples, this may be in a subacromial space within the shoulder. At this juncture, the base 370 of sheath 372 is retracted to expose implant 310. Balloon 340 of implant 310 is then inflated, e.g., via input of air or fluid through operator port 158. During this process, filament 312 remains positioned such that it passes through opening 338, 339. In this manner, a free end of filament 312 opposite an end of the filament that is secured to tissue may then be tensioned and secured to fitting 320 so that implant 310 is fixed relative to the anchor. Implant delivery instrument 350 may be removed from the patient at any time after inflation. Removal of instrument 350 from implant 310 causes a seal to engage within a pathway extending from fitting 320 to inlet 342, thereby preserving the inflated condition of the balloon 340. The features of the instrument 350 and implant 310 that cause the balloon 340 to be sealed may be as described elsewhere in the present disclosure. Implant 310 with implant delivery instrument 350 removed is shown in FIG. 12. The procedure is completed with the closure of the portal into the patient.

In one aspect, a method includes implantation of implant 410 into a patient. An implant system of this aspect may include implant delivery instrument 50 shown in FIG. 1 and implant 410. Optionally, the method may include an initial step of implant system assembly. For assembly, sheath 72 of implant delivery instrument 50 may be retracted, or is already retracted, and implant 410 may be prepared and received on implant delivery instrument 50 in the same manner as described above for implant system 1. In some examples, a further assembly step may be an initial attachment of central body 422 to balloon 440 prior to attachment of implant 410 onto implant delivery instrument 50. Assembly of the implant system is then complete. In some examples, the implant system is initially provided in an assembled condition.

With the implant system in an assembled condition, the method proceeds with an anchor (not shown) being secured to tissue of the patient at a location proximate to a target site for receipt of the implant. The anchor may be any biocompatible anchor capable of receiving and holding a filament and capable of being secured to soft or hard tissue. A filament 412 is attached at one end to the anchor and has an opposite free end that is accessible for use. In some examples, filament 412 is attached directly to the tissue. In preparation for use of connection portion 432, a strap 433 of connection portion 432 may be passed through the opening in base 434, as shown in FIG. 14. The free end of filament 412 is then passed through an opening of the plurality of openings 438 of connection portion 432.

In some aspects, the selection of an opening from among the plurality of openings 438 may be based, in part, on a desired degree of fixation of implant 310 to the tissue of the patient. For example, an opening proximate to second end 432B of connection portion 432 may be used for a tight fixation having a reduced length of strap 433 between central body 422 (to which connection portion 432 is coupled) and the filament 412 affixing the connection portion 432 to the anchor or tissue of the patient. In another example, an opening proximate to the first end 432A of connection portion 432 may be used for a loose fixation having an increased length of strap 433 between central body 422 and the filament 412 affixing the connection portion 432 to the anchor or tissue of the patient. In this way, each of the plurality of openings 438 may provide respective degrees of fixation of the implant 310 to the tissue of the patient, allowing for optimized fixation of the implant 310 regardless of differences in balloon size and/or patient anatomy.

Implant delivery instrument 50 with implant 410 attached is then directed through a prepared access, i.e., portal into the patient to position a distal end 78 of instrument 50 at the target site within the patient, i.e., the location where the implant 410 is to be deployed within the patient. In some examples, this may be in a subacromial space within the shoulder. At this juncture, the base 70 of sheath 72 is retracted to expose implant 410. Balloon 440 of implant 410 is then inflated, e.g., via input of air or fluid through operator port 58. Implant delivery instrument 50 may be removed from the patient at any time after inflation. Removal of implant delivery instrument 50 from implant 410, causes a seal to engage within a pathway extending from central body 422 to inlet 442, thereby preserving the inflated condition of the balloon 440. The features of the implant delivery instrument 50 and implant 410 that cause the balloon 440 to be sealed may be as described elsewhere in the present disclosure.

Connection portion 432, with filament 412 passing through an opening from among the plurality of openings 438 of connection portion 432, is then advanced into the surgical site and positioned over receiving portion 425 of central body 422, and strap 433 is tightened to secure connection portion 432 to central body 422, as shown in FIG. 15. At this juncture, filament 412 remains secured to the patient at one end, continues to be received through an opening of the plurality of openings 438 of connection portion 432 now secured to implant, and has a free end that extends out of the patient for further use in securement of the implant 410. This configuration is shown in FIG. 15. Filament 412 may then be tensioned and secured to connection portion 432 or another part of fitting 420 to secure implant 410 to the tissue within the patient. The procedure is completed with the closure of the portal into the patient. In a variation of this aspect, the method may be performed without the use of connection portion 432 and instead, filament 412, secured to tissue at one end, may be wrapped around receiving portion 425 to secure implant 410 to the patient.

In one aspect, a method includes implantation of implant 510, shown in FIG. 16, into a patient. Such method may include any number of steps as described above for the method of implantation of implant 110, particularly until implant 510 is advanced to and positioned at a desired implantation location within the patient. With implant 510 in a desired implantation location, a filament 512 secured to tissue of the patient is passed around narrow central portion 525 of inflation fitting 520 and secured to implant 510. Implant 510 may be inflated using implant delivery instrument 50, 150 before or after securement of implant 510 to the tissue via tensioning and securement of filament 512 to implant 510. Once implant 510 is secured to the tissue and the balloon 540 is inflated, implant delivery instrument 50, 150 is withdrawn to cause balloon 540 to be sealed and then removed from the patient. The procedure is completed with the closure of the portal into the patient.

In one aspect, a method includes implantation of implant 610, shown in FIGS. 17A-B, into a patient. Such method may include any number of steps as described above for the method of implantation of implant 110, particularly steps up to advancement of implant 610 into a patient so that implant 610 is positioned at a desired implantation location in the patient. In at least some examples, prior to placement within the patient, implant 610 including fixation members 612A-D may be loaded onto implant delivery instrument 50 as part of assembly of the implant system prior to use. With implant 610 in a desired implantation location, one or more filaments secured to tissue within the patient (e.g., coupled to anchors coupled to tissue within the subacromial space within the patient) may be passed through a loop 615A-D of a respective fixation member 612A-D. The one or more filaments may then be then tied or otherwise fixed to one or more loops 615A-D to secure implant 610 to the anchor (not shown). In one example, four filaments (not shown) are used for fixation, with each loop 615A-D receiving one respective filament. Fixation is completed via tensioning of each filament followed by securement of the respective filaments to loops 615A-D (e.g., by tying the filament to the loop 615A-D. Implant 610 may then be inflated using implant delivery instrument 50, 150. The implant delivery instrument 50, 150 may then be decoupled from the implant 610, and the implant delivery instrument 50, 150 may be withdrawn from the patient. As discussed above, decoupling of the implant 610 from the implant delivery instrument 50, 150 may cause balloon 640 to be sealed. The procedure may be completed with the closure of the portal into the patient.

In one aspect, a method includes implantation of implant 710, shown in FIG. 18, into a patient. Such method may include any number of steps as described above for the method of implantation of implant 110, particularly until implant 710 is advanced to and positioned at a desired implantation location in the patient, for example, using implant delivery instrument 50 or 150. With implant 710 in a desired implantation location, one or more filaments secured to tissue of the patient (e.g., coupled to one or more anchors coupled to tissue within the subacromial space within the patient) are wrapped around a neck portion 761 of a respective fixation member 712A-D. The filaments may then be tensioned then tied or otherwise fixed to fixation member 712A-D to secure implant 710 to the one or more anchors (not shown) at the implantation site. In one example, four filaments (not shown) are used for securement, with each fixation member 712A-D being secured by a respective filament. The implant delivery instrument 50, 150 may then be decoupled from the implant 710, and the implant delivery instrument 50, 150 may be withdrawn from the patient. As discussed above, decoupling of the implant 710 from the implant delivery instrument 50, 150 may cause balloon 740 to be sealed. The procedure may be completed with the closure of the portal into the patient.

FIG. 19A illustrates an exemplary implant system 800 in which implant 810 has been loaded into sheath 872 of an implant delivery instrument 850 (which may be substantially similar to implant delivery instruments 50, 150). Unless otherwise indicated, like reference numerals relating to implant delivery instrument 850 refer to like elements of implant delivery instrument 50 shown in FIG. 1, but within the 800-series of numerals. In some examples, implant 810 may be preloaded into instrument implant delivery instrument 850 during manufacturing/assembly, while in other examples, implant 810 may be loaded into implant delivery instrument 850 in advance of or during a surgical procedure (e.g., by a member of the surgical team). Filaments 802A-D may each be passed through a respective opening 838A-D in a respective tab 812A-D of implant 810. Before or after passing filaments 802A-D respectively through openings 838A-D, filaments 802A-D may be secured to anchors at an implantation site in a patient. Implant 810 may then be inserted into sheath 872 of implant delivery instrument 850, such that filaments 802A-D extend into a distal end 878 of sheath 872, pass through openings 838A-D, and extend back out of the distal end 878 of sheath 872. Inserting implant 810 into sheath 872 may include retracting sheath 872, coupling implant 810 to shaft 860 and advancing sheath 872 over implant 810 with implant 810 rolled or folded. In some examples, filaments 802A-D may each be passed through a respective opening 838A-D in a respective tab 812A-D of implant 810 while implant 810 is coupled to implant delivery instrument 850 with sheath 872 retracted. Then, sheath 872 may be advanced over implant 810 with filaments 802A-D extending from the distal end 878 of sheath 872. With implant 810 in sheath 872 of implant delivery instrument 850, implant 810 may then be inserted into the patent to the implantation site using implant delivery instrument 850, with implant 810 guided by filaments 802A-D (e.g., by applying tension on the free ends of filaments 802A-D while moving implant delivery instrument 850 toward the implantation site.). Sheath 872 may then be retracted to expose implant 810, which may unfurl, unfold, unroll, etc. at the implantation site. Filaments 802A-D may then be tensioned and secured or coupled (e.g., tied) to tabs 812A-D, tied together or coupled with knotless fixation methods (e.g., finger traps), or secured back to an anchor in order to secure implant 810 to tissue 805 at the implantation site. The balloon 840 may then be inflated using implant delivery instrument 850, which may then be decoupled from balloon 840 and removed from the implantation site. In some examples, balloon 840 may be inflated or partially inflated before implant 810 is secured to tissue 805.

It should be understood that, as discussed above with respect to FIG. 19A, in various aspects disclosed herein, filaments may be passed through an implant before the implant is coupled to the implant delivery instrument. In other aspects, filaments may be passed through the implant when the sheath is retracted and the implant is coupled to the implant delivery instrument before advancing the sheath over the implant. In other aspects, and as discussed below with respect to implant delivery instrument 950 of FIG. 19B, the implant delivery instrument 950 may include features allowing filaments to pass through the implant while the implant is positioned within the sheath.

The example implant delivery instrument 950 of an implant system 900 FIG. 19B may be substantially similar to the implant delivery instrument 850 of FIG. 19A, except as shown and described herein. Unless otherwise indicated, like reference numerals relating to implant delivery instrument 950 refer to like elements of implant delivery instrument 850 shown in FIG. 19A, but within the 900-series of numerals. The implant delivery instrument 950 of FIG. 19B includes openings or windows 975A-D in sheath 972 positioned to provide access to tabs 812A-D when balloon 940 is loaded into sheath 972. This may allow filaments 902A-D (which may each already be coupled to a respective anchor at the implantation site) to be passed through openings 838A-D in tabs 812A-D with balloon 940 within sheath 972 (e.g., balloon 940 may be preloaded into sheath 972 during a manufacturing or assembly process). Windows 975A and 975B may extend to the distal end 978 of sheath 972, while windows 975C and 975D are positioned at a proximal portion of sheath 972. A first slot 974 extends between windows 975A and 975D on a first side of sheath 972, and a second slot 974 extends between windows 975B and 975C on a second side of sheath 972. When sheath 972 is retracted at the implantation site, filaments 902A and 902D may pass through a respective slot 974 and a respective window 975B, 975A. Windows 975A, 975D and windows 975B, 975C may be considered part of a respective slot 974. For example, each slot 974 may be considered to include widened portions referred to as windows 975A, 975D and windows 975B, 975C, respectively. The widened portions (i.e., windows 975A-D) are configured to be positioned adjacent to the tabs 812A-D when the shaft 960 is coupled to the implant 810. After the filaments 902A-D are passed through a respective opening 838A-D, the implant 810 may be inserted into the patient to the implantation site of the patient, sheath 972 may be retracted, filaments 902A-D may be tensioned and tied, and balloon 940 may be inflated as discussed above with respect to FIG. 19A. Though four filaments 902A-D, each respectively passing through one of the openings 838A-D, and four windows 975A-D in sheath 972 are shown, it should be understood that implant system 900 may have more or fewer openings 838 and windows 975 and/or that more or fewer filaments 902 may be used to secure implant 810 to the implantation site. Further, a filament 902 may be passed through more than one opening 838. For example, a first filament may be passed through openings 838B and 838A, and a second filament may be passed through openings 838C and 838D.

In some examples, implant 810 may be coupled to implant delivery instrument 950 with sheath 972 retracted when the filaments 902A-D are each passed through a respective opening 838A-D in a respective tab 812A-D of implant 810. Then, sheath 972 may be advanced over implant 810 with filaments 902A and 902D passing through (while extending out of) the respective slot 974 to a proximal portion of the sheath. In such examples, implant delivery instrument 950 may not include windows, as the filaments 902A-D are passed through the respective openings 838A-D while sheath 972 is retracted. The slots 974 may instead extend to the distal end 978 of sheath 972. Filaments 902B, 902C may also extend out of the slot near the distal end 978 of sheath 972. As implant delivery instrument 950 and implant 810 are guided along filaments 802A-D to the implantation site, the ends of filaments 902A-D couped to anchors at the implantation site may extend out of the distal end 978 of 972, and the free ends of filaments 902A-D may extend out of the slots. The free ends of 902A-D may be tensioned as the implant delivery instrument 950 is guided to the implantation site.

Thus, as discussed above, an implant system may include an implant and an implant delivery instrument, which may be configured to receive the implant in a collapsed configuration. The implant may include a balloon including a body and an inlet being in fluid communication with an internal cavity of the body, a fitting attached to the inlet of the balloon and configured to receive a fluid for inflating the body of the balloon, and a connection feature configured to receive a filament therethrough. The connection feature may be or may include, for example, a portion of the fitting or a peripheral portion of the implant, such as a tab, a channel, a strap, a loop member coupled to a strap, etc. The connection feature may include an opening extending therethrough. The implant delivery instrument may include a sheath configured to receive the implant in the collapsed configuration and a shaft configured to be coupled to the fitting. The shaft may include a conduit configured to provide the fluid for inflating the body of the balloon. The sheath may be retractable relative to the shaft to release the implant from the sheath. In some examples, the sheath includes a slot configured to allow a filament coupled to the connection feature extend out of a side of the sheath, the slot extending to a distal end of the sheath. The slot may include a widened portion configured to be positioned adjacent to the connection feature when the conduit interfaces with the fitting

FIGS. 19C-19I illustrate a method of implanting implant 810 using implant delivery instrument 950 of FIG. 19B. FIG. 19C shows an implantation site including tissue 2105 having four fixation anchors 2103A-D coupled thereto. It should be understood that "tissue," as used herein, refers generally to tissue in the joint and may include, bone, tendons, muscle, fascia, etc., unless otherwise specified. The fixation anchors 2103A-D may be the same as or similar to the other fixation anchors discussed herein. Fixation anchors 2103A and 2103C are coupled by a first filament 2191 and fixation anchors 2103B and 2103D are coupled by a second filament 2191. The fixation anchors 2103A-D and filaments 2191 may have been placed in the implantation site earlier in the surgical procedure and used for other purposes (e.g., for a double row repair). Shuttles 2193A-B may be preloaded in respective openings in fixation anchors 2103A-B (which may be medial anchors). Shuttles 2193A-B may be filaments (sutures, etc.) with loops 2194A-B at one end for receiving another filament or suture. Another filament may be pulled through the loop 2194A-B, and the opposite end of the shuttle 2193A-B may be pulled to pull (or shuttle) the other filament or suture through the opening in fixation anchors 2103A-B. Shuttles may similarly be used to pull filaments though openings or eyelets in the various implants described herein. For example, shuttles may be preloaded into openings 838A-D to assist in pulling filaments though openings 838A-D to slidably couple implant 810 to the filaments. Filaments 2102C-D (sutures, etc.) may be respectively coupled to fixation anchors 2103C-D. Filaments 2102C-D may respectively include a self-locking and/or knotless fixation features. For example, Filaments 2102C-D are shown to include finger traps 2189C-D. Each finger trap 2189C-D may be a structure configured to receive another filament (or in this case, the end of the same filament) and to tighten around the filament when the filament is pulled such that the finger trap 2189C-D resists the filament from being removed from the finger trap 2189C-D. Thus the finger traps 2189C-D provide tensionable, knotless, securing of filaments without the need to tie the filaments together. As discussed above, other knotless and/or self-locking features may be used in place of finger traps 2189C-D.

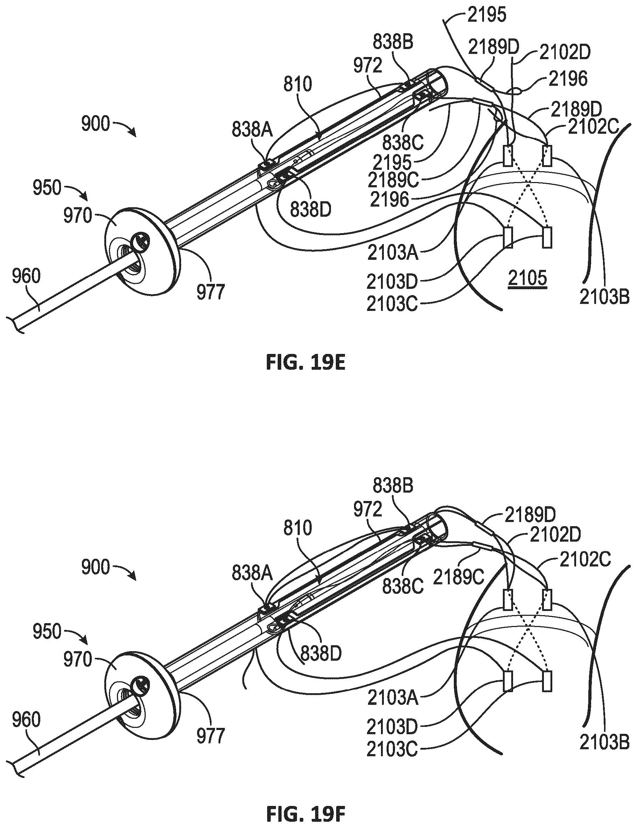

As shown in FIG. 19D, filament 2102C is passed through openings 838D and 838C and through loop 2194B of shuttle 2193B. Similarly, filament 2102D may be passed through openings 838A and 838B and through loop 2194A of shuttle 2193A. Shuttles 2193A and 2193B may then be pulled through the respective openings in fixation anchors 2103A and 2103B to pull the filaments 2102D and 2102C through the respective openings in fixation anchors 2103A and 2103B. As shown in FIGS. 19D and 19E, finger trap shuttles 2195 (e.g., similar to shuttles 2193A-B) with loops 2196 extend through finger traps 2189C and 2189D. Finger traps 2189C and 2189D may each be preloaded with the finger trap shuttle 2195. The ends of filaments 2102C-D may be passed through respective loops 2196, and finger trap shuttles 2195 may pull filaments 2102C-D through finger traps 2189C-D, respectively.

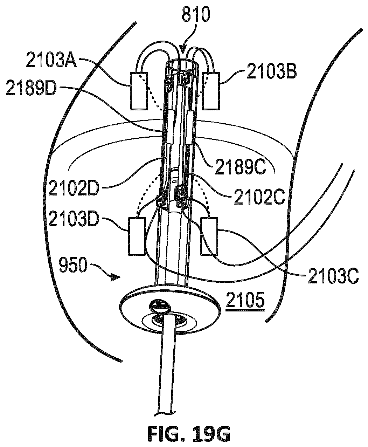

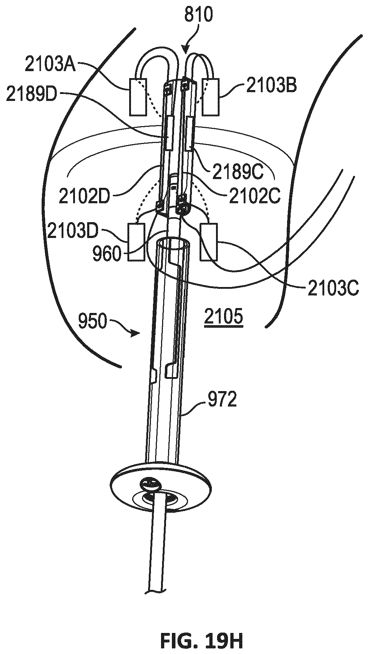

As shown in FIG. 19F, after being pulled through finger trap 2189C, the end of filament 2102C is pulled back through openings 838C and 838D, and after being pulled through finger trap 2189D, the end of filament 2102D is pulled back through openings 838B and 838A. As shown in FIG. 19G, the ends of filaments 2102C-D are continued to be pulled through the respective openings 838A-D. Filaments 2102C-D are used to guide implant delivery instrument 950 to the implantation site as they are pulled through the respective openings 838A-D. Thus, implant 810 is delivered to the implantation site by advancing implant 810 along filaments 2102C-D such that the filaments 2102C-D pass through openings 838B and 838A as implant 810 is moved toward the implantation site. With the implant delivery instrument 950 and implant 810 positioned as shown in FIG. 19G, the sheath 972 of implant delivery instrument 950 may be retracted to expose implant 810. Implant 810 may then unroll or unfold, for example, to a flattened configuration. FIG. 19H shows implant delivery instrument 950 with sheath 972 retracted. Implant 810 remains coupled to shaft 960. While implant 810 is shown in a collapsed (e.g., rolled, folded, etc.) configuration in FIG 19H, it should be understood that in some examples, implant 810 may begin unrolling or unfolding (e.g., to a flattened configuration) as soon as implant 810 is released from sheath 972. In other examples, balloon 840 may be partially inflated (e.g., via a fluid conduit in shaft 960) after implant 810 is released from sheath 872, which may cause implant 810 to unroll more quickly and/or completely. As discussed above, the free ends of filaments 2102C and 2102D, which pass through openings 838D and 838A, respectively, may extend from the sheath through windows 975C and 975D respectively. As sheath 972 is retracted from the position shown in FIG. 19G to the position shown in FIG. 19H, the free ends of filaments 2102C and 2102D may pass through the respective slot 974 on the respective side of the sheath 972 to the distal end 978 of sheath 972, exposing implant 810, which may then unfold or unroll to a flattened or expanded configuration.

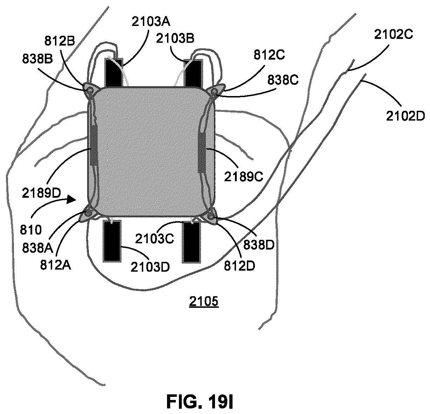

As shown in FIG. 19I, the implant 810 has been expelled from the implant delivery instrument 950 and has unfolded or unrolled to a flattened or expanded configuration, and the implant delivery instrument 950 has been removed from the implantation site. By continuing to pull filaments 2102C and 2102D through finger traps 2189C and 2189D, respectively, filaments 2102C and 2102D may be tensioned to secure implant 810 in place at the implantation site. Once filaments 2102C and 2102D are pulled through finger traps 2189C and 2189D, respectively, and finger traps 2189C and 2189D are tensioned, finger traps 2189C and 2189D will hold the tension and resist loosening of filaments 2102C and 2102D. In tensioning filaments 2102C and 2102D, finger traps 2189C and 2189D may be pulled back through opening 838C and opening 838B, respectively, as shown in FIG. 19I. Thus, as shown in FIG. 19I, once filaments 2102C and 2102D are tensioned and implant 810 is secured, filament 2102C, which is coupled to fixation anchor 2103C, extends through opening 838D in tab 812D, opening 838C in tab 812C, an opening in fixation anchor 2103B, and back through opening 838C, and is secured by finger trap 2189C, and filament 2102D, which is coupled to fixation anchor 2103D, extends through opening 838A in tab 812A, opening 838B in tab 812B, an opening in fixation anchor 2103A, and back through opening 838B, and is secured by finger trap 2189D. The ends of filaments 2102C-D can then be cut or tied off, as the finger traps 2189C-D secure filaments 2102C-D and resist the ends of the filaments 2102C-D pulling out. Balloon 840 of implant 810 may be inflated at any time after sheath 972 is withdrawn to expose implant 810, before, while, or after fully securing implant 810 to fixation anchors 2103A-D. While the method shown FIGS. 19C-19I is illustrated with respect to implant 810, it should be understood that similar methods may be used to implant the various implants, balloons, and augments described herein. Shuttles as described above may be used any time a filament is pulled through an opening (e.g., in a fixation anchor or in an implant), and finger traps as described above may be used in place of knots and other fixation methods described herein. In some examples, an implant may be advanced into the patient to the implantation site in an implant delivery instrument without having first passed filaments through portions of the implant. For example, filaments may be passed through portions of the implant after the implant is within the patient. As discussed above, the implant may include openings with shuttles preloaded into the openings to assist in pulling filaments through the openings while the implant is inside the patient. It should be appreciated that shuttles may similarly be used to pull filaments through any openings or eyelets disclosed herein.

FIGS. 19J-19P illustrate a method of implanting implant 810 using implant delivery instrument 950 of FIG. 19B in a manner similar to the method shown in FIGS. 19C-19I. FIG. 19J shows an implantation site including tissue 2205 four fixation anchors 2203A-D coupled thereto. The fixation anchors 2203A-D may be the same as or similar to the other fixation anchors discussed herein. Fixation anchors 2203A and 2203C are coupled by a first filament 2291 and fixation anchors 2203B and 2203D are coupled by a second filament 2291. The fixation anchors 2203A-D and filaments 2291 may have been placed in the implantation site earlier in the surgical procedure and used for other purposes (e.g., for a double row repair). Shuttles 2293A-B may be preloaded in respective openings in fixation anchors 2203A and 2203B (which may be medial anchors). Shuttles 2293A-B may be filaments (sutures, etc.) with loops 2294A-B at one end for receiving another filament or suture. Another filament may be pulled through the loop 2294A-B, and the opposite end of the shuttle 2293A-B may be pulled to pull (or shuttle) the other filament or suture through the opening in fixation anchors 2203A-B. Filaments 2202C-D (sutures, etc.) may be respectively coupled to fixation anchors 2203C-D. Filaments 2202C-D may respectively include finger traps 2289C-D. Each finger trap 2289C-D may be a structure configured to receive another filament (or in this case, the end of the same filament) and to tighten around the filament when the finger trap 2289C-D is tensioned such that the finger trap 2289C-D resists the filament from being removed from the finger trap 2289C-D. Thus, the finger traps 2289C-D provide tensionable, knotless, securing of filaments without the need to tie the filaments together. As discussed above, other knotless and/or self-locking features may be used in place of finger traps 2289C-D. Filaments 2202C'-D' (sutures, etc.) may also be respectively coupled to fixation anchors 2203C-D. for example, filament 2202C' is passed through loop 2294B of shuttle 2293B. In some examples, filaments 2202C'-D' may not be included, and instead a second ends of filaments 2202C-D may be used in place of filaments 2202C'-D'. Said another way, reference numbers 2202C-D and 2202C'-D' may refer to four separate filaments, or reference numbers 2202C and 2202C' may refer to two ends of the same (first) filament and reference numbers 2202D and 2202D' may refer to two ends of the same (second) filament.

As shown in FIG. 19K, filament 2202C' is passed through loop 2294B of shuttle 2293B. Similarly, filament 2202D' is passed through loop 2294A of shuttle 2293A. Shuttles 2293A and 2293B may then be pulled through the respective openings in fixation anchors 2203A and 2203B to pull the filaments 2202D' and 2202C' through the respective openings in fixation anchors 2203A and 2203B.

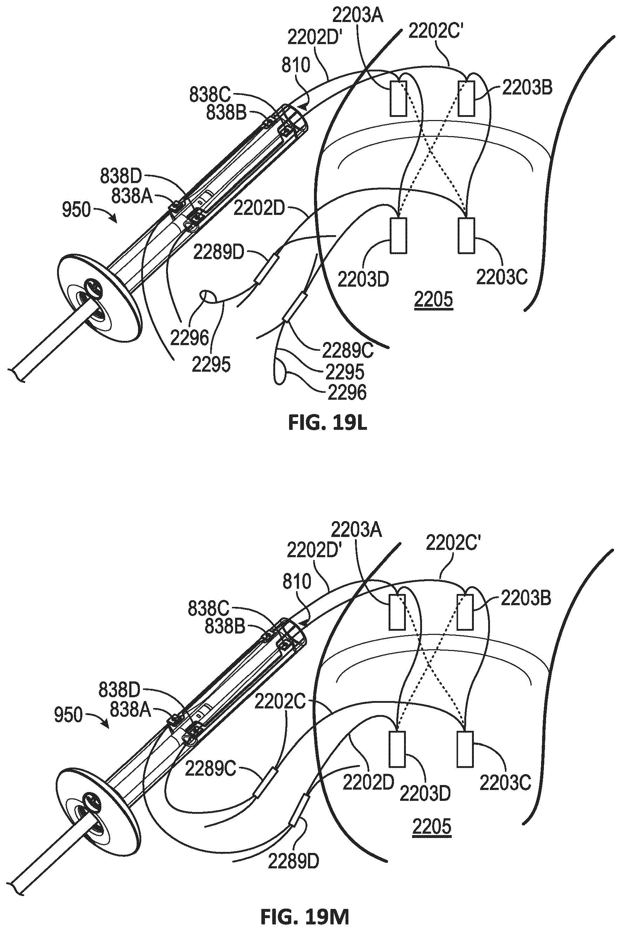

As shown in FIG. 19L, the free end of filament 2202C' is passed through opening 838C in tab 812C of implant 810 and through opening 838D of tab 812D, while the implant 810 is positioned in the implant delivery instrument 950 (e.g., similar to as shown in FIG. 19D). The free end of filament 2202D' is passed through opening 838B in tab 812B of implant 810 and through opening 838A of tab 812A, while the implant 810 is positioned in the implant delivery instrument 950. This may be performed outside of the patient. For example, implant delivery instrument 950 may be positioned outside the patient, and filaments 2202C' and 2202D' may extend out of the patient and be fed through the respective openings 838A-D. In other examples, implant delivery instrument 950 may be advanced into the patient, with implant 810 inside sheath 972, and the free ends of filaments 2202C' and 2202D' may be fed through the respective openings 838A-D with implant delivery instrument 950 and implant 810 inside the patient. Finger trap shuttles 2295 with loops 2296 may each be preloaded into one of finger traps 2289C and 2289D. The ends of filaments 2202C'-D' may be passed through respective loops 2296, and finger trap shuttles 2295 may then be pulled through finger traps 2289C-D, respectively, to pull the free ends of filaments 2202C'-D' through finger traps 2289C-D, respectively, as shown in FIG. 19M.

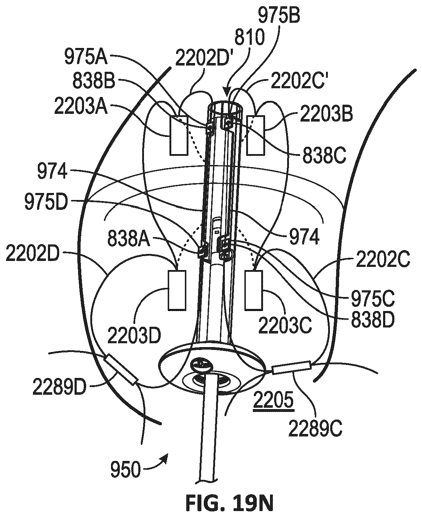

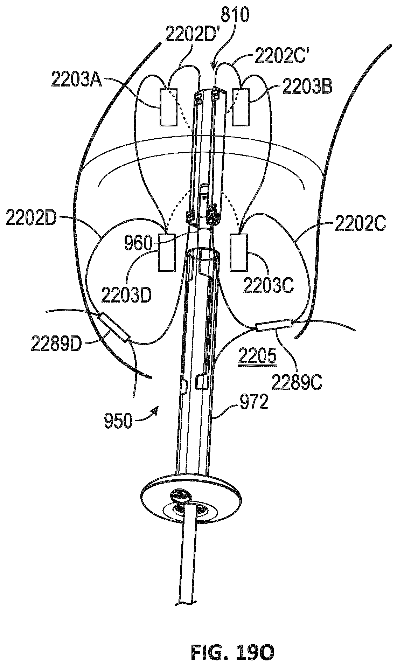

As shown in FIG. 19N, after the ends of filaments 2202C'-D' are pulled through finger traps 2289C-D, filaments 2202C'-D' are continued to be pulled through finger traps 2289C-D, respectively, to tension filaments 2202C'-D'. Filaments 2202C'-D' may be used to guide implant delivery instrument 950 to the implantation site as they are pulled through the finger traps 2289C-D. With implant delivery instrument 950 and implant 810 positioned as shown in FIG. 19N, the sheath 972 of implant delivery instrument 950 may be retracted to expose implant 810. Implant 810 may then unroll or unfold, for example, to a flattened or expanded configuration. FIG. 19O shows implant delivery instrument 950 with sheath 972 retracted. Implant 810 remains coupled to shaft 960. While implant 810 is shown in a collapsed (e.g., rolled) configuration in FIG. 19O, it should be understood that in some examples, implant 810 may begin unrolling or unfolding as soon as implant 810 is released from sheath 972. In other examples, balloon 840 may be partially inflated (e.g., via a fluid conduit in shaft 960) after implant 810 is released from sheath 872, which may cause implant 810 to unroll more quickly and/or completely. As discussed above, the free ends of filaments 2202C' and 2202D', which pass through openings 838D and 838A, respectively, may extend from the sheath through windows 975C and 975D respectively. As sheath 972 is retracted, the free ends of filaments 2102C and 2102D may pass through the respective slot 974 on the respective side of the sheath 972 to the distal end 978 of sheath 972.

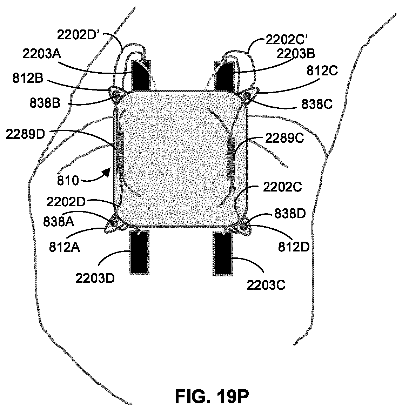

As shown in FIG. 19P, the implant 810 has been expelled from the implant delivery instrument 950 and has unfolded or unrolled to a flattened or expanded configuration, and the implant delivery instrument 950 has been removed from the implantation site. By continuing to pull filaments 2202C' and 2202D' through finger traps 2289C and 2289D, respectively, filaments 2202C' and 2302D' may be tensioned to secure implant 810 in place at the implantation site. As discussed above, finger traps 2289C and 2289D may be self-locking, such that once filaments 2202C and 2202D are pulled through finger traps 2289C and 2289D, respectively, finger traps 2189C and 2189D will hold the tension and resist loosening of filaments 2202C' and 2202D'. Thus, as shown in FIG. 19P, once filament 2202C' is tensioned to secure implant 810, filament 2202C', which is coupled to fixation anchor 2203C, extends through an opening in anchor 2203A and an opening 838C in tab 812C, and is secured by finger trap 2289C of filament 2202C (which is coupled to anchor 2203C and extends through opening 838D of tab 812D). Once filament 2202C' is tensioned to secure implant 810, filament 2202D', which is coupled to fixation anchor 2103D, extends through an opening in anchor 2203A and opening 838B in tab 812B and is secured by finger trap 2289D of filament 2202D (which is coupled to anchor 2203D and extends through opening 838A of tab 812A). The ends of filaments 2202C'-D' can then be cut or tied off, as the finger traps 2289C-D secure filaments 2202C'-D' and resist the ends of the filaments 2202C'-D' pulling out (as the finger traps 2289C-D are self-locking when tensioned). The free end of filaments 2202C-D and finger traps 2289C-D may be pulled through openings 838C-D. Implant 810 is thus secured by (a) filament 2202C', which is coupled to fixation anchor 2203C, passes through an opening in fixation anchor 2203B and opening 838B in tab 812B and is secured by finger trap 2289C, which is coupled via filament 2202C to fixation anchor 2203C through opening 838C in tab 812C, and (b) filament 2202D', which is coupled to fixation anchor 2203D, passes through an opening in fixation anchor 2203A and opening 838A in tab 812A and is secured by finger trap 2289D, which is coupled via filament 2202D to fixation anchor 2203D through opening 838D in tab 812D. Balloon 840 of implant 810 may be inflated at any time after sheath 972 is withdrawn to expose implant 810, before, while, or after fully securing implant 810 to fixation anchors 2203A-D. While the method shown FIGS. 19J-19P is illustrated with respect to implant 810, it should be understood that similar methods may be used to implant the various implants, balloons, and augments described herein.

FIGS. 21-24 illustrate a different method of implantation of implant 810. FIG. 21 shows an implantation site including four anchors 803A'-D' secured to tissue 805'. In the example shown in FIGS. 21-24, respective first ends of two filaments 802B', 802C' are first coupled respectively to two of the anchors 803B', 803C'. In some examples, filaments 802B', 802C' may have been previously coupled to anchors 803B', 803C' during an earlier portion of the surgical procedure (e.g., a double row repair of a rotator cuff). The free end of the filament 802B' may be passed through the opening 838B and then through the opening 838A, as shown in FIG. 22. The free end of the filament 802C' may be passed through the opening 838C and then through the opening 838D, as also shown in FIG. 22. The free ends of filaments 802B', 802C' may then be passed through openings in anchors 803A' and 803D', respectively, as shown in FIG. 23. The implant 810 may then be moved along the filaments 802B', 802C' into the patient to the implantation site while the free ends of filaments 802B', 802C' are pulled through openings in anchors 803A' and 803D', respectively. In some examples, 803A' and 803D' may each be provided with a shuttle (e.g., similar to shuttles 2293A and 2293B of FIG. 19J) to assist in pulling filaments 802B', 802C' through the respective openings. The filaments 802B', 802C' may then be tensioned a desired amount and secured respectively to the anchors 803A', 803D', as shown in FIG, 24. In some examples, filaments 802B', 802C' may be secured to additional filaments coupled respectively to anchors 803A', 803D', for example, by tying the filaments together in a knot or using knotless techniques (e.g., finger traps). As discussed above with respect to FIGS. 20A-20B, the filaments 802B', 802C' may be only partially tensioned so as to allow some movement of implant 810. Thus, implant 810 balloon implant may be movable to some degree along filaments 802B', 802C' after securing filaments 802B', 802C' to anchors 803A', 803D', respectively. While not shown, it should be understood that an implant delivery instrument 850 (e.g., implant delivery instrument 850, 950) may be used to deliver implant 810, and implant 810 may be released from a sheath (e.g., sheath 872, 972), for example, when implant 810 is in the position shown in FIG. 24.

In one aspect, an implant 910 is as shown in FIGS. 26A-26B. Implant 910 includes balloon 940 and first and second tabs 912A-B extending from a periphery of balloon 940. Each tab 912A-B includes an opening 938A-B therethrough sized for receipt of a filament. The implant 910 may be secured to the implantation site (e.g., to anchors) using a filament 902 in a method similar to those discussed above with respect to FIGS. 20A-24. For example, the filament 902 may be coupled at a first end to the anchor 903D, and the other end may be threaded through the openings 938A, 938B. The implant 910 may then be moved into the patient to the implantation site, and the filament 902 may be tensioned and the free end coupled to the anchor 903A. As shown in FIG. 26A-26B, the filament 902 is connected to the lateral row anchors 903A, 903D in a rotator cuff, and implant 910 is not coupled to the medial row anchors 903B, 903C. In other examples, the tabs 912A-B may be tacked to stapled to tissue (e.g., bone) at the implantation site. Tabs 912A-B may be monolithic with balloon 940 or may be connected or attached to balloon 940. Balloon 940 may also include an inlet (not shown) for fluid communication between an external environment and an internal cavity of the balloon 940. The inlet may be as described with respect to any one of the aspects contemplated by the present disclosure. Implant 910 may be provided without any attachment means, such as filaments and/or anchors, although such attachment means may be used to secure implant 910 in a patient, as described herein.

In one aspect, an implant 1010 is as shown in FIGS. 27-29. Implant 1010 includes balloon 1040 and a connection portion 1032 extending from one side of balloon 1040. The connection portion 1032 includes a conduit 1044 extending from balloon 1040 and first and second tabs 1012A-B extending from different sides of conduit 1044. Conduit 1044 includes a lumen therethrough that is in fluid communication with an internal cavity of balloon 1040 (e.g., similar to conduit 144 of implant 110). In some examples, conduit 1044 extends from balloon 1040 such that when balloon 1040 is inserted into a patient, conduit 1044 points in a trailing direction relative to the advancement of implant 1010. As depicted, first and second tabs 1012A-B extend from generally opposing lateral sides of conduit 1044, and each tab 1012A-B includes a respective opening 1038A-B therethrough (e.g., similar to the opening 138 extending through the connection portion 132 of implant 110 as shown in FIG. 5). Thus, implant 1010 may be substantially similar to implant 910 except that tabs 1012A-B extend from conduit 1044 rather than from the body of the balloon (e.g., like tabs 912A-B of balloon 940). First and second openings 1038A-B are each sized for receipt of a filament therethrough. For example, as depicted in FIGS. 27-29, filaments 1002A-B passing through respective openings 1038A-B of implant 1010. Additionally, one or more fixation anchors, such as fixation anchors 1003A-D shown in FIGS. 27-29, may be used to secure filaments 1002A-B to tissue of a patient at an implantation site. It should be appreciated that implant 1010 may be provided without any filaments and/or anchors. As an example of the method shown in FIGS. 27-29, filaments 1002A-B may be respectively secured to anchors 1003A-B. In some examples, filaments 1002A-B may have been previously secured to anchors 1003A-B during an earlier portion of the surgical procedure (e.g., a double row repair of a rotator cuff). As shown in FIG. 27, filament 1002A may be drawn out of the patient (or may already extend out of the patient) and passed through opening 1038A, and filament 1002B may be drawn out of the patient (or may already extend out of the patient) and passed through opening 1038B. As shown in FIG. 28, implant 1010 may be inserted into the patient, guided along filaments 1002A-B to the implantation site. As shown in FIG. 29, filaments 1002A-B may then be tensioned and secured to tabs 1012A and 1012B, respectively (e.g., by tying knots with filaments 1002A-B), or by securing the free end of the filaments back to the respective anchors 1003A, 1003B (e.g., with the filaments looped through the openings 1038A, 1038B. In some examples, a second filament may be coupled to each anchor 1003A, 1003B and the filaments 1002A, 1002B may be coupled to a respective second filament (e.g., using a self-locking structure such as a finger trap).

In one aspect, an implant 1110 is as shown in FIGS. 30A-B. Implant 1110 includes balloon 1140 and two fixation members in the form of a tab 1112 and a connection portion 1132 of a fitting 1120. As shown in FIG. 30B, opposing top and bottom surfaces of balloon 1140 are generally square or rectangularly shaped. However, as discussed above with respect to FIG. 5, the balloon 1140 may not be limited to any particular shape. Fitting 1120 extends from a first side 1146 of balloon 1140 while tab 1112 extends from a second side 1148 of balloon 1140 opposite first side 1146. In some examples, connection portion 1132 and tab 1112 are aligned such that when implant 1110 is folded for receipt in a sheath of an instrument, such as sheath 872 of implant delivery instrument 850 as shown in implant system 1100 of FIG. 30A, a portion of implant 1110 including connection portion 1132 and tab 1112 need not be folded. For example, connection portion 1132 and tab 1112 may each be positioned substantially along a centerline of the balloon 1140 or otherwise aligned with an axis along which the balloon 1140 may be folded or rolled. Connection portion 1132 and tab 1112 each includes a respective opening 1138A-B therethrough. Openings 1138A-B are sized for receipt of a filament therethrough. Balloon 1140 may also include an inlet 1125 for fluid communication between an external environment and an internal cavity of the balloon 1140. The inlet 1125 may be as described with respect to any one of the aspects contemplated by the present disclosure. As discussed above, implant 1110 may receive one or more filaments. For example, as depicted in FIG. 30B, filaments 1102A-B are passed through the respective openings 1138A-B of implant 1110. Additionally, one or more fixation anchors, such as fixation anchors 1103A-B shown in FIG. 30B, may be used to secure filaments 1102A-B to tissue 1105 of a patient at an implantation site. It should be appreciated that implant 1110 may be provided without any filaments and/or anchors.

In one aspect, an implant 1210 is as shown in FIG. 31A. Implant 1210 includes balloon 1240 and four fixation members in the form of tabs 1212A-D. As shown in FIG. 31A, opposing top and bottom surfaces of balloon 1240 are generally square or rectangularly shaped, with each corner of the four corners having one tab of the respective tabs 1212A-D extending therefrom. However, as discussed above with respect to FIGS. 5 and 19A-20B, the balloon 1240 may not be limited to any particular shape or number of tabs. Each tab 1212A-D includes a neck portion 1261 narrower than a free end 1263 of the tab. This is shown in detail in FIG. 31B. Balloon 1240 may also include an inlet (not shown) for fluid communication between an external environment and an internal cavity of the balloon 1240. The inlet may be as described with respect to any one of the aspects contemplated by the present disclosure. Further, implant 1210 may optionally be secured with one or more fixation elements 1203A-D, as shown in FIG. 31A. Each fixation element 1203A-D is configured to wrap over the neck portion 1261 of a respective tab 1212A-D such that the free ends of fixation element 1203A-D are pressed into tissue 1205 of a patient and secure the tab 1212A-D to tissue 1205. Examples of fixation elements may include u-shaped bars as shown in FIG. 31B. Other examples of fixation elements 1203A-D may include tacks, staples, nails, retention screws and/or any other mechanical fastener suitable for securing implant 1210 to tissue 1205 of a patient. It should be appreciated that in FIG. 31A, tissue 1205 is hard tissue in the form of bone, which may be an acromion, clavicle, humerus or other bone in the shoulder or another joint. Further, while FIG. 31A illustrates hard tissue, the tissue to which the implants contemplated by the present disclosure are secured may be hard tissue, soft tissue, or combinations thereof. For example, implant 1210 may also be secured onto rotator cuff tissue, may be secured onto bone as described above, and/or may be partially secured onto soft tissue and partially secured onto bone.

In one aspect, an implant 1310 is as shown in FIG. 32A. Unless otherwise indicated, like reference numerals refer to like elements of implant 1210 shown in FIG. 31A, but within the 1300-series of numerals. As shown in FIG. 32A, implant 1310 includes balloon 1340 and four fixation members in the form of tabs 1312A-D. As shown in FIG. 32A, each tab 1312A-D has a width greater than a width of a fixation element 1303A-D (e.g., a staple, a U-shaped bar, a pin, a tack, a screw etc.) configured to secure the tab 1312A-D to tissue 1305. Thus, the fixation element 1303A-D may pierce through the tab 1312A-D such that the upper portion of the fixation element 1303A-D may secure the tab 1312A-D to the tissue 1305, as shown in detail in FIG. 32B. In other examples, one end of a fixation element 1303A-D (e.g., a staple, a U-shaped bar etc.) may be pressed through the tab 1312A-D and the other end may be pressed directly into tissue without passing through a tab 1312A-D. In such examples, the width of the tab 1312A-D may not be greater than a width of the fixation element 1303A-D. Balloon 1340 also includes an inlet (not shown) for fluid communication between an external environment and an internal cavity of the balloon. The inlet may be similar or equivalent to the inlet of any one of the aspects described herein. As discussed above, the implant 1310 may be secured with fixation elements 1303A-D, shown in FIGS. 32A-32B as U-shaped bars or staples. In other examples, fixation elements 1303A-D may be tacks, nails, retention screws and/or any other mechanical fastener suitable for securing implant 1310 to tissue 1305 of a patient. As discussed above, each fixation element 1303A-D may be configured to either pierce a respective tab 1312A-D or to pass through openings in the tab 1312A-D to secure the tab 1312A-D to tissue 1305.

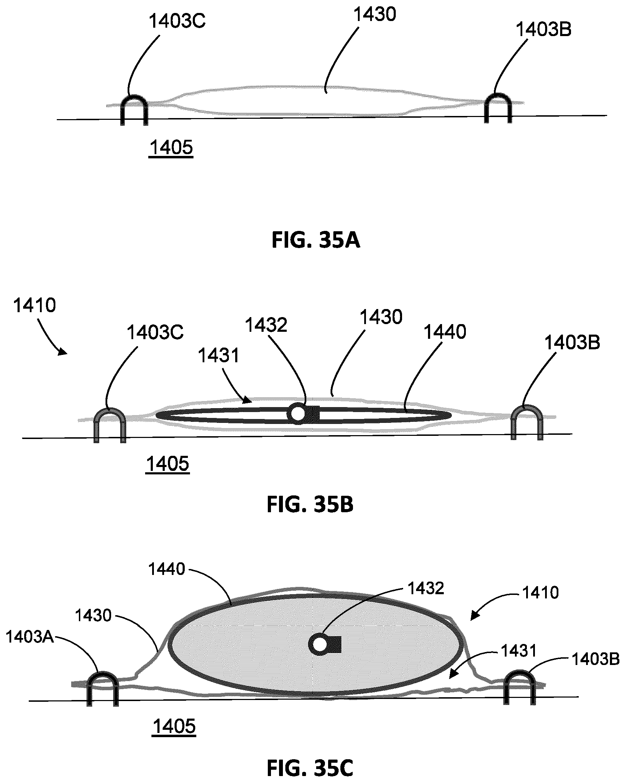

In one aspect, an implant 1410 is as shown in FIGS. 33-35C. Implant 1410 includes augment 1430 and balloon 1440. Augment 1430 may be for example, allograft, autograft (e.g., tissue from the patients biceps tendon of facia lata), animal, or synthetic, alone or in combination (e.g., as a biocomposite material). For example, augment 1430 may be made from or may include collagen, including cross-linked collagen, non-cross-linked collagen, reconstituted collagen, native collagen, human collagen, bovine collagen, and/or xenograph collagen. In other examples, augment 1430 may be made from or may include a synthetic material, a polyester material, an absorbable material, an organic material, silk, or combinations thereof. In some examples, augment 1430 may include a flexible or stretchy material such as FLEXBAND^{®} biomaterial. For example, collagen or other augment material may be deposited, attached, or coated onto the flexible material to form the augment. The flexibility of augment 1430 when formed using a flexible material may provide better performance of augment 1430 throughout a range of motion of the joint (e.g., the rotator cuff) in which the augment 1430 is implanted. Implant 1410 may be secured to tissue at an implantation site by one or more fixation elements, such as fixation elements 1403A-D shown in FIGS. 33-35C. In the depicted aspect, each fixation element 1403A-D is a U-shaped bar or staple that is configured to pierce the augment 1430 to secure the augment 1430 to tissue, such as tissue 1405. In other examples, fixation elements may be tacks, staples, nails, retention screws and/or any other mechanical fastener suitable for securing implant 1410 to tissue 1405 of a patient. In some examples, each fixation element 1403A-D may be disposed through augment 1430 at or near a corner of the augment 1430, as shown in FIG. 33. In other examples, the fixation elements 1403 may be placed at other locations on the augment that are determined to be best suited for a specific procedure. Implant 1410 is configured so that balloon 1440 may be coupled to augment 1430 of implant 1410. In some examples and as shown in FIGS. 33-35C, augment 1430 includes a pouch that defines an internal cavity 1431, and balloon 1440 is disposable within such internal cavity. In this manner, when augment 1430 is secured to tissue 1405 of a patient and balloon 1440 is disposed in the internal cavity 1431 of augment 1430, balloon 1440 may also be secured to the tissue 1405 via augment 1430. As shown in FIG. 35C, after the augment 1430 and the balloon 1440 are attached to the tissue 1405, the balloon 1440 may be inflated. The volume of the internal cavity 1431 and/or the flexibility of the augment 1430 may be sufficient to allow inflation of the balloon 1440.

In other examples, balloon 1440 may be attached to a surface of augment 1430 by, for example, suturing of augment 1430 and balloon 1440 together or coupling balloon 1440 to augment 1430 using an adhesive. In one example, two or more flexible members may be crisscrossed over a surface of balloon 1440 and secured to augment 1430 on opposing sides of balloon 1440, e.g., to form straps, e.g., as shown in FIGS. 38A-38B. Other filament or flexible member arrangements may also be used so that the position of balloon 1440 is controlled and otherwise secured relative to augment 1430. Balloon 1440 may also include an inlet (not shown) for fluid communication between an external environment and an internal cavity of the balloon 1440. The inlet may be as described with respect to any one of the aspects contemplated by the present disclosure. In some examples, the balloon may be coupled to the augment prior to insertion of the implant in to the patient. For example, the balloon 1540 shown in FIG. 38B may be positioned below the straps 1581 before insertion of the implant 1510 into the patient. In other examples, the augment may be delivered and secured to the implantation site first, and the balloon may be coupled to the augment at the implantation site. For example, in the process illustrated by FIGS 38A-38B, the augment 1530 is secured to tissue 1505 before the balloon 1540 is delivered to the implantation site and positioned below the straps 1581.

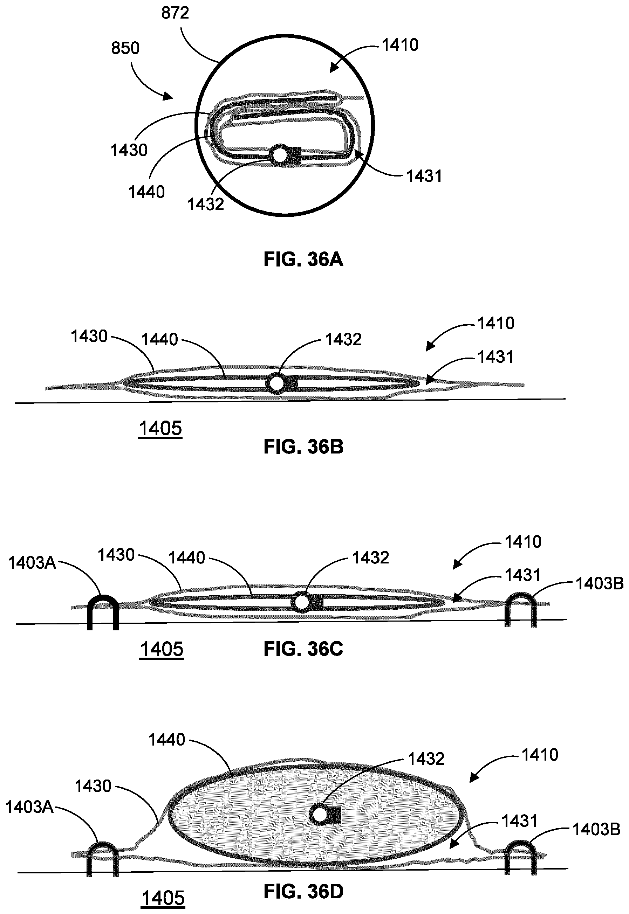

FIGS. 36A-36D are end views illustrating part of a process of implanting implant 1410 into an implantation site using implant delivery instrument 850, according to an example aspect. It should be understood that implant delivery instruments 50, 150, 350, 950, etc. may be used in place of implant delivery instrument 850. In contrast to the process illustrated in FIGS. 34-35C, in the process illustrated by FIGS. 36A-36D, the balloon 1440 of implant 1410 is positioned within internal cavity 1431 of augment 1430 and rolled up within sheath 872 of implant delivery instrument 850 before insertion into the patient (e.g., rather than inserting balloon 1440 into augment 1430 after securing augment 1430 to tissue 1405 as shown in FIGS. 34-35C). In other examples, balloon 1440 may not be positioned inside an internal cavity of augment 1430 but may otherwise be coupled to augment 1430 before insertion into the patient. A connection portion 1432 (e.g., similar to connection portion 432) may be coupled to a distal end of a shaft (not shown; e.g., similar to shaft 60 of implant delivery instrument 50). The implant delivery instrument 850 may be used to position the implant 1410 in the implantation site. FIG. 36B shows the implant 1410 after the sheath 872 (not shown) has been retracted. Augment 1430 and balloon 1440 unfurl, unfold, unroll, etc. and flatten over tissue 1405 at the implantation site. As shown in FIG. 36C, fixation elements 1403A-B (e.g., U-shaped bars, staples, tacks, screws, etc.) are pushed through augment 1430 (but not through balloon 1440) and into tissue 1405 to secure implant 1410 to tissue 1405. It should be understood, however, that other fixation methods described herein may be used to secure implant 1410 to tissue 1405. FIG. 36D shows implant 1410 (still coupled to tissue 1405) after the balloon 1440 has been inflated (e.g., through a shaft coupled to connection portion 1432).

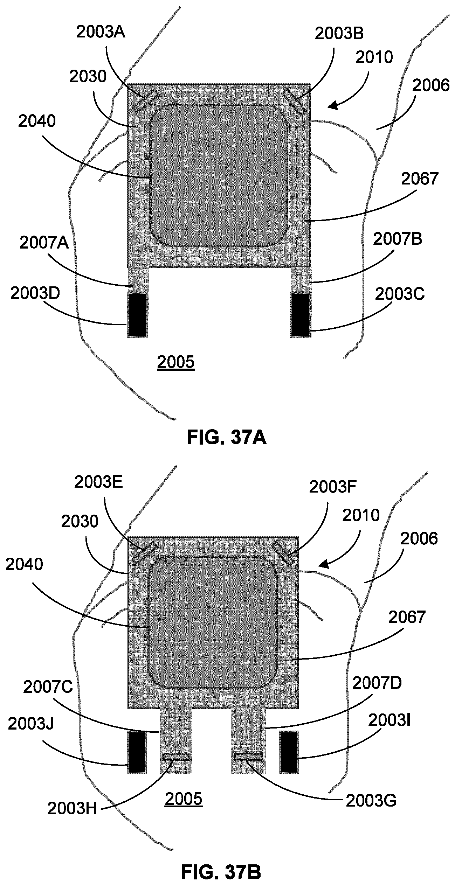

FIGS. 37A-37B illustrate implants 2010 according to example aspects. In some examples, implant 2010 may be similar to implant 1410 except as shown and described herein. As shown in FIGS. 37A-37B, like reference numerals refer to like elements of implant 1410 shown in FIG. 33-35C, but within the 2000-series of numerals. Implant 2010 of FIG. 37A includes an augment 2030 and a balloon 2040. In various examples, balloon 2040 may be coupled to or secured within augment 2030 before moving implant 2010 to the implantation site and securing augment 2030 to tissue 2005 or augment 2030 may be secured to tissue 2005 before balloon 2040 is coupled to or secured within augment 2030. A first (e.g., medial) end of augment 2030 is shown secured to tissue 2005 in two locations (e.g., corners) by two fixation elements 2003A-B, which may be, for example, U-shaped bars, stapes, tacks, screws, etc. (similar to fixation elements 1403A-D) extending through the material of the augment 2030. However, unlike implant 1410, the opposite (e.g., lateral) end of the implant 2010 is not secured with fixation elements similar to fixation elements 1403A-D. Instead, augment 2030 includes two leg portions 2007A-B extending from a main body 2067 of augment 2030 that may be connected to fixation anchors 2003C-D (e.g., similar to fixation anchors 803A-D). In some examples, fixation anchors 2003C-D may be twist anchors or screw-in achors, which include a shank with screw threads and an eyelet. Leg portions 2007A-B may be passed through the eyelet, and the twist anchors may be threadedly coupled to holes in the tissue 2005 (e.g., bone), pinning the leg portions 2007A-B between the twist anchors and the bone. In other examples, the leg portions 2007A-B may be stitched, tied, adhered, or otherwise coupled in various ways to the fixation anchors 2003C-D. In some examples, the fixation anchors 2003C-D may have been previously installed into the tissue 2005 in an earlier surgical procedure (e.g., to secure suture anchors used in a double-row repair). While two leg portions 2007A-B are shown, it should be understood that the augment 2030 may include any number of leg portions extending from main body 2067. Further, the shape of the augment 2030 and/or the balloon 2040 may not be limited to any particular shape.

As an example of implanting the implant 2010 of FIG. 37A in a patient, the implant 2010, with balloon 2040 already coupled to augment 2030 (e.g., in an internal cavity of augment 2030), may be inserted into an implantation site of a patient. Similar to the examples discussed above, implant 2010 may be inserted into the patient to the implantation site in a rolled or folded configuration (e.g., using implant delivery instrument 50, 150, etc.). The implant 2010 may unfurl, unfold, unroll, etc. once inserted into the patient. A first (e.g., medial) side of augment 2030 may then be coupled to tissue 2005 by pressing fixation elements 2003A-B through the material of augment 2030 into tissue 2005. The leg portions 2007A, 2007B on the other (e.g., lateral) side of implant 2010 may then be coupled respectively to fixation anchors 2003D, 2003C (e.g., pulled through an eyelet or opening in a fixation anchor 2003C-D) as discussed above, and fixation anchors 2003C-D may be coupled to tissue 2005 as discussed above. Alternatively, the fixation anchors 2003C-D may be coupled to tissue 2005 before the leg portions 2007A-B are coupled to the fixation anchors 2003C-D. In other examples, the leg portions 2007A-B may be secured to tissue 2005 on the lateral side using fixation anchors 2003C-D before the fixation elements 2003A-B are used to secure the medial side. Once the augment 2030 is secured to tissue 2005, the balloon 2040 may be inflated. In some examples, as discussed above with respect to FIGS. 35A-35C, augment 2030 may be delivered first and secured to tissue 2005 and soft tissue 2006 as discussed above, then balloon 1440 may be delivered and coupled to and/or inserted into augment 1430.

Implant 2010 of FIG. 37B is similar to implant 2010 of FIG. 37A, except that both the upper (as shown) corners and leg portions 2007C-D are coupled to the tissue 2005 by fixation elements 2003E-H, similar to fixation elements 2003A-B (e.g., U-shaped bars, staples, tacks, screws), that extend through the material of the augment 2030. Fixation anchors 2003I-J may be suture anchors previously installed into the tissue 2005 for use in an earlier portion of the surgical procedure (e.g., as suture anchors for a double-row repair) and may not be used to secure leg portions 2007C-D to tissue 2005. As shown in FIG. 37B, the leg portions 2007C-D may be positioned away from the corners of the main body 2067 (as compared to the leg portions 2007A-B of FIG. 37A) and may extend and be coupled to the tissue 2005 between the fixation elements 2003 I-J. As discussed above with respect to FIG. 37A, the implant 2010 of FIG. 37B may not be limited to any particular shape, and the leg portions of augment 2030 may not be limited to any particular number or location. The implant 2010 of FIG. 37A may be implanted in a manner similar to that discussed above with respect to implant 2010 of FIG. 37A, except that the leg portions 2007C-D may be secured to tissue 2005 using fixation elements 2003G-H rather than fixation elements 2003I-J. After each of the fixation elements 2003E-H are pressed through the material of augment 2030 and secured to tissue 2005, balloon 2040 may be inflated. As discussed above, balloon 2040 may be partially or fully inflated before augment 2030 is secured to tissue 2005.

In one aspect, an implant 1510 is as shown in FIGS. 38A-38B. Implant 1510 includes augment 1530 and balloon 1540. Implant 1510 may be secured by one or more fixation elements 1503A-D shown in FIGS. 38A-38B. Unless otherwise indicated, like reference numerals refer to like elements of implant 1410 shown in FIGS. 33-34, but within the 1500-series of numerals. Implant 1510 as depicted includes a pair of flexible elements arranged in a crisscross formation to define straps 1581. In some examples, straps 1581 may be made of the same material as augment 1530 (or any of the augment materials described herein). In some examples, straps 1581 may be integrally formed as part of augment 1530. In variations that are not shown, straps 1581 may be defined by two or more flexible elements secured to augment 1530 in other patterns. Such arrangements may provide for a restraint on objects, i.e., a balloon, positioned between the flexible elements and augment 1530 to capture such object within straps 1581. For example, the augment 1530, with the straps 1581 already attached to the body 1533 of the augment 1530, may be secured to tissue 1405 using fixation elements 1503A-D as shown in FIG. 38A-38B. With the augment 1530 secured, the balloon 1540 may then be positioned between the body 1533 and straps 1581 of augment 1530, with straps 1581 crisscrossing over balloon 1540 as shown in FIG. 38B. Balloon 1540 may then be inflated, causing straps 1581 to secure balloon 1540 to augment 1530. Thus, straps 1581 allow balloon 1540 to be easily secured to augment 1530 and the implantation site, as straps 1581 tension around balloon 1540 as balloon 1540 is inflated without the need for subsequent operations to couple balloon 1540 to augment 1530 or tissue 1505. In some examples, balloon 1540 may then be positioned between the body 1533 and straps 1581 of augment 1530 before augment 1530 balloon 1540 are delivered to the implantation site.

In one aspect, an implant 1610 is as shown in FIG. 41. FIGS. 39-41 illustrate steps in a method of implanting the implant 1610 in which an balloon and an augment are separately coupled to tissue at the implantation site, rather than the balloon and augment being coupled together. Unless otherwise indicated, like reference numerals refer to like elements of implant 1510 shown in FIG. 38B, but within the 1600-series of numerals. Implant 1610 includes augment 1630 and balloon 1640. Balloon 1640 includes four tabs 1612A-D, each including an opening 1638A-D sized to receive a filament for securing the balloon 1640 to the implantation site. The augment 1630 may be secured to the implantation site by one or more fixation elements 1613A-D, shown as U-shaped bars or staples pressed through augment 1630. As shown in FIG. 39, the joint (e.g., a shoulder joint) is shown with fixation anchors 1603A-D that were previously secured to tissue in an earlier portion of the procedure and filaments 1691 stretched over tissue in the joint (e.g. for rotator cuff repair). To position the implant 1610 at the implantation site in the joint, additional filaments 1602A-D may be coupled to (or may previously have been coupled to) the fixation anchors 1603A-D as shown in FIG. 39, or the filaments 1691 may include additional length that can be used to secure implant 1610.

. Next, as shown in FIG. 40, augment 1630 may be secured to the implantation site, for example, using fixation elements 1613A-D (e.g., tacks, staples, screws, etc.). Then, as shown in FIG. 41, balloon 1640 may be positioned on top of augment 1630, and filaments 1602A-D may then be respectively fed through openings 1638A-D, tensioned, and secured to the tabs 1612A-D of balloon 1640 to secure balloon 1640 to the implantation site. In some examples, tabs 1612A-D may not include openings 1638A-D, and the filaments may be pressed through the material of tabs 1612A-D (e.g., with a suture passer or needle). Notably, in some examples and as shown in FIGS. 39-41, balloon 1640 may not be secured to augment 1630.

Augment 1630 may be positioned below balloon 1640 such that augment 1630 is facing (e.g., is in contact with) the tendon or other tissue in the joint. Similarly, in each aspect in which an implant includes an augment and a balloon, the augment may be positioned between the balloon and the tissue in the joint. In some examples, augment 1630 and balloon 1640 may each be delivered to the implantation site using separate delivery instruments (e.g., implant delivery instrument 850, implant delivery instrument 950, etc.). In some examples, the augment may be positioned on top of a degradable (e.g., biodegradable balloon), such that the augment may be in contact with the tissue only after the balloon degrades.

In one aspect, an implant 1710 is as shown in FIG. 42. Unless otherwise indicated, like reference numerals refer to like elements of implant 110 shown in FIGS. 5-7, but within the 1700-series of numerals. Implant 1710 includes a balloon 1740 and a tab 1712 extending from a periphery of balloon 1740. Tab 1712 has an opening 1738 therethrough. In other examples (not shown), implant 1710 may include two or more tabs. A filament 1702 may be coupled to an anchor 1703 secured to an acromion bone 1705 of a patient. The anchor 1703 may be secured to a side of the acromion bone 1705 opposite the position of the balloon 1740. The filament 1702 may fed through opening 1738 in tab 1712 of balloon 1740, and the balloon 1740 may be guided along the filament 1702 into the patient to the implantation site (e.g., under the acromion bone 1705 of a shoulder joint as shown in FIG. 42). The filament 1702 may then be tensioned and secured to tab 1712 (e.g., by tying a knot with filament 1702) or by securing the free end of filament 1702 back to 1702 (e.g., with the filaments looped through the opening1738. In some examples, a second filament may be coupled to anchor 1703 and filament 1702 may be coupled to the second filament (e.g., using a knotless and/or self-locking structure such as a finger trap).

In one aspect, an implant 1810 is as shown in FIGS. 44-46, with FIG. 43 illustrating an implantation site of implant 1810 before implant 1810 is positioned therein. Implant 1810 includes augment 1830 and balloon 1840. In some examples, balloon 1840 may be coupled to augment 1830, for example, using an adhesive, before implant 1810 is inserted into the patient. The balloon 1840 includes four openings 1838A-D extending therethrough, which may extend through tabs 1812A-D as shown in FIGS. 44-46. Openings 1838A-D are sized for receipt of a filament therethrough. As shown in FIGS. 44-46, openings 1838A-D are positioned proximate corners of balloon 1840. In other examples, openings 1838A-D may be positioned along one or more edges of balloon 1840, at a combination of edges and corners. In some examples, the balloon 1840 may include fewer or greater than four openings. As discussed above, the tabs 1812A-D may be in different shapes or positions than as shown. For example, balloon 1840 may include a flap of material extending around the perimeter of balloon 1840 with openings extending therethrough for coupling balloon 1840 to the implantation site. Where balloon 1840 includes fixation members 1812A-D or other features for securing the balloon 1840 to an implantation site, such fixation members may number fewer or greater than four. The openings 1838A-D of implant 1810, whether directly within the balloon 1840 or within a fixation member, may be positioned anywhere on a periphery of balloon 1840, and may be at any position with respect to each other. As shown in FIGS. 44-46, opposing top and bottom surfaces of balloon 1840 are generally square or rectangularly shaped. However, as discussed above with respect to FIG. 5, the balloon 1840 may not be limited to any particular shape Balloon 1840 may also include an inlet (not shown) for fluid communication between an external environment and an internal cavity of the balloon 1840. The inlet may be as described with respect to any one of the aspects contemplated by the present disclosure.

In some examples, augment 1830 may be attached to a surface of balloon 1840. Augment 1830 may be attached to balloon 1840 so that when implanted, augment 1830 is in between the tissue of the patient at the repair site and balloon 1840 (e.g., underneath balloon 1840, as shown). In other variations, augment 1830 may face away from the tissue when implanted. In some examples, attachment may be accomplished through suturing of augment 1830 and balloon 1840 together. In one example, two or more filaments may be crisscrossed over a surface of balloon 1840 and secured to augment 1830 on opposing sides of balloon 1840, e.g., to form straps. Other filament arrangements may also be used so that the position of balloon 1840 is controlled and otherwise secured relative to augment 1830. Filaments (e.g., suture) may be used to secure implant 1810 to tissue. For example, as depicted in FIGS. 44-46, implant 1810 is secured by filaments 1802B-C passing through respective sets of openings 1838A-B and 1838C-D. Additionally, implant 1810 may be secured to one or more fixation anchors (e.g., using filaments 1082B-C), such as fixation anchors 1803A-D shown in FIGS. 43-46. As discussed above with respect to the other implants, implant 1810 may be guided along (e.g., parachuted down) filaments 1802B-C attached to the anchors 1803B-C to the implantation site.

In one aspect, an implant 1910 is as shown in FIGS. 48-50, with FIG. 47 illustrating an implantation site of implant 1910 before implant 1910 is positioned therein. Implant 1910 includes augment 1930 and balloon 1940. Augment 1930 has a larger footprint than balloon 1440 such that material of augment 1430 extends beyond the perimeter of 1440, allowing for securing of the implant 1910 through the augment material. As shown in FIGS. 48-50, augment 1930 includes openings 1938A-D extending through respective corners of augment 1930. Openings 1938A-D are sized for receipt of a filament therethrough. In some examples, implant 1910 may include fewer or greater than four openings extending therethrough. For example, implant 1910 may include one opening, two openings, etc. The openings through implant 1910 may be positioned anywhere on a periphery of augment 1930 and may be at any position with respect to each other. In other examples, filaments or other fixation members may be passed directly through the material of augment 1930 (e.g., with a suture passer or needle), without passing through preformed openings, to secure implant 1910. Augment 1430 and balloon 1440 may be coupled in any manner discussed herein. For example, in some examples, augment 1930 includes a pouch that defines an internal cavity, and balloon 1940 is disposable within such internal cavity (e.g., similar to internal cavity 1431 of augment 1430 described above). In this manner, when augment 1930 is secured to tissue 1905 of a patient and balloon 1940 is disposed in the internal cavity of augment 1930, balloon 1940 may also be secured to the tissue 1905 via augment 1930. In other examples, augment 1930 may be attached to a surface of balloon 1940. Augment 1930 may be attached to balloon 1940 so that when implanted, augment 1930 is in between the tissue of the patient at the repair site and balloon 1940. In other variations, augment 1930 may face away from the tissue when implanted. In some examples, attachment may be realized through suturing of augment 1930 and balloon 1940 together. In one example, two or more filaments may be crisscrossed over a surface of balloon 1940 and secured to augment 1930 on opposing sides of balloon 1940, e.g., to form straps. Other filament arrangements may also be used so that the position of balloon 1940 is controlled and otherwise secured relative to augment 1930. Balloon 1940 may include an inlet (not shown) for fluid communication between an external environment and an internal cavity of the balloon 1940. The inlet may be as described with respect to any one of the aspects contemplated by the present disclosure. Filaments (e.g., sutures) may be used to secure implant 1910 to tissue. For example, as depicted in FIGS. 48-50, implant 1910 may be secured to tissue 1905 by filaments 1902B-C that pass through respective sets of openings 1938A-B and 1938C-D. Additionally, implant 1910 may be secured to one or more fixation anchors (e.g., by filaments 1902B-C), such as fixation anchors 1903A-D shown in FIGS. 47-50. As discussed above with respect to the other implants, implant 1910 may be guided along (e.g., parachuted down) filaments 1902B-C attached to the anchors 1903B-C to the implantation site.

The method of securing implant 1910 to tissue 1905 illustrated in FIGS. 47-50 may be substantially similar to the method of securing implant 1810 to tissue 1805 illustrated in FIGS. 43-46, except that filaments 1902B-C may pass through openings 1938A-D in augment 1930 rather than through openings in tabs of a balloon (e.g. tabs 1812A-D in balloon 1840) or may be pressed directly through material (e.g. with a suture passer or needle) rather than through openings. FIG. 48 shows filament 1902B coupled to anchor 1903B and passing through openings 1938B and 1938A in augment 1930, and filament 1902C coupled to anchor 1903C and passing through openings 1938C and 1938D in augment 1930. As shown in FIG. 49, filament 1902B is then pulled through an opening in anchor 1903A, and filament 1902C is pulled through an opening in anchor 1903D. Filaments 1902B-C are pulled through the respective openings in anchors 1903A and 1903D as implant 1910 is guided along filaments 1902B-C to the implantation site. As shown in FIG. 50, filament 1902B is then tensioned and secured (e.g., tied) to anchor 1903A, and filament 1902C is secured to (e.g., tied) to anchor 1903D. As discussed above, an implant delivery instrument (e.g., implant delivery instrument 850, implant delivery instrument 950, etc.) may be used to deliver implant 1910.

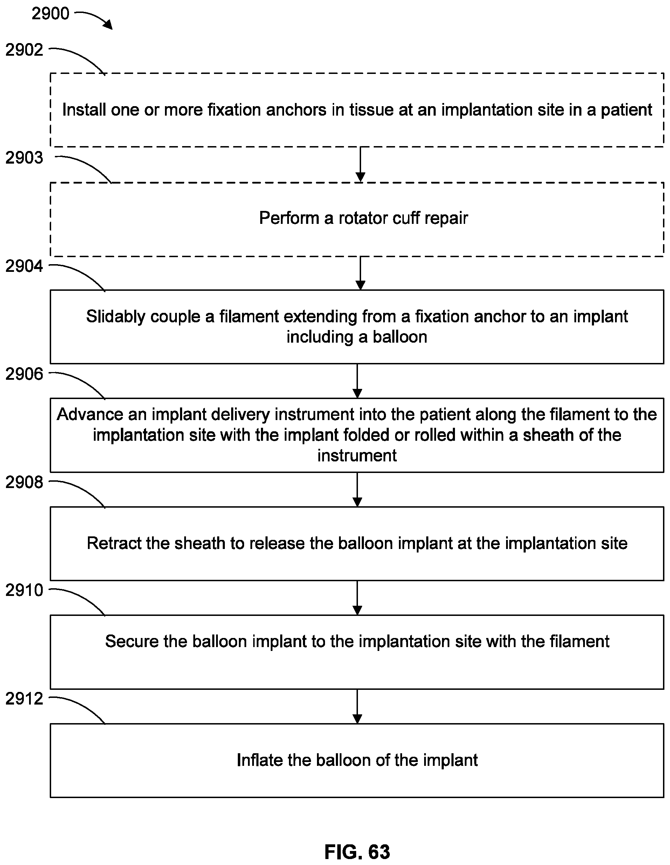

FIG. 63 illustrates a method 2900 of delivering an implant, according to an example aspect. One example of the method 2900 is illustrated by the implantation of implant 810 of implant system 800 into a patient, as shown in FIGS. 20A-20B. An intended implantation location may be in a joint of the patient, such as the shoulder. In such cases, tissue 805 onto which the implant 810 may be placed may be one of the acromion, clavicle or humerus. In some examples, the implantation location may be on soft tissue in the joint. The implant 810 may be implanted, for example, using the implant delivery instrument 850 shown in FIG. 19A or the implant delivery instrument 950 shown in FIG. 19B. Optionally, the method 2900 may include the initial assembly step of implant system 800. For assembly, sheath 872 of implant delivery instrument 850 may be retracted, or is already retracted, and implant 810 may be prepared (e.g., folded or rolled) and coupled to implant delivery instrument 850 as shown in FIG. 19A, for example, in the same manner as described above with respect to implant system 1 . Assembly of implant system 800 is then complete. In some examples, implant system 800 is initially provided in an assembled condition. In still further examples, implant 810 is kept separate from implant delivery instrument 850 and independently delivered to an implantation site.

In one example of the method 2900, placement of implant 810 into the patient follows a previously completed joint repair at the target site (e.g., a double-row repair of soft tissue (e.g., during an earlier portion of the surgical procedure in which implant 810 is implanted). This is shown via filaments 891 in FIG. 20A, such filaments 891 crossing respectively from anchor 803A to anchor 803C and from anchor 803B to anchor 803D to repair soft tissue 806, e.g., a tear in soft tissue 806. In some examples, soft tissue 806 is a tendon in the shoulder joint. With the double row repair already in place, additional filaments 802A-D may have been previously attached to the respective anchors 803A-D before or during securing anchors 803A-D to tissue 805 for the double row repair or coupled to 803A-D after securing 803A-D to tissue 805. Each filament 802A-D may thus extend from one of anchors 803A-D. Some examples of method 2900 include operation 2902, in which one or more fixation anchors (e.g., anchors 803A-D) are installed in tissue (e.g., tissue 1805) at the implantation site in the patient. For example, the method 2900 may be performed without a preceding double row repair to repair tissue. Instead, anchors may be implanted into tissue in the first instance and respective filaments may be secured to such anchors to prepare the implant receiving site for the subsequent method steps. Thus, filaments (e.g., filaments 802A-D) may be respectively coupled at one end to the anchors before or after installation of the anchors into the tissue. At the time the implant is placed at the implantation site, free ends of each filament 802A-D are drawn out of the patient or may remain outside the patient during the process of coupling filaments 802A-D to anchors 803A-D and/or securing anchors 803A-D to tissue 805. Some examples of method 2900 include operation 2903, in which a rotator cuff repair (e.g., a double row repair) is performed. The rotator cuff repair may include, for example, coupling anchors in a medial portion of the joint to anchors in a lateral portion of the joint using filaments (e.g., sutures). In some examples, other joint repair procedures (e.g., shoulder surgeries other than rotator cuff repair, repair of joints other than the shoulder) may be performed at operation 2903.

At operation 2904 of method 2900, a filament extending from a fixation anchor is slidably coupled to an implant including a balloon (e.g., balloon 840 of implant 810). The filament may be passed through one or more openings in the implant (e.g., in a peripheral portion of the implant) or directly through material of the implant (e.g., tabs extending from the implant) in one or more locations. As discussed above the filaments may extend out of the patient such that the filaments may be slidably coupled to the implant before the implant is advanced into the patient. For example, as shown in FIG. 20A, each filament 802A-D is passed through a respective opening 838A-D at a corner of implant 810.

At operation 2906 of the method 2900, an implant delivery instrument is advanced into the patient along the filament to the implantation site with the implant folded, rolled, or otherwise collapsed within a sheath of the instrument. For example, implant 810 may be received on (e.g., coupled to) implant delivery instrument 850, after passing a filaments 802A-D through openings 838A-D in the implant (e.g., in operation 2904), and sheath 872 may be advanced over implant 810 as shown in FIG. 19A prior to advancement of implant delivery instrument 850 into the patient and toward the intended implantation site. The free ends of the filaments 802A-D may run through the proximal end 877 of the sheath 872. Alternatively, the implant delivery instrument 850 may be preassembled with the implant 810 pre-positioned within the sheath 872. Each filament 802A-D may be tensioned, and implant delivery instrument 850 may then be advanced into the patient to the implantation site while being guided along the filaments 802A-D, which may be pulled through openings 838A-D respectively as implant delivery instrument 850 and implant 810 move toward the implantation site proximate anchors 803A-D. As discussed above, filaments 802A-D may be directly coupled to tissue 805 without the use of any anchors 803A-D. Thus, implant 810 may be guided down (or "parachuted" down) into the patient via filaments 802A-D to the intended implantation site proximate the locations that 802A-D are coupled to tissue 805. At operation 2908 of the method 2900, the sheath is retracted to release the implant. For example, sheath 872 may be retracted to expose implant 810. In other examples, the implant may removed from the sheath using other methods. For example, an implant may be pushed out of a distal end of a sheath of an implant delivery instrument rather than retracting the sheath.

In some examples, implant delivery instrument 850 may not be advanced fully to the implantation site, and implant 810 may be released and then parachuted down for a remainder of the distance to the implantation site. When implant delivery instrument 850 is not advanced fully to the implantation site, exposed implant 810 may be released and then parachuted down for a remainder of the distance to the implantation site. Further, once implant delivery instrument 850 is removed from the patient following disengagement of implant delivery instrument 850 from implant 810, the free end of each filament 802A-D may be used to secure implant 810 at the implantation site. In some cases, the filaments 802A-D may be passed through respective openings 838A-D of implant 810 after implant 810 is positioned inside the patient and released form the sheath 872.

When implant 810 is positioned at the implantation site, each filament 802A-D is already attached to tissue 805 (e.g., via an anchor 803A-D) and has been passed through a respective opening 83 8A-D of implant 810 outside of the patient before the implant is slid along the filaments 802A-D to the implantation site. At operation 2910 of method 2900, the filament is used to secure the implant at the implantation site. For example, as shown in FIG. 20B, each filament 802A-D may be attached (e.g., tied) to a respective tab 812A-D and/or an edge of balloon 840 through an opening 838A-D. In other examples, filaments may be passed through one or more openings and fastened to an anchor rather than being tied to a tab. In this manner, implant 810 is then secured to tissue 805. Securement of filaments 802A-D to a part of implant 810 may be through tying or knotting of the filaments 802A-D, or through the use of knotless fixation. Anchors for knotted or knotless applications may be those described elsewhere in the present disclosure, for example. The filament may also be employed in a knotless application through the formation of a finger trap, as discussed above. In some examples, a filament other than the filament used to guide the implant to the implantation site may be used to secure the implant to tissue, or another fixation element (e.g., a staple, a pin, a screw, etc.) may be used to secure the implant to tissue.

At operation 2912 of method 2900, the balloon of the implant is inflated. For example, in variations where implant delivery instrument 850 is used to advance implant 810 to the implantation site, implant delivery instrument 850 may be used to inflate balloon 840. In variations of the example where implant 810 is advanced without an instrument, implant delivery instrument 850 may be secured to an inlet of balloon 840 to inflate the balloon 840 via air or fluid after the balloon is positioned in the patient. As discussed above, in some examples, the balloon may be inflated or partially inflated before the filament or filaments are tensioned and secured (e.g., at operation 2910) The procedure is completed with the withdrawal of the implant delivery instrument 850, sealing of the balloon 840 and closure of the portal into the patient.

As another example, the method 2900 may be performed for implantation of implant 810, as shown in FIGS. 21-24. The optional method of assembly of an implant system for use with implant 810 may be as described above for implant system 800. In some examples, the implant system is initially provided in an assembled condition. In still further examples, implant 810 is kept separate from implant delivery instrument 850 and independently delivered to an implantation site.

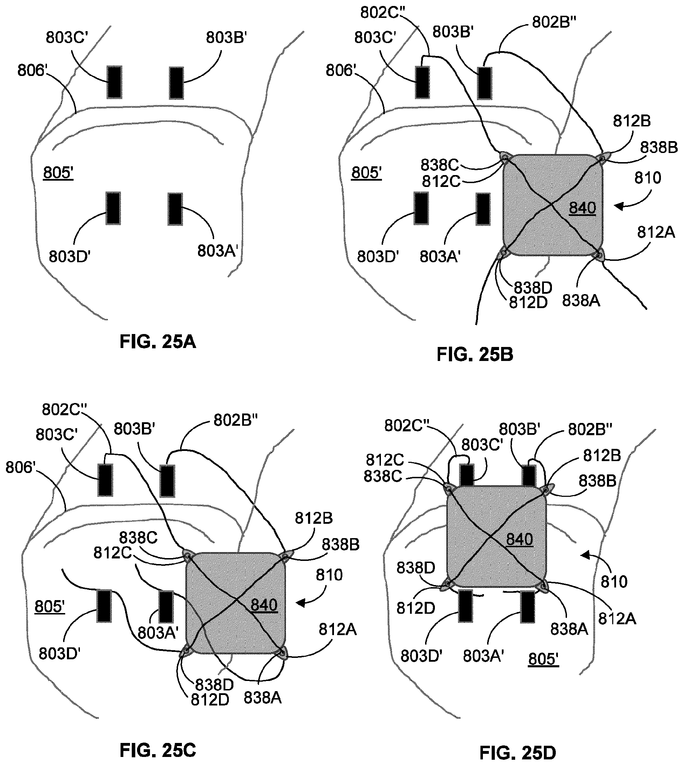

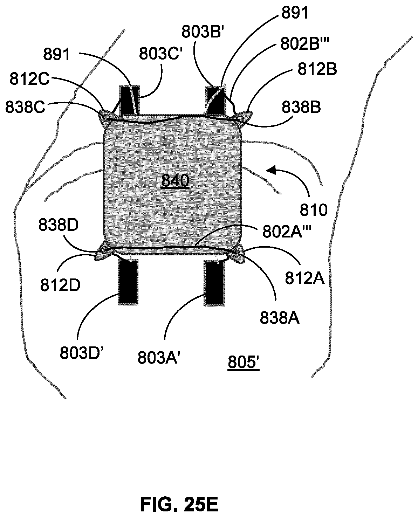

Initially, and as shown in FIG. 21, anchors 803A'-803D' are secured to tissue 805' at locations proximate the desired implantation site (e.g., at operation 2902 of method 2900). Before or after securing the anchors to the tissue, a filament 802B' is secured to anchor 803B' and a filament 802C' is secured to anchor 803C'. In some examples, the filaments 802B', 802C' may have been previously attached to the fixation anchors 803B', 803C', for example, at the time of a double row repair. When the implantation site is in a subacromial space, anchors may be secured to tissue along a medial row (e.g., 803B', 803C') and a lateral row (803A', 803D'). A filament may be passed from an anchor on the medial row to an anchor on the lateral row while passing over the implant or through openings in tabs of the implant as described below and shown in FIGS. 21-25D. The filaments may be arranged substantially parallel to each other, each extending from a medial anchor to an adjacent lateral anchor as shown in FIGS. 21-24 or may crisscross over the implant as shown in FIGS. 25A-25D. In other examples, filaments may be arranged substantially parallel to each other, each extending from a first medial anchor to a second medial anchor or a first lateral anchor to a second lateral anchor as shown in FIG. 25E. In some examples, the lateral row and/or the medial row may include more than two anchors. In some examples, an anchor may be coupled to multiple other anchors (e.g., with multiple filaments).

In one variation of this example, at operation 2904 of method 2900, a free end of filament 802B' is drawn out of the patient and is passed through opening 838B at one corner of implant 810 and opening 838A at another corner of implant 810, as shown in FIG. 22. Similarly, free end of filament 802C' is drawn out of the patient and is passed through opening 838C' at one corner of implant 810 and opening 838D at another corner of implant 810. When, for example, implant delivery instrument 850 is used to deliver implant 810, the filaments may be passed through the respective openings 838A-D when implant 810 is coupled to shaft 860 and sheath 872 is retracted. When, for example, implant delivery instrument 950 is used to deliver implant 810, the filaments may be passed through the respective openings 838A-D through windows 975A-D. Free ends of filaments 802B', 802C' are then passed through respective anchors 803A', 803D', as shown in FIG. 23. Then, the free ends of respective filaments 802B', 802C' are pulled and tensioned to bring implant 810 down to the desired implantation site location. This occurs because each filament 802B', 802C' is secured to one anchor and passes through another anchor, and in this way, when the filaments 802B', 802C' are pulled, a length of each respective filament between the anchors decreases, pulling implant 810 toward a space between the anchors 803A'-803D'.

In another variation of this example, at operation 2906 of method 2900 implant 810 is received on implant delivery instrument 850 and sheath 872 is advanced over implant 810 prior to advancement of implant delivery instrument 850 into the patient. Alternatively, the implant delivery instrument 850 may be preassembled with the implant 810 pre-positioned within the sheath 872. Implant delivery instrument 850 is then advanced into the patient and toward the intended implantation site. At operation 2908 of method 2900, proximate the implantation site, or from a location within the patient still at a distance from the implantation site, the sheath 872 is retracted to expose implant 810. Then, the process of receiving the respective filaments 802B, 802C through openings 838A-838D and securing implant 810 to anchors 803A'-803D' proceeds as described above. Alternatively, in yet another variation of this example, the filaments 802B', 802C' may be passed through openings 838A-838D while implant 810 is attached to implant delivery instrument 850 outside of the patient, with the free ends of the filaments extending through the proximal end 877 of the sheath 872. Then, the sheath 872 may be closed over the implant 810 loaded with the filaments 802B', 802C' and the implant delivery instrument 850 may be advanced into the patient. Inside the patient at a location proximate the implantation site, or from a location within the patient still at a distance from the implantation site, sheath 872 of implant delivery instrument 850 is withdrawn and implant 810 is released. When implant 810 is released at a distance from the implantation site, filaments 802B', 802C' may be used to aid in the advancement of implant 810 over a remainder of a distance to the implantation site. Further, once implant delivery instrument 850 is removed entirely, each filament 802B', 802C' may be retrieved from its respective free end for further use to secure implant 810 to the tissue. When implant 810 is positioned proximate the implantation site, each filament 802B', 802C' is already attached to tissue 805', e.g., via an anchor 803B'-803C', and also passes through a respective opening 838A-838D of implant 810. In such position, at operation 2910 of method 2900, each filament 802B', 802C' may be secured to a respective anchor 803A', 803D' to complete the implant 810 securement, as shown in FIG. 24. Securement of filaments 802B', 802C' to a part of implant 810, e.g., a respective tab 812A'-812D' and/or an edge of balloon 840, may be through tying or knotting of the filaments 802B', 802C', or through use of knotless fixation. In this manner, implant 810 is then secured to tissue 805'. In any of the contemplated variations of the above method, at operation 2912 of method 2900, implant delivery instrument 850 may be used to inflate balloon 840 before or after securing the filaments 802B', 802C' to the implant 810. The procedure is completed with the withdrawal of the instrument, sealing of the balloon, and closure of the portal into the patient.

FIGS. 25A-25D illustrate an alternative arrangement for securing 810// in which filaments 802B", 802C" are be crisscrossed over balloon 840. As shown in FIG. 25B, filament 802B" is coupled to anchor 803B', passed through opening 838B, crossed over balloon 840, and passed through opening 838D. Filament 802C" is coupled to anchor 803C', passed through opening 838C, crossed over balloon 840, and passed through opening 838A. As shown in FIG. 25C, filament 802B" is then passed through anchor 803D', and filament 802C" is then passed through anchor 803A'. Then, as shown in FIG. 25D, filaments 802B" and 802C" are tensioned to secure implant 810 to tissue 805. The tension in filaments 802B', 802C' in FIGS. 21-24 and in filaments 802B", 802C" may serve to help repair the joint. For example, when performed on a rotator cuff, the methods of coupling balloon 840 to tissue 805 shown in FIGS. 21-24 and 25A- 25D may in effect also serve as a double row repair in which the implant 810 is positioned underneath filaments that also serve to compress and reattach torn rotator cuff tissue to the bone.

FIG. 25E illustrates another alternative arrangement for securing implant 810 in which filament 802A‴ extends from a first lateral anchor 803A' to a second lateral anchor 803D', and filament 802B‴ extends from a first medial anchor 803B' to a second medial anchor 803C'. As shown in FIG. 25E, filament 802A‴ is coupled to anchor 803A', passed through opening 838A, passed through opening 838D, and secured to anchor 803D'. Filament 802B‴ is coupled to anchor 803B', passed through opening 838B, passed through opening 838C, and secured to anchor 803C'. In each of the alternative arrangements shown in FIGS. 25A-D and 25E, implant 810 may be delivered using an implant delivery instrument (e.g., implant delivery instrument 850, implant delivery instrument 950, etc. as discussed above.

In one aspect, a method includes implantation of implant 910, shown in FIGS. 26A-26B, into a patient. Such method may be performed through performance of the same steps as described above for implantation of implant 810 used with or without implant delivery instrument 850. In performance of this method, a filament secured to an anchor within a patient at one end may be passed through openings 938A-938B and then secured to another anchor to fix implant 910 to the tissue of the patient (e.g., lateral rotator cuff tissue), where the one or more anchors are themselves secured to the tissue. In some examples, the filament 902 may be passed through material of the tabs 912A-B rather than through openings 938A-B in the tabs 912A-B. In examples where the filament is used to parachute the implant 910 down to the target site, such filament may be tensioned. In variations of the method, implant 910 may be preloaded in an implant delivery instrument 50. And in some examples of this variation, filaments secured to the tissue of the patient may be loaded through openings 938A, 938B prior to use of the implant delivery instrument 50 to deliver the implant 910 into the patient.

In another aspect, a method includes implantation of implant 1710, shown in FIG. 42, into a patient. Such method may be performed through performance of the same steps as described above for implantation of implant 810 used with or without implant delivery instrument 850. In performance of this method, a filament 1702 that is secured to an anchor 1703 within a patient at one end may be passed through opening 1738 and then secured to tab 1712 of the implant 1710 or another anchor to fix implant 1710 to the tissue of the patient, where the one or more anchors are themselves secured to the tissue. Here, anchor 1703 is secured to an acromion bone 1705, as shown in FIG. 42. To receive anchor 1703 in acromion bone 1705, a hole may initially be drilled through acromion bone 1705. In some examples, filament 1702 is passed into and through the hole and anchor 1703 is secured on a far side of acromion bone 1705, opposite a side that faces implant 1710. In examples where the filament 1702 is used to parachute the implant 1710 down to the target implantation site, such filament 1702 may be tensioned. In variations of the method, implant 1710 may be preloaded in an implant delivery instrument 50. And in some examples of this variation, filaments secured to the tissue of the patient may be loaded through opening 1738 prior to use of the implant delivery instrument 50 to deliver the implant 1710 into the patient. In each of the above described aspects, non-limiting examples of an anchor that may be used include a suture anchor, a button, or an interference screw.

As another example, the method 2900 may be performed for implantation of implant 1010, as shown in FIGS. 27-29. The optional method of assembly of an implant system for use with implant 1010 may be as described above for implant system 800 in which the implant 1010 is inserted into sheath 872 of implant delivery instrument 850. In some examples, the implant system is initially provided in an assembled condition, with implant 1010 disposed within sheath 872 of implant delivery instrument 850. In still further examples, implant 1010 is kept separate from implant delivery instrument 850 and independently delivered to an implantation site.

Initially, at operation 2902 of method 2900, anchors 1003A-D are secured to tissue 1005 at locations proximate the desired implantation site. In some examples, two anchors may be used rather than four, e.g., anchors 1003A, 1003B. Then, a filament 1002A is secured to anchor 1003A and a filament 1002B is secured to anchor 1003B. When the implantation site is in a subacromial space, anchors 1003C, 1003D may optionally be on a medial row, while anchors 1003A, 1003B may be on a lateral row. In one variation of this aspect, free end of filament 1002A is drawn out of the patient (or already extends out of the patient) and is passed through opening 1038A on tab 1012A, as shown in FIG. 27. Similarly, the free end of filament 1002B is drawn out of the patient (or already extends out of the patient) and is passed through opening 1038B on tab 1012B. Balloon 1040 is then parachuted down along filaments 1002A, 1002B to the implantation site in the patient. Additionally, the free ends of respective filaments 1002A, 1002B may be used to guide implant 1010 to the desired implantation location. FIG. 28 shows implant 1010 at the implantation site after being slid down filaments 1002A, 1002B passing through openings 1038A-B. To facilitate such conveyance of balloon 1040, filaments 1002A, 1002B may initially be tensioned. When balloon 1040 arrives at a desired location, each filament 1002A, 1002B may be secured to a respective anchor 1003A, 1003B to complete the implant 1010 securement, as shown in FIGS. 29. In another variation of this aspect, implant 1010 is received on implant delivery instrument 850 and sheath 872 is advanced over implant 1010 prior to advancement of implant delivery instrument 850 into the patient. Alternatively, the implant delivery instrument 1050 may be preassembled with the implant 1010 pre-positioned within the sheath 872. At operation 2906 of method 2900, implant delivery instrument 850 is then advanced into the patient and toward the intended implantation site. Proximate the implantation site, or from a location within the patient still at a distance from the implantation site, the sheath 872 is retracted to expose implant 1010. Then, the process of receiving the respective filaments 1002A, 1002B through openings 1038A, 1038B and securing implant 1010 to anchors 1003A, 1003B proceeds as described above.

Alternatively, in yet another variation of this aspect, the filaments 1002A, 1002B may be passed through openings 1038A, 1038B before implant 1010 is attached to implant delivery instrument 850 outside of the patient, with the free ends of the filaments extending through the proximal end 877 of the sheath 872. Then, at operation 2908 of method 2900, sheath 872 may be closed over the implant 1010 loaded with the filaments 1002A, 1002B and the implant delivery instrument 850 may be advanced into the patient. Inside the patient at a location proximate the implantation site, or from a location within the patient still at a distance from the implantation site, sheath 872 of implant delivery instrument 850 is withdrawn and implant 1010 is released. When implant 1010 is released at a distance from the implantation site, filaments 1002A, 1002B may be used to aid in the advancement of implant 1010 over a remainder of a distance to the implantation site. Further, once implant delivery instrument 850 is removed entirely, at operation 2910 of method 2900, each filament 1002A, 1002B may be retrieved from its respective free end for further use to secure implant 1010 to the tissue. In any of the contemplated variations, at operation 2912 of method 2900, implant delivery instrument 850 may be used to inflate balloon 1040 before or after securing implant 1010 to tissue using filaments 1002A-B. Implant 1010 may be secured to tissue 1005 in the same manner as described above for implant 810. The procedure is completed with the withdrawal of the instrument to seal the balloon and the closure of the portal into the patient.

In yet another aspect, a method may be performed for implantation of implant 1110, as shown in FIGS. 30A-30B. Implant 1110 may be implanted in each of the ways described above for implant 1010. As discussed above, FIG. 30A shows implant 1110 folded or rolled up inside sheath 872 of implant delivery instrument 850. Implant delivery instrument 850 may be positioned above an implantation site, and sheath 872 may be retracted to expose and unfold implant 1110. In examples of the method where implant 1110 is parachuted to the implantation site, filaments 1102A, 1102B are passed through openings 1138A, 1138B in tab 1112 and connection portion 1132, respectively, so that balloon 1140 may be advanced down to the implantation site. To facilitate such conveyance of balloon 1140, filaments 1102A, 1102B may initially be tensioned. Respective filaments 1102A, 1102B may be secured to respective anchors 1103A, 1103B to secure implant 1110 to tissue 1105. FIG. 30B shows implant 1110 after filaments 1102A, 1102B have been passed through openings 1138A, 1138B and tensioned to secure implant 1110 to anchors 1103A-B. In examples where implant 1110 is loaded into implant delivery instrument 850, balloon 1140 may be folded without impacting tabs 1112 and connection portion 1132 as shown in FIG. 30A, thereby making the folding of the balloon 1140 less difficult and allowing for a more compact folded shape.

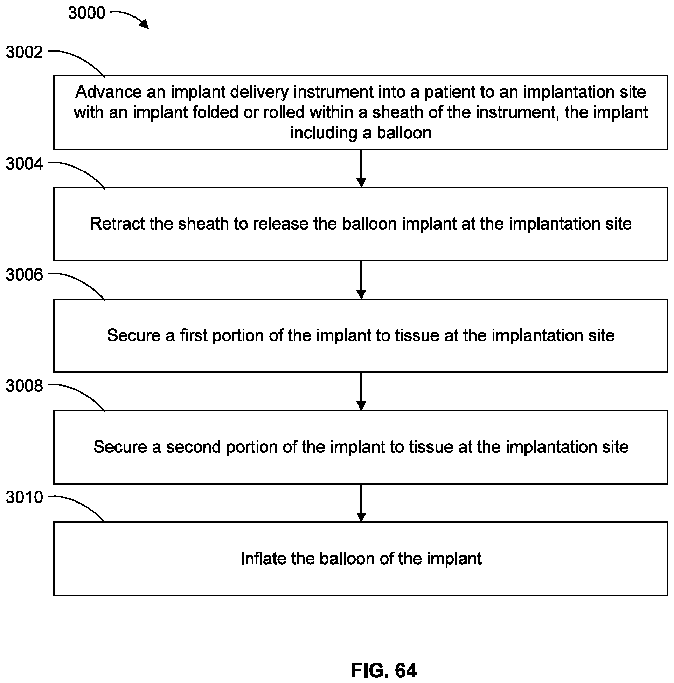

FIG. 64 illustrates a method 3000 of delivering an implant, according to example aspect. One example of the method 3000 is illustrated by the implantation of implant 1210 into a patient, shown in FIGS. 31A-B. At operation 3002 of method 3000, an implant delivery instrument is advanced into a patient to an implantation site with an implant folded or rolled within a sheath of the instrument, the implant including a balloon. For example, the method 3000 may begin with assembly of an implant system using implant delivery instrument 50 assembled as described for implant system 1 above. In some examples, the implant system 1 including implant 1210 and implant delivery instrument 50 is initially provided in an assembled condition with implant 1210 folded or rolled and enclosed within sheath 72, as shown in FIG. 1, but with implant 1210 rather than implant 10. Implant delivery instrument 50 with implant 1210 attached is then directed through a prepared access, i.e., portal into the patient to position a distal end 78 of instrument 50 proximate the target site within the patient, i.e., the location where the implant 1210 is to be deployed within the patient. In some examples, this may be in a subacromial space within the shoulder. At operation 3004 of method 3000, the sheath is retracted to release the implant at the implantation site. For example, the base 70 of sheath 72 may be retracted to expose implant 1210. At operation 3006 of method 3000, a first portion of the implant is secured to tissue at the implantation site, for example, using fixation elements (e.g., staples, tacks, pins, screws, etc.) pressed into tissue through or around a portion of the implant. For example, fixation elements 1203B and 1203C may be placed over a neck portion of respective tabs 1212B and 1212C and pressed into a medial portion of tissue 1205 to secure implant 1210 to tissue 1205, as shown in FIGS. 31A-B. At operation 3008 of method 3000, a second portion of the implant is secured to tissue at the implantation site, for example, using fixation elements (e.g., staples, tacks, pins, screws, etc.) pressed into tissue through or around a portion of the implant. For example, fixation elements 1203A and 1203D may be placed over a neck portion of respective tabs 1212B and 1212C and pressed into a lateral portion of tissue 1205 to secure implant 1210 to tissue 1205, as shown in FIG. 31A. At operation 3010 of method 3000, the balloon of the implant is inflated. For example, balloon 1240 of implant 1210 may be inflated, e.g., via input of air or fluid through operator port 58 of instrument 50. In some examples, the balloon may be partially or completely inflated before the implant is secured to the tissue at the implantation site. Implant delivery instrument 50 may be removed from the patient at any time after inflation. Removal of implant delivery instrument 50 from implant 1210, causes a seal to engage within access into an internal volume of balloon 1240, thereby preserving the inflated condition of the balloon 1240. The procedure is completed with the closure of the portal into the patient.

As another example the method 3000 may be performed for implantation of implant 1310 into a patient, shown in FIGS. 32A-B. Such method may be performed through performance of the same steps as described above for implantation of implant 1210 used with implant delivery instrument 50. As described elsewhere in the present disclosure, each fixation element 1303A-D is configured to either pierce a respective tab 1312A-D or to pass through openings in the tab 1312A-D to secure the tab 1312A-D to tissue 1305. After balloon 1340 is secured to tissue 1305, the balloon 1340 may be inflated using implant delivery instrument 50.

As another example, the method 3000 may be performed for implantation of implant 1410 into a patient as shown in FIGS. 36A-36D. Balloon 1440 may be positioned within internal cavity 1431 of augment 1430 before augment 1430 and balloon 1440 are advanced into the patient. At operation 3002 of method 3000, implant delivery instrument 850 with implant 1410 attached is directed through a prepared access, i.e., portal into the patient to position a distal end 78 of instrument 50 proximate the target site within the patient, i.e., the location where the implant 1410 is to be deployed within the patient. In some examples, this may be in a subacromial space within the shoulder. At operation 3004 of method 3000, sheath 872 is retracted to expose implant 1410. At operation 3006 of method 3000, a first portion of implant 1410 is secured to tissue by pressing fixation element 1403A through a portion of augment 1430 and into tissue 1405. At operation 3008 of method 3000, a second portion of implant 1410 is secured to tissue by pressing fixation element 1403B through a portion of augment 1430 and into tissue 1405. It should be appreciated that securement of augment 1430 to tissue 1405 occurs before receipt of balloon 1440 at the target site, and thus is completed before inflation of balloon 1440. Once augment 1430 is secured as shown in FIG. 33, at operation 3010 of method 3000, implant delivery instrument 50 may be used to receive balloon 1440, deliver balloon to augment 1430 as shown in FIG. 34, and inflate balloon 1440. Once augment 1430 is secured as shown in FIG. 36C, at operation 3010 of method 3000, implant delivery instrument 850 may be used to inflate balloon 1440. In some examples, balloon 1440 may be fully or partially inflated before or after implant 1410 is secured to tissue 1405 or may be inflated after implant 1410 is secured to tissue 1405.

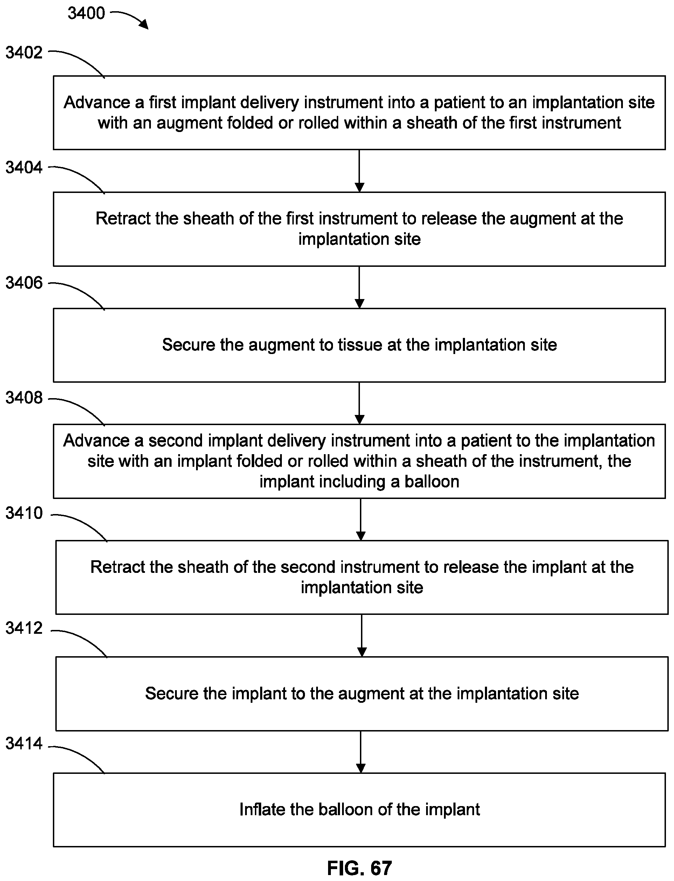

FIG. 67 illustrates a method 3400 of delivering an implant, according to example aspect. One example of the method 3400 is illustrated by the implantation of implant 1410 into a patient, shown in FIGS. 33-35C. In this example, a portion of the implant 1410, augment 1430, may be secured to tissue before another portion of the implant 1410, balloon 1440, is coupled to augment 1430. Balloon 1440 may be coupled to augment 1430, for example, using an adhesive, by suturing a portion of balloon 1440 to augment 1430, by positioning balloon 1440 in an internal cavity of augment 1430, by positioning balloon 1440 between augment 1430 and one or more flexible members coupled to augment 1430, etc. Augment 1430 may be positioned below balloon 1440 such that augment 1430 is facing (e.g., is in contact with) the tendon or other tissue in the joint.

At operation 3402 of method 3400, a first implant delivery instrument is advanced into a patient to an implantation site with an augment folded, rolled, or otherwise collapsed within a sheath of the instrument. For example, a first implant delivery instrument 50 with augment 1430 attached may be directed through a prepared access, i.e., portal into the patient to position proximate the target site within the patient, i.e., the location where the augment 1430 is to be deployed within the patient. In some examples, the implant delivery instrument 50 is initially provided in an assembled condition with augment 1430 collapsed and enclosed within sheath, for example, as shown in FIG. 1 but with augment 1430 in place of implant 10. In some examples, this may be in a subacromial space within the shoulder. At operation 3404 of method 3400, the sheath of the first implant delivery instrument is retracted to release the augment. For example, sheath 72 of the first instrument may be retracted to expose augment 1430. At operation 3406 of method 3400, the augment is secured to tissue at the implantation site. For example, augment 1430 may be secured to tissue 1405 in the same way discussed above with respect to FIG. 36C. It should be appreciated that augment 1430 is secured to tissue 1405 before receipt of balloon 1440 at the target site, and thus is completed before inflation of balloon 1440.

Once augment 1430 is secured to tissue 1405 as shown in FIGS. 33 and 35A, at operation 3408 of method 3400, a second implant delivery instrument is advanced into a patient to the implantation site with an implant folded, rolled, or otherwise collapsed within a sheath of the instrument, the implant including a balloon. For example, a second implant delivery instrument 50 with balloon 1440 attached may be directed through the prepared access, i.e., portal into the patient to position proximate the target site within the patient. In some examples, the implant delivery instrument 50 is initially provided in an assembled condition with balloon 1440 collapsed and enclosed within sheath, for example, as shown in FIG. 1 but with augment 1430 in place of implant 10. In some examples, the same implant delivery instrument used to deliver the augment may subsequently be loaded with the implant and used to deliver the implant. In some examples, this may be in a subacromial space within the shoulder. At operation 3010 of method 3400, the sheath of the second implant delivery instrument is retracted to release the implant. For example, sheath 72 of the second instrument may be retracted to expose balloon 1440. At operation 3412 of method 3400, the implant is secured to the augment. In the example depicted in FIGS. 33-35C, augment 1430 includes an internal cavity configured to receive balloon 1440 when delivered via implant delivery instrument 50. For example, at operation 3412, balloon 1440 may be positioned within internal cavity 1431 of augment 1430 to secure balloon 1440 to augment 1430. In some examples, sheath 72 may be positioned within internal cavity 1431 with balloon 1440 in sheath 72 when sheath 72 is retracted, thus releasing balloon 1440 from sheath 72 within internal cavity 1431. In some examples, the tissue augment may have an internal cavity with two or more openings. in some examples, an implant (e.g., a balloon) may be secured to an augment at operation 3412 using other methods as discussed above (e.g., sutures, adhesive, flexible members, etc.). At operation 3414 of method 3400 the balloon of the implant is inflated. In some examples, balloon 1440 may be partially inflated before being inserted into internal cavity 1431 of augment 1430.

As another example, the method 3400 may be performed for implantation of implant 1510 into a patient as shown in FIGS. 38A-38B. In this method, augment 1530, inclusive of straps 1581 as shown in FIG. 38A, is placed onto tissue 1505 and/or soft tissue 1506, such as a rotator cuff, using, in some examples, an implant delivery device as described in the method 3400 utilizing augment 1430, and then secured onto soft tissue 1506 via fixation elements 1503A-D. Fixation elements 1503A-D may be as described elsewhere in the present disclosure. For example, fixation elements may be tacks, staples, nails, retention screws and/or any other mechanical fastener suitable for securing implant 1510 to soft tissue 1506 of a patient. In other examples, augment 1530 may secured directly to tissue 1505 using filaments (e.g., sutures) pressed through the material of augment 1530 as discussed above. It should be appreciated that securement of augment 1530 to soft tissue 1506 occurs before receipt of balloon 1540 at the target site, and thus is completed before inflation of balloon 1540. For example, at operation 3402, a first implant delivery instrument including a sheath with augment 1530 may be advanced into the patient. At operation 3404, the sheath may be retracted to release augment 1530. At operation 3404, the sheath may be retracted to release augment 1530. At operation 3406, augment 1530 may be secured to tissue 1505 with fixation elements 1503A-D. Once augment 1530 is secured as shown in FIG. 38A, at operation 3408, a second implant delivery instrument 50 with balloon 1540 with its sheath 72 may be advanced to the implantation site in the patient. At operation 3410, sheath 72 may be retracted to release balloon 1540. At operation 3412, balloon 1540 may be received within straps 1581 as shown in FIG. 38B to retain balloon 1540, i.e., capture the balloon, relative to augment 1530. At operation 3414, inflation of balloon 1540 may commence once balloon 1540 is captured within straps 1581 of the previously secured augment 1530. As discussed above, straps 1581 allow balloon 1540 to be easily secured to augment 1530 and the implantation site, as straps 1581 tension around balloon 1540 as balloon 1540 is inflated without the need for subsequent operations to couple balloon 1540 to augment 1530 or tissue 1505. In a variation of this aspect, balloon 1540 and augment 1530 may be pre-assembled within the balloon/augment assembly (e.g., with the balloon 1540 positioned within the straps 1581) and then delivered and implanted in a similar manner to the variant of the method utilizing implant 1410. Providing balloon 1540 and augment 1530 separately may allow for a smaller opening (e.g., portal) into the patient and/or a larger implant 1510 compared to examples in which a balloon and an augment are passed through a portal while coupled together.

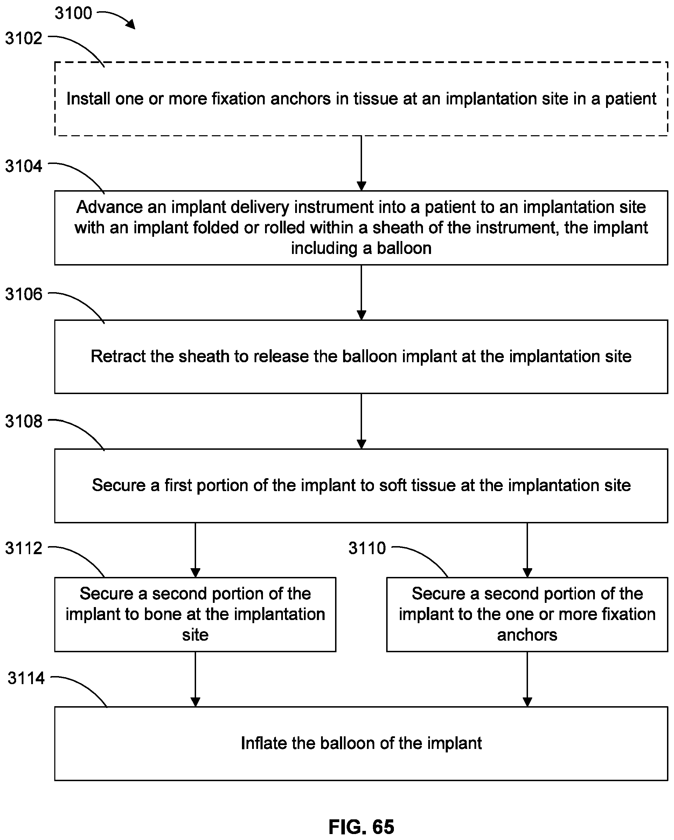

FIG. 65 illustrates a method 3100 of delivering an implant, according to example aspect. One example of the method 3100 is illustrated by the implantation of implant 2010 into a patient, shown in FIG. 37A. An implant delivery instrument (e.g., implant delivery instrument 850) may be used to deliver implant 2010 to an implantation site in a patient. The method 3100 may include operation 3102 in which one or more fixation anchors are installed in tissue at the implantation site. For example, as shown in FIG. 37A, fixation elements 2003C-D are installed into tissue 2005. In some examples, the method 3100 may include performing a joint repair procedure (e.g., a double row repair of a rotator cuff), for example, by coupling anchors together using sutures, as discussed above with respect to operation 2903. At operation 3104 of method 3100, an implant delivery instrument may be advanced into a patient to an implantation site with an implant folded or rolled within a sheath of the instrument, the implant including a balloon. For example, implant 2010 may be rolled or folded and positioned within sheath 872 of implant delivery instrument 850, and implant delivery instrument 850 may be advanced into the patient to the implantation site. At operation 3106 of method 3100, the sheath is retracted to release the implant at the implantation site. For example, sheath 872 of implant delivery instrument 850 may be retracted to expose implant 2010.

At operation 3108 of method 3100, a first portion of the implant is secured to soft tissue (e.g., tendon tissue) at the implantation site, for example, using fixation elements (e.g., staples, tacks, screws, etc.) pressed into the tendon tissue through or around a portion of the implant. The tendon tissue may be in a medial portion of the joint. For example, as shown in FIG. 37A, fixation elements 2003A, 2003B are pressed through augment 2030 into soft tissue 2006 (e.g., tendon tissue). At operation 3110 of method 3100, a second portion of the implant is secured to the fixation anchors at the implantation site. For example, as shown in FIG. 37A, leg portions 2007A, 2007B are secured to fixation elements 2003D and 2003C, respectively. Leg portions 2007A-B may be stitched, tied, adhered, or otherwise coupled in various ways to the fixation elements 2003C-D (e.g., to eyelets of fixation elements 2003C-D). In some examples, leg portions 2007A-B may be secured to fixation elements 2003C-D prior to or upon installation of fixation elements 2003C-D. For example, leg portions 2007A-B may be coupled to or passed through an eyelet in a threaded portion of fixation elements 2003C-D (e.g., twist anchors), and the installation of fixation elements 2003C-D to tissue 2005 may secure leg portions 2007A-B to tissue 2005. The fixation elements 2003-D may be secured, for example, to bone in a lateral portion of the joint. At operation 3114 of method 3100, the balloon (e.g., balloon 2040) may be inflated.

As another example, the method 3100 may be performed for implantation of implant 1410 into a patient as shown in FIG. 37B. The method 3100 of delivering implant 1410 shown in FIG. 37B may be substantially the same as the method 3100 shown in FIG. 37A, except that operation 3110 is replaced by operation 3112. At operation 3112, the second portion of the implant is secured to bone at the implantation site, for example, using fixation elements (e.g., staples, tacks, screws, etc.) pressed into the bone through or around a portion of the implant. For example, as shown in FIG. 37B, fixation elements 2003H and 2003G are pressed through leg portions 2007C and 2007D, respectively, and into tissue 2005 (e.g., bone). The fixation elements 2003H and 2003G may be secured, for example, to bone in a lateral portion of the joint. In this example, method 3100 may not include operation 3102. However, anchors 2003I and 2003J may have been installed in tissue 2005 for other purposes during the surgical procedure.

In another aspect, a method includes implantation of implant 1610 into a patient, as shown in FIGS. 39-41. Optionally, this method may begin following a previously completed rotator cuff repair, such as a double cuff repair. In the depicted aspect, the method begins after such a repair, with filaments 1691 of the double cuff repair lapped over soft tissue 1606, i.e., rotator cuff tissue, and attached to respective pairs of fixation anchors 1603A, 1603C and 1603B and 1603D. In the example shown, the cuff repair is completed with filaments crossing each other. In other examples, the filaments may be oriented in parallel and/or in any other orientation suitable for a cuff repair. Returning to the method, in an initial step, additional filaments are attached (or have previously been attached) to the previously established fixation anchors. Specifically, respective filaments 1602A-D are attached to respective fixation anchors 1603A-D, as shown in FIG. 39. Then, an implant delivery instrument may be used, for example, in the ways described in the method utilizing augment 1430, to deliver augment 1630 to the implantation site. In this manner, augment 1630 may be loaded onto the implant delivery instrument in any of the ways described for the method utilizing augment 1430. Once delivered, augment 1630 may be attached to soft tissue 1606 via augment fixation elements 1613A-D, as shown in FIG. 40. In a variation, augment 1630 may be attached to other tissue. The augment fixation elements 1613A-D may be u-shaped bars, although in other examples, the augment fixation anchors may be tacks, staples, nails, retention screws and/or any other mechanical fastener suitable for attaching augment 1630 to tissue 1606 of a patient. It should be appreciated that securement of augment 1630 to soft tissue 1606 occurs before receipt of balloon 1640 at the target site, and thus is completed before inflation of balloon 1640.

The method continues with delivery of balloon 1640 to the implantation site via the implant delivery instrument. In some examples, the implant delivery instrument may be used in a similar manner to that contemplated for delivery of augment 1630. Filaments 1602A-D, which remain fixed to respective fixation anchors 1603A-D, each include a free end that may be passed through a respective opening 1638A-D in a tab 1612A-D of balloon 1640 (e.g., outside of the patient). Balloon 1640 may then be guided (e.g., parachuted) into the implantation side along the filaments 1602A-D. Balloon 1640 is then released from the implant delivery instrument over augment 1630. Each filament 1602A-D is then attached (e.g., tied) to the corresponding tab 1612A-D, as shown in FIG. 41, or secured back to the respective anchor 1603A-D (e.g., directly or to a second filament, for example, using a self-locking feature such as a finger trap) while looped through the respective opening 1638A-D. Thus, with four fixation anchors 1603A-D and four tabs 1612A-D on balloon 1640, the balloon is secured at four different locations. In this configuration, balloon 1640 rests on augment 1630 but is not attached to augment 1630. Further, augment 1630 is positioned such that it is in between soft tissue 1606 and balloon 1640. The method may be completed with inflation of balloon 1640 and withdrawal of any instrumentation from the patient. In an alternative arrangement using only two filaments, each filament may be coupled to a first fixation anchor 1603A-D, passed through two openings 1638A-D in balloon 1640, and coupled to a second fixation anchor 1603A-D. The filaments may be arranged in parallel, similar to filaments 802B' and 802C' as shown in FIGS. 21-24, or may crisscross over balloon 1640, similar to filaments 802B' and 802C' as shown in FIGS. 25A-25D.

As another example, the method 2900 may be performed for implantation of implant 1810 or implant 1910 as shown in FIGS. 43-46 and FIGS. 47-50, respectively. In these examples, the implants 1810, 1910 include a balloon 1840, 1940 that connected to an augment 1830, 1930 as a single unit before implantation. In FIGS. 43-46, balloon 1840 extends beyond the perimeter of augment 1830 (which may be coupled to an underside of balloon 1840), and implant 1810 is coupled to tissue 1805 via fixation members or tabs 1812A-D. In FIGS. 47-50, augment 1930 extends beyond the perimeter of balloon 1940, and implant 1910 is coupled to tissue 1905 through the edges or corner of augment 1930, for example, using openings 1938A-D or by pressing a fixation anchor directly through the material of augment 1930. An intended implantation location may be in a joint of the patient, such as the shoulder. In such cases, tissue 1805, 1905 onto which the implant 1810, 1910 may be placed may be one of the acromion, clavicle or humerus. The optional method of assembly of an implant system for use with implant 1810, 1910 may be as described above for implant system 800. In some examples, the implant system is initially provided in an assembled condition. In still further examples, implant 1810, 1910 is kept separate from implant delivery instrument 850 and independently delivered to an implantation site.

In this method, the repair of soft tissue 1806, 1906 is addressed by placement of implant 1810, 1910 over a tissue repair as shown in FIGS. 43-46 and FIGS. 47-50. In this manner, the securement of implant 1810, 1910 provides and/or supplements a repair of soft tissue 1806. For the sake of brevity, the following description refers to implant 1810 (as shown in FIGS. 43-46), though it should be appreciated that the same steps may apply with the use of implant 1910 (as shown in FIGS. 47-50). Initially and as shown in FIG. 43, at operation 2902 of method 2900 fixation anchors 1803A-1803D are secured to tissue 1805 at locations proximate the desired implantation site. Then, a filament 1802B is secured to fixation anchor 1803B and a filament 1802C is secured to fixation anchor 1803C. In some examples, the filaments 1802B, 1802C may have been previously attached to the fixation anchors 1803B, 1803C, for example, at the time of a double row repair. When the implantation site is in a subacromial space, fixation anchors 1803B, 1803C may optionally be on a medial side, while fixation anchors 1803A, 1803D may be on a lateral side.

In one variation of this example, at operation 2904 of method 2900, a free end of filament 1802B is drawn out (or already extends out) of the patient and is passed through opening 1838B at one corner of implant 1810 and opening 1838A at another corner of implant 1810, as shown in FIG. 44. Similarly, free end of filament 1802C is drawn out (or already extends out) of the patient and is passed through opening 1838C at one corner of implant 1810 and opening 1838D at another corner of implant 1810. In some examples, the filaments 1802B, 1802C may be passed through openings 1838A-1838D while implant 810 is attached to implant delivery instrument 850 outside of the patient, with the free ends of the filaments extending through the proximal end 877 of the sheath 872. Free ends of filaments 1802B, 1802C may then be passed through respective fixation anchors 1803A, 1803D, as shown in FIG. 45. Then, at operation 2906 of method 2900, the free ends of respective filaments 1802B, 1802C are pulled and tensioned to guide implant 1810 (and in some examples, the implant delivery instrument 850) down to the desired implantation site location. This occurs because each filament 1802B, 1802C is secured to one fixation anchor and passes through another fixation anchor, and in this way, when the filaments 1802B, 1802C are pulled, a length of each respective filament between the fixation anchors decreases, pulling implant 1810 toward a space between the fixation anchors 1803A-1803D. If the implant delivery instrument 850 is used, at operation 2908 of method 2900, the implant 1810 may then be removed from the sheath 872 at the implantation site. After tensioning filaments 1802B, 1802C, at operation 2910 of method 2900, the free ends of filaments 1802B, 1802C may then be secured to fixation anchors 1803A, 1803D, respectively. Further, in an alternative arrangement, filaments 1802B, 1802C may be crisscrossed over balloon 1840 when traversing a distance between two openings from among openings 1838A-1838D, as shown and described with respect to implant 810 in FIGS. 25B-25D. Thus, balloon 1840 of implant 1810 may be similar or equivalent to balloon 840 of implant 810 and may be implanted in a manner similar to that shown in FIGS. 25A-25D. At operation 2912 of method 2900, after or before securing filaments 1802B, 1802C to fixation anchors 1803A, 1803D, the balloon 1840 may be inflated.

As another example, the method 2900 may be performed for implantation of implant 810 as shown in FIGS. 19C-H. In this example, rather than tying filaments directly to anchors as shown, for example, in FIGS. 24 and 25B, a filament may be fastened to an anchor in operation 2910 by passing the filament through an opening in an anchor and fastening the free end of the filament to another portion of the filament (e.g., using a finger trap). For example, as shown in FIGS. 19C-19I, filament 2102D, which is coupled to anchor 2103D, is passed through openings 838A, 838B in tabs 812A, 812B, and passed through an opening in anchor 2103A. The free end of filament 2102D is then coupled to a more proximal portion of filament 2102D using finger trap 2189D.

In yet another aspect, a method includes implantation of implant 1810 or implant 1910 into a patient using the method as shown in FIGS. 20A-20B and as described above for implant 810. In still further aspects, a method includes implantation of implant 1810 or implant 1910 into a patient using the method as shown in FIGS. 27-29 and as described above for implant 1010. When implant 1810 is used in the aspect shown in FIGS. 27-29, the balloon 1840 of implant 1810 may optionally include a conduit similar to conduit 1044 and fixation members similar to fixation members 1012A-B, as shown in FIGS. 27-29. Said another way, balloon 1840 of implant 1810 may be similar or equivalent to balloon 1040 of implant 1010. When implant 1910 is used in the aspect shown in FIGS. 27-29, augment 1930 may include a fixation member, e.g., an extension or tab on one side with two openings defined therein each sized to receive a filament. In still further aspects, a method includes implantation of implant 1810 or implant 1910 into a patient using the method shown in FIGS. 30A-30B and as described above for implant 1110. When implant 1810 is used in the aspect shown in FIGS. 30A-30B, the balloon 1840 of implant 1810 may optionally include fixation members similar to tab 1112 as shown in FIG. 30B. When implant 1910 is used in the aspect shown in FIGS. 30A-30B, augment 1930 may include a fixation member on each side of opposing sides of augment 1930, where each fixation member defines an opening sized to receive a filament.

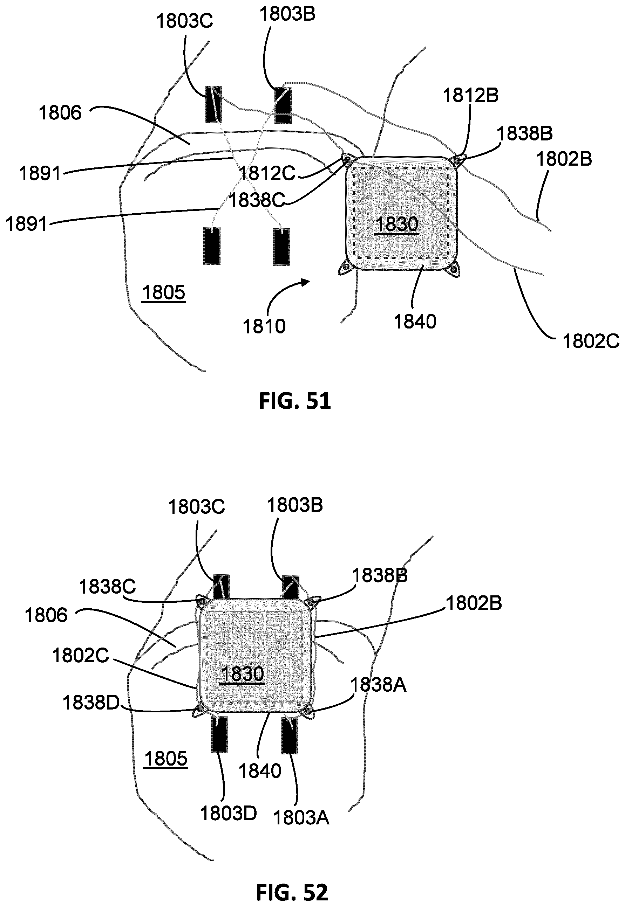

As additional examples, the method 2900 may be performed for implantation of implant 1810 or implant 1910 as shown in FIGS. 51-52 and FIGS. 53-54, respectively. An intended implantation location may be in a joint of the patient, such as the shoulder. In such cases, tissue 1805, 1905 onto which the implant 1810, 1910 may be placed may be one of the acromion, clavicle or humerus. The optional method of assembly of an implant system for use with implant 1810, 1910 may be as described above for implant system 800. In some examples, the implant system is initially provided in an assembled condition. In still further examples, implant 1800, 1900 is kept separate from implant delivery instrument 850 and independently delivered to an implantation site.

In one example of this aspect shown in FIGS. 51-52, the method of implant 1810 placement into the patient follows a previously completed repair of soft tissue at the target site, for example, a double row repair. This is shown via filaments 1891 in FIG. 51, such filaments 1891 crossing between anchors 1803A-D to repair soft tissue 1806, e.g., a tear in soft tissue 1806. In some examples, soft tissue 1806 is a tendon in the shoulder joint. With the double row repair already in place, additional filaments 1802B, 1802C are attached to the respective fixation anchors 1803B, 1803C (or were attached at the time of the double row repair), e.g., medial fixation anchors, so that each filament 1802B, 1802C extends from respective fixation anchors 1803B, 1803C. Then, in one variation of this example, free ends of each filament 1802B, 1802C are drawn out of the patient so that each filament 1802B, 1802C may be passed through a respective opening 1838B, 1838C at corners of implant 1810, as shown in FIG. 51. From this position, each filament 1802B, 1802C is tensioned, and then implant 810 is guided down, i.e., parachuted down into the patient via filaments 1802B, 1802C to the intended implantation site proximate fixation anchors 1803A-D.

When implant 1810 is positioned at the implantation site, filaments 1802B, 1802C may be tensioned and then attached or otherwise passed through respective fixation anchors 1803A, 1803D, as shown in FIG. 52. Tensioning of the filaments may reduce a length of the installed filament. In some examples, filaments 1802B, 1802C are first passed through respective openings 1838A, 1838D of balloon 1840 and then attached to fixation anchors 1803A, 1803D. In other examples, each fixation anchor 1803A-D may include a separate filament 1802A-D coupled thereto that may be coupled to an opening 1838A-D in the implant 1810, as discussed above.

In a variation of the above-described example, implant 1810 is received on implant delivery instrument 850 and sheath 872 is advanced over implant 1810 prior to advancement of implant delivery instrument 850 into the patient and toward the intended implantation site. Implant delivery instrument 850 is then advanced into the patient, and sheath 872 is then retracted to expose implant 1810. With implant 1810 exposed, the respective filaments 1802B, 1802C are passed through respective openings 1838B, 1838C of implant 1810. In such variation, implant delivery instrument 850 may be advanced part way to the implantation site or fully to the implantation site. When implant delivery instrument 850 is not advanced fully to the implantation site, exposed implant 1810 may be released and then parachuted down for a remainder of the distance to the implantation site. Alternatively, in yet another variation of this example, the filaments 1802B, 1802C may be passed through openings 1838B, 1838C while implant 1810 is attached to implant delivery instrument 850 outside of the patient, with the free ends of the filaments 1802B, 1802C run through the proximal end 877 of the sheath 872. Then, the sheath 872 may be closed over the implant 1810 that is loaded with the filaments 1802B, 1802C, and the implant delivery instrument 850 may be advanced into the patient. Inside the patient, sheath 872 of implant delivery instrument 850 is withdrawn and implant 1810 is released. The free ends of filaments 1802B-C may be tensioned and secured to fixation anchors 1803A-B respectively, as discussed above. As with the variation described immediately above, implant delivery instrument 850 may be advanced part way to the implantation site or fully to the implantation site. When implant delivery instrument 850 is not advanced fully to the implantation site, exposed implant 1810 may be released and then parachuted down for a remainder of the distance to the implantation site. Further, once implant delivery instrument 850 is removed from the patient, which follows disengagement of implant delivery instrument 850 from implant 1810, each filament 1802B, 1802C may be retrieved from its respective free end for further use.

In variations where implant delivery instrument 850 is used to advance implant 1810, 1910 to the implantation site, implant delivery instrument 850 may similarly be used to inflate balloon 1840, 1940. The procedure is completed with the withdrawal of the implant delivery instrument 850, sealing of the balloon 1840, 1940 and closure of the portal into the patient.

In another aspect, a method may be performed as described above and shown in FIGS. 51-54 for the methods of implantation of implant 1810, 1910 but without a preceding double row repair to repair tissue. Instead, anchors may be implanted into tissue in the first instance and respective filaments may be secured to such anchors to prepare the implant receiving site for the subsequent method steps.

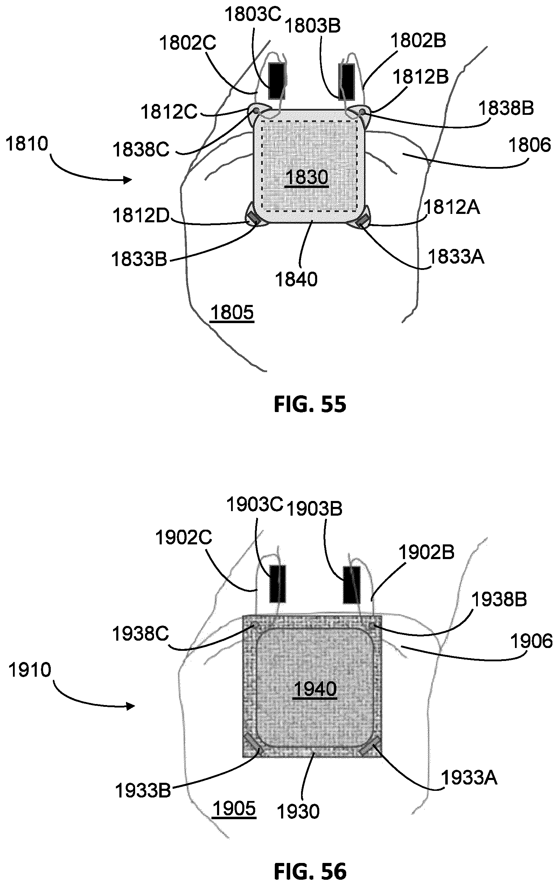

In yet another aspect, methods may be performed for implantation of implant 1810 or implant 1910 as shown in FIGS. 55 and 56, respectively. An intended implantation location may be in a joint of the patient, such as the shoulder. In such cases, tissue 1805, 1905 onto which the implant 1810, 1910 may be placed may be one of the acromion, clavicle or humerus. The optional method of assembly of an implant system for use with implant 1810, 1910 may be as described above for implant system 800. In some examples, the implant system is initially provided in an assembled condition. In still further examples, implant 1800, 1900 is kept separate from implant delivery instrument 850 and independently delivered to an implantation site.

In one example of the aspect shown in FIG. 55, the method of implant 1810 placement into the patient may optionally follow a previously completed joint repair at the target site (e.g., a double-row repair of a rotator cuff). In some examples, soft tissue 1806 at the target site is a tendon in the shoulder joint. With the double row repair already in place, additional filaments 1802B, 1802C are attached to the respective fixation anchors 1803B, 1803C (or were attached at the time of the double row repair), e.g., medial fixation anchors, so that each filament 1802B, 1802C extends from respective fixation anchors 1803B, 1803C. Then, in one variation of this example, free ends of each filament 1802B, 1802C, which may be outside of the patient, may be passed through a respective opening 1838B, 1838C at corners of implant 1810, as shown in FIG. 55. From this position, each filament 1802B, 1802C is tensioned, and then implant 810 is guided down, i.e., parachuted down into the patient via filaments 1802B, 1802C to the intended implantation site proximate fixation anchors 1803B-C. Then, as shown in FIG. 55, filament 1802B, 1802C may be attached or otherwise passed back through respective fixation anchors 1803B, 1803C to secure one side of implant 1810 to tissue 1805, e.g., a medial side. Alternatively, suture may be knotted at the fixation anchors 1803B, 1803C. Fixation elements 1833A-B are then pressed through fixation members, e.g., tabs 1812A, 1812D and into tissue 1805 as shown in FIG. 55 to secure implant 1810 to tissue 1805, e.g., on a lateral side. Fixation elements 1833A-B may be the same as fixation elements 1203A-D shown in FIGS. 31A-B or, for example, tacks, pins, staples, etc. While FIG. 55 shows an implant 1810 including a balloon 1840 with an augment coupled thereto, in some examples, the method of securing implant 1810 may be used in an implant that includes a balloon without an augment.

FIG. 56 illustrates a method of implanting implant 1910 that is similar to the method of implanting implant 1810 discussed above with respect to FIG. 55, except that implant 1910 is coupled to tissue 1905 at the implantation site via the augment 1930 rather than via the balloon 1940 (e.g., in contrast to implant 1810 being coupled to tissue 1805 by balloon 1840). Filament 1902C, which is coupled to fixation anchor 1903C, is passed through opening 1938C in augment 1930 (e.g., outside the patient), and filament 1902B, which is coupled to fixation anchor 1903B, is passed through opening 1938B in augment 1930 (e.g., outside the patient). Implant 1910 may then be guided into the patient along the filaments 1902B-C to the implantation site. Filaments 1902B-C may then be used to secure implant 1910 to fixation anchors 1903B-C, respectively, for example, by tying the ends of filaments 1902B-C to fixation anchors 1903B-C, respectively. Fixation elements 1933A-B may then be then pressed through the corners of balloon 1640 and into tissue 1905 as shown in FIG. 56 to secure implant 1910 to tissue 1905, e.g., on a lateral side. Fixation elements 1933A-B may be the same as fixation elements 1203A-D shown in FIGS. 31A-B or, for example, tacks, pins, staples, etc.

In variations of the examples discussed above with respect to FIGS. 55 and 56, implant 1810 or implant 1910 may be received on implant delivery instrument 850 for use in the method in the same ways as described above for the methods discussed above with respect to FIGS. 51-54. In examples of the methods shown in FIGS. 55 and 56 where implant 1810 or 1910 is advanced without an instrument, implant delivery instrument 850 may then afterward be secured to an inlet (not shown) of balloon 840, 940 to inflate the balloon 840, 940 via air or fluid. In variations where implant delivery instrument 850 is used to advance implant 1810, 1910 to the implantation site, implant delivery instrument 850 may similarly be used to inflate balloon 1840, 1940. The procedure is completed with the withdrawal of the implant delivery instrument 850, sealing of the balloon 1840, 1940 and closure of the portal into the patient.

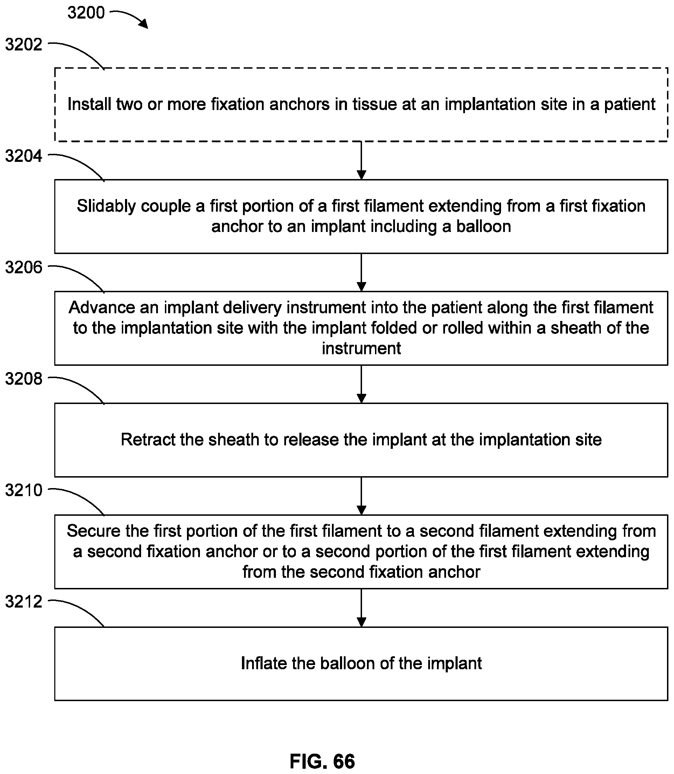

FIG. 66 illustrates a method 3200 of delivering an implant, according to example aspect. One example of the method 3200 is illustrated by the implantation of implant 810 into a patient, shown in FIGS. 19J-19P. An implant delivery instrument (e.g., implant delivery instrument 950) may be used to deliver implant 2010 to an implantation site in a patient. The method 3200 may include operation 3202 in which on or more fixation anchors are installed in tissue at the implantation site. For example, as shown in FIG. 19J, anchors 2203C-D are installed into tissue 2205. In some examples, the method 3100 may include performing a joint repair procedure (e.g., a double row repair of a rotator cuff), for example, by coupling anchors together using sutures, as discussed above with respect to operation 2903.

At operation 3204 of method 3200, a first portion of a filament extending from a first fixation anchor is slidably coupled to an implant including a balloon (e.g., balloon 840 of implant 810). The filament may be passed through one or more openings in the implant (e.g., in a peripheral portion of the implant) or directly through material (e.g., tabs extending from the implant) in one or more locations. For example, as shown in FIG. 19L, the free end of filament 2202C', which extends from fixation anchor 2203B, is passed through opening 838B in tab 812B (shown in FIG. 19P) of implant 810 and through opening 838C of tab 812C (shown in FIG. 19P), while the implant 810 is positioned in the implant delivery instrument 950 (e.g., similar to as shown in FIG. 19D), and the free end of filament 2202D', which extends from fixation anchor 2203B, is passed through opening 838A in tab 812A (shown in FIG. 19P) of implant 810 and through opening 838D of tab 812D (shown in FIG. 19P), while the implant 810 is positioned in the implant delivery instrument 950.

At operation 3206 of method 3200, an implant delivery instrument may be advanced into the patient along the first filament to an implantation site with the implant folded or rolled within a sheath of the instrument. For example, implant 810 may be rolled or folded and positioned within sheath 872 of implant delivery instrument 950, and implant delivery instrument 950 may be advanced into the patient to the implantation site along (e.g., guided by) filament 2202C' and/or 2202D'. At operation 3208 of method 3200, the sheath is retracted to release the implant at the implantation site. For example, sheath 872 of implant delivery instrument 950 may be retracted to expose implant 810.

At operation 3210 of method 3200, the first portion of the first filament is secured to a second filament extending from a second anchor or to a second portion of the first filament coupled to the second anchor. For example, as shown in FIG. 19P, to secure implant to tissue 2205, filament 2202D' and/or filament 2202D may be tensioned with finger trap 2289D maintaining the tension on the filaments 2202D, 2202D', and filament 2202C' is secured to filament 2202C through finger trap 2289C and tensioned. As discussed above, filaments 2202C and 2202C' may be first and second portions of a single filament, and filaments 2202D and 2202D' may be first and second portions of a single filament, rather than all of filaments 2202C, 2202D, 2202C', and 2202D' being separate filaments. At operation 3212 of method 3200, the balloon (e.g., balloon 840) may be inflated. In some examples, the balloon may be partially or completely inflated after the filaments have been partially tensioned. For example, the filaments may be partially tensioned, then the balloon may be partially inflated, then the filaments may be fully tensioned, then the balloon may be fully inflated. Other examples may include further intermediate tensioning and inflation steps.

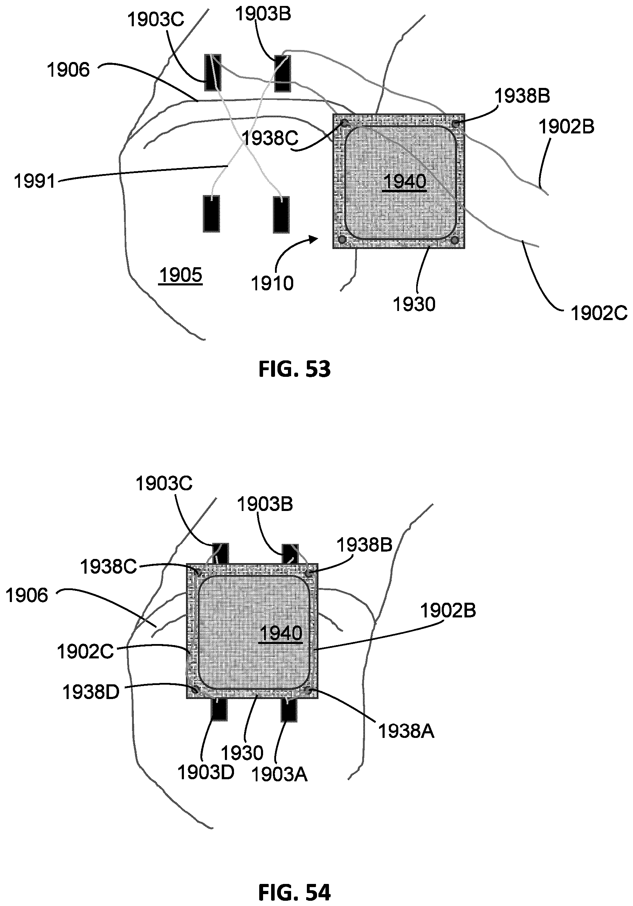

FIGS. 53-54 illustrate a method of implanting implant 1910 that is similar to the method of implanting implant 1810 discussed above with respect to FIGS. 51-52, except that implant 1910 is coupled to tissue 1905 at the implantation site via the augment 1930 rather than via the balloon 1940 (e.g., in contrast to implant 1810 being coupled to tissue 1805 by balloon 1840). As shown, the method follows a previously completed repair of soft tissue at the target site, for example, a double row repair. This is shown via filaments 1991 in FIG. 53, such filaments 1991 crossing between anchors 1903A-D to repair soft tissue 1906, e.g., a tear in soft tissue 1906. Filament 1902C, which is coupled to fixation anchor 1903C, is passed through opening 1938C in augment 1930 (e.g., outside the patient) and then through opening 1938D in augment 1930. Filament 1902B, which is coupled to fixation anchor 1903B, is passed through opening 1938B in augment 1930 (e.g., outside the patient) and then through opening 1938A. Implant 1910 may then be guided into the patient along the filaments 1902B-C to the implantation site. Filaments 1902B-C may then be used to secure implant 1910 to fixation anchors 1903A and 1903D, respectively, for example, by tying the ends of filaments 1902B-C to fixation anchors 1903A and 1903D, respectively. In an alternative arrangement, filaments 1902B, 1902C may be crisscrossed over implant 1910.

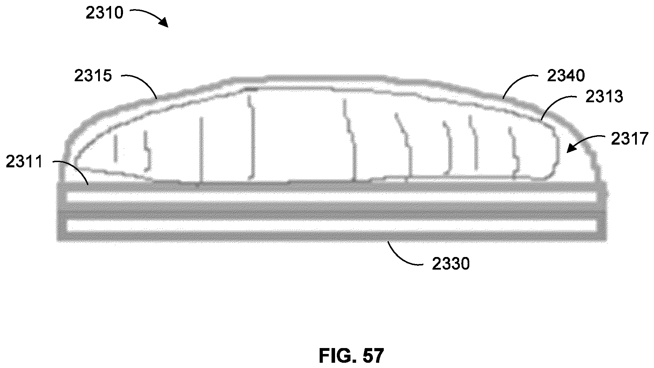

Referring now to FIG. 57, an implant 2310 is shown according to an example aspect. Implant 2310 may be similar to implants 1410, 2010 and like reference numerals refer to like elements of implants 1410, 2010 but within the 2300-series of numerals. Implant 2310 includes an augment 2330 and a balloon 2340. Augment 2330 and balloon 2340 may be substantially similar to the other augments and balloon discussed herein, however, balloon 2340 may be manufactured by electrospinning material (e.g., polymer material) onto augment 2330 (which may be made of collagen). A base layer 2311 of balloon 2340 may first be applied to augment 2330 using electrospinning. A mold form 2313 may then be placed on the base layer 2311. Additional material may then be applied all around the mold form using electrospinning to form the body 2315 of the balloon 2340. The mold form 2313, which may be made of a water soluble material (e.g., a salt), may then be removed to form an inner cavity 2317 of balloon 2340. For example, water may be inserted into an opening in balloon 2340 to dissolve mold form 2313 to form inner cavity 2317. By using electrospinning to form ballon 2340 directly on augment 2330, no adhesive may be required to bond balloon 2340 to augment 2330, and the manufacturing process may be simplified.

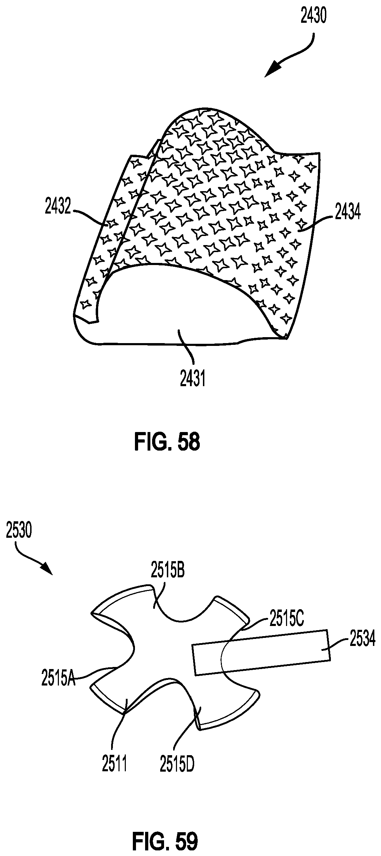

Referring now to FIG. 58, an augment 2430 is shown according to an example aspect. Augment 2430 includes a cavity 2431 configured to receive a balloon (e.g., balloon 1440, balloon 2040, etc.). Cavity 2431 is accessible from openings at opposite ends of augment 2430. A closed first edge 2432 and closed second edge 2434 extend between the opposite openings. As shown in FIG. 58, the edges 2432, 2434 are continuously closed. In other examples, either or both of edges 2432, 2434 may be non-continuously closed. For example, edges 2432, 2434 may have periodic or intermittent openings. In some examples, the openings in edges 2432, 2434 may not be large enough to allow a balloon inserted into one of the openings on the open ends of augment 2430. Augment 2430 may be used in various ways in a surgical procedure as discussed above with respect to the other augments described herein and may be made with similar materials. For example, augment 2430 may be similar to augment 1430, and cavity 2431 may be similar to internal cavity 1431 of augment 1430. Thus, when augment 2430 is secured to tissue of a patient and a balloon (e.g., balloon 1440) is disposed in the internal cavity 2431 of augment 2430 and coupled to augment 2430, balloon 1440 may also be secured to the tissue via augment 2430. Similar to the aspect shown in FIG. 35A, after the augment 2430 and the balloon are attached to the tissue, the balloon may be inflated. The volume of the internal cavity 2431 and/or the flexibility of the augment 2430 may be sufficient to allow inflation of the balloon. Augment 2430 may be secured to tissue using any of the methods for coupling augments to tissue described herein. For example, fixation elements (e.g., u-shaped bars, staples, pins, screws, etc.) may be pressed through edges 2432, 2434 of augment 2430 and into tissue. Alternatively, filaments or sutures may be passed through edges 2432, 2434 and coupled to tissue or anchors previously coupled to the tissue. In other examples, edges 2432, 2434 may include openings or eyelets configured to receive a suture or filament for securing augment 2430 to tissue. In still other examples, a balloon in the cavity 2431 may be secured directly to tissue such that augment 2430 is secured to tissue via the balloon. Augment 2430 may be inserted into an implantation site using implant delivery instrument 850, 950, or any of the other implantation delivery instruments disclosed herein. For example, augment 2430 may implanted using implant delivery instrument 850 as shown in FIGS. 36A-36D, with augment 2430 in place of augment 1430.

Referring now to FIG. 59, an augment 2530 is shown according to an example aspect. Augment 2530 may be used in various ways in a surgical procedure as discussed above with respect to the other augments described herein and may be made with similar materials. Augment 2530 may have a body 2511 made from a balloon-type structure with peripheral portions spaced apart by cut outs. As shown in FIG. 59, body 2511 of augment 2530 includes first, second, third and fourth peripheral portions 2515A-D, each being spaced apart from the others by cut outs in body 2511 providing access to an internal volume of body 2511 from multiple directions. The internal volume of body 2511 is also sized to receive a balloon (e.g. balloon 1440). The internal volume of body 2511 is expandable in conjunction with expansion of the balloon from within body 2511. Augment 2530 may also optionally include a supplemental material segment 2534 to modify receiver assembly 400 into a two body assembly (e.g., with a second body 2511 coupled to the opposite end of supplemental material segment 2534). Supplemental material segment 2534 may also be used to secure augment 2530 to tissue by for example, pressing a fixation anchor, filament, or suture through supplemental material segment 2534 or through another portion of augment 2530. Augment 2530 may be inserted into an implantation site using implant delivery instrument 850, 950, or any of the other implantation delivery instruments disclosed herein. For example, augment 2530 may be implanted using implant delivery instrument 850 as shown in FIGS. 36A-36D, with augment 2530 in place of augment 1430.

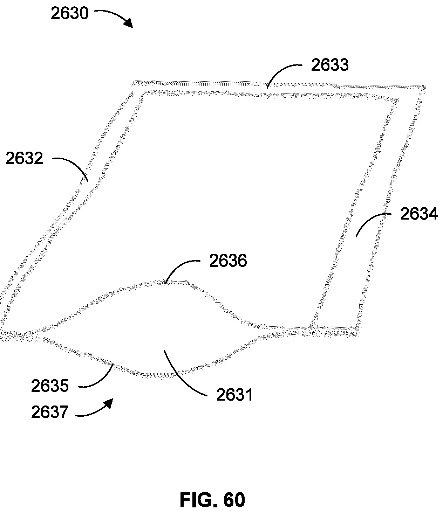

Referring now to FIG. 60, an augment 2630 is shown according to an example aspect. Augment 2630 may be substantially similar to augment 2430, except that a third edge of augment 2630 may be closed, with only one end providing an opening into a cavity. For example, as shown in FIG. 60, augment 2630 includes a cavity 2631 configured to receive a balloon (e.g., balloon 1440, balloon 2040, etc.). Cavity 2631 is accessible from an opening on only one end 2637 of augment 2630 between edges 2635 and 2636. A first edge 2632, a second edge 2633, and a third edge 2634 may all be closed. As shown in FIG. 60, edges 2632, 2633, 2634 are continuously closed, but as discussed above with respect to edges 2432, 2434 of augment 2430, any or all of edges 2632, 2633, 2634 may be non-continuously closed, with openings providing access to cavity 2631 (which may not be large enough for a balloon to pass therethrough). In some examples, open end 2637 may be completely open from edge 2632 to edge 2634, with edges 2635, 2636 not coupled at all along the length of one end 2637. In other aspects, edges 2635, 2636 may be partially coupled, for example, adjacent the edges 2632, 2634 such that the opening does not extend fully from edge 2632 to edge 2634. In some examples, edges 2635, 2636 may be coupled (e.g., adhered, stitched, etc.) together after a balloon has been positioned within cavity 2631. In other examples, augment 2630 may include a suture or filament coupled to openings or eyelets at edges 2635, 2636 and surrounding the opening in end 2637 that may act as a drawstring that may be tensioned to close the opening in end 2637 after a balloon has been inserted into cavity 2631.

Augment 2630 may be used in various ways in a surgical procedure as discussed above with respect to the other augments described herein and may be made with similar materials. For example, augment 2630 may be similar to augment 2430, and cavity 2631 may be similar to internal cavity 2431 of augment 2430. Thus, when augment 2630 is secured to tissue of a patient and a balloon (e.g., balloon 1440) is disposed in the internal cavity 2631 of augment 2630 and coupled to augment 2630, balloon 1440 may also be secured to the tissue via augment 2630. Similar to the aspect shown in FIG. 35A, after the augment 2630 and the balloon are attached to the tissue, the balloon may be inflated. The volume of the internal cavity 2631 and/or the flexibility of the augment 2630 may be sufficient to allow inflation of the balloon. Augment 2630 may be secured to tissue using any of the methods for coupling augments to tissue described herein. For example, fixation elements (e.g., u-shaped bars, staples, pins, screws, etc.) may be pressed through edges 2632, 2633, 2634 of augment 2630 and into tissue. Alternatively, filaments or sutures may be passed through edges 2632, 2633, 2634 and coupled to tissue or anchors previously coupled to the tissue. In other examples, edges 2632, 2633, 2634 may include openings or eyelets configured to receive a suture or filament for securing augment 2630 to tissue. In still other examples, a balloon in the cavity 2631 may be secured directly to tissue such that augment 2630 is secured to tissue via the balloon. Augment 2630 may be inserted into an implantation site using implant delivery instrument 850, 950, or any of the other implantation delivery instruments disclosed herein. For example, augment 2630 may implanted using implant delivery instrument 850 as shown in FIGS. 36A-36D, with augment 2630 in place of augment 1430.

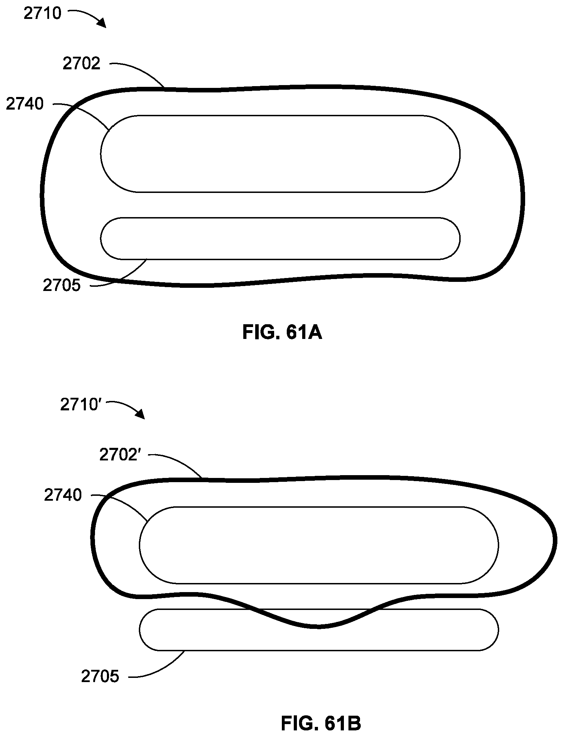

Referring now to FIG. 61A, an implant 2710 is shown according to an example aspect. Implant 2710 includes a balloon 2740 that is secured to tissue at an implantation site. In the aspect shown, the tissue is soft tissue of a rotator cuff 2705, but it will be appreciated that similar methods of securing implant 2710 may be applied in other implantation sites. Balloon 2740 is secured to the rotator cuff 2705 by at least one belt 2702 that wraps around rotator cuff 2705 and balloon 2740. Belts 2702 may be tensioned so as to hold balloon 2740 in a particular location on rotator cuff 2705 or may be only partially tensioned to allow some movement of balloon 2740, as discussed above with respect to FIGS. 20A-20B. Balloon 2740 may be the same as or similar to other balloons discussed herein and may be made of similar materials. Implant 2710 may further include an augment coupled to balloon 2740 as discussed herein with respect to other implants (e.g., implant 1810). For example, balloon 2740 may include tabs or fixation members similar to tabs 812A-D of balloon 840 with openings similar to openings 838A-D. Belt 2702 may be passed through these openings on either side of balloon 2740 so the balloon 2740 may be secured to rotator cuff 2705. In other aspects, belt 2702 may wrap around rotator cuff 2705 but may be secured to balloon 2740 (e.g., to openings in tabs at the edges of balloon 2740) without wrapping around balloon 2740. In some examples, belt 2702 may be a filament or suture or may be a wider, ribbon-like band. For example, belt 2702 may have a width equal to, narrower than, or wider than a width of balloon 2740. In some examples, implant 2710 may be secured using multiple belts 2702 along the length of balloon 2740.

FIG. 61B shows an implant 2710' according to an example aspect. Implant 2710' may be substantially similar to implant 2710 except that implant 2710' is secured to tissue (e.g., rotator cuff 2705) by at least one belt 2702' that pierces rotator cuff 2705. As shown in FIG. 61B, belt 2702' pierces the top of rotator cuff 2705 without passing all the way through rotator cuff 2705, passes through a partial width of rotator cuff 2705, and extends back out of the top surface of rotator cuff 2705. Belt 2702' then wraps around balloon 2740, in a manner substantially similar to belt 2702 as shown in FIG. 61A. Like the belt 2702, the belt 2702' may be coupled to balloon 2740 rather than wrapping around balloon 2740. Further, while FIG. 61B shows belt 2702' passing only partially through the thickness of rotator cuff 2705 and partially through the width of rotator cuff 2705, in other examples, belt 2702' may extend through the entire thickness of rotator cuff 2705, piercing the lower surface of rotator cuff 2705 and/or may pass through the entire width of rotator cuff 2705 (from left to right, as shown). Belt 2702' may be a suture or filament, or may be a wider, ribbon-like band as discussed above. As discussed above with respect to belt 2702, implant 2710' may be secured by multiple belts 2702' along the length of implant 2710'.

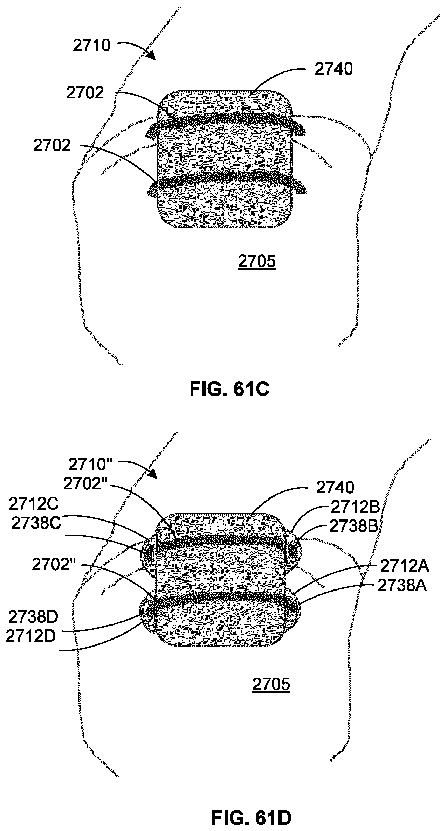

FIG. 61C is a top view of implant 2710 (e.g., as shown in FIG. 61A) secured to tissue by two belts 2702 that wrap around balloon 2740. As discussed with respect to FIG. 61A, the belts 2702 may wrap around tissue 2705, which, as discussed above may be soft tissue of a rotator cuff. Aside from wrapping around balloon 2740, the belts 2702 may not otherwise interface with implant 2710. For example, belts 2702 may not pass through openings, tabs, or other features of implant 2710. The belts 2702' may wrap around balloon 2740' (e.g., as shown in FIG. 61B) in a similar manner to secure implant 2710' to tissue 2705, though as discussed above, belts 2702' may pass through a portion of the tissue 2705 rather than wrapping around tissue 2705. Thus, the top view of implant 2710' secured to tissue 2705 may appear the same as the top view shown in FIG. 61C. In some examples, the belts 2702' may crisscross across balloons 2740' rather than extending parallel across balloon 2740'

FIG. 61D is a top view of an implant 2710" secured to tissue 2705 by two belts 2702". Implant 2710" may be substantially similar to implants 2710, 2710', except that implant includes tabs 2712A-D each with a respective opening 2738A-D extending therethrough. A first belt 2702" extends through opening 2738B, across the top of balloon 2740", and through opening 2738C. A second belt 2702" extends through opening 2738A, across the top of balloon 2740", and through opening 2738D. Belts 2702" may secure implant 2710" to tissue 2705 in a manner similar to or the same as belts 2702 as shown in FIG. 61A or a manner similar to or the same as belts 2702' as shown in FIG. 61B. Specifically, belts 2702" may wrap around soft tissue 2705 in the joint (e.g., soft tissue of a rotator cuff), as shown in FIG. 61A, or may extend through at least a portion of the soft tissue 2705 in the joint, as shown in FIG. 61B. In some examples, the belts 2702" may crisscross across balloons 2740" rather than extending parallel across balloon 2740'

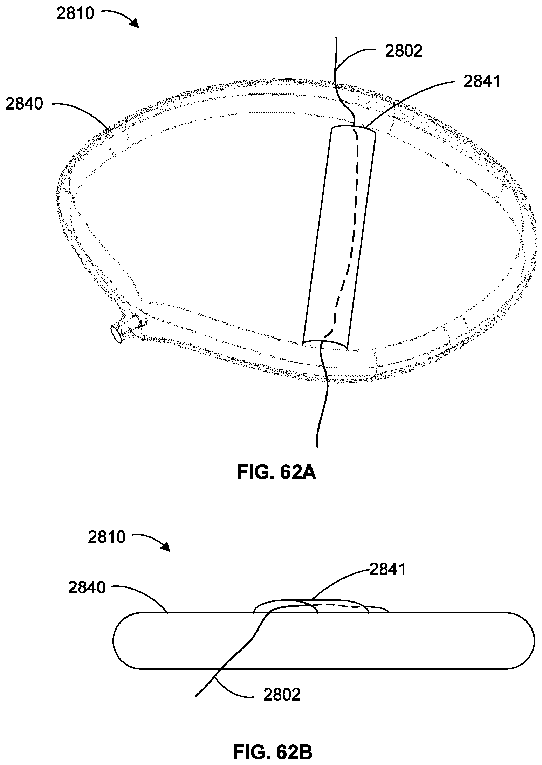

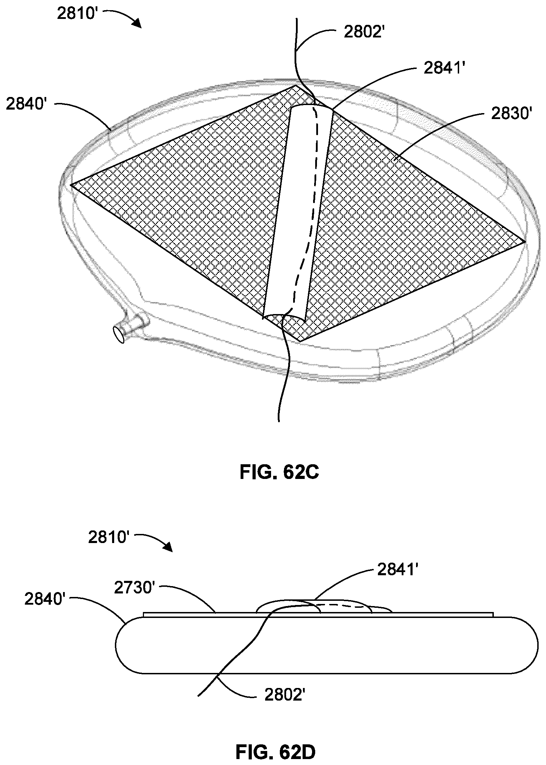

FIGS. 62A-62B illustrate an implant 2810 according to an example aspect. Implant 2810 includes a balloon 2840 that includes a channel 2841 extending along a surface of the balloon 2840 (and forming a peripheral portion of the implant 2810) through which a filament 2802 (e.g., a suture) may be passed. The ends of filament 2802 may be secured to an anchor coupled to tissue at an implantation site or may be directly coupled to the tissue, as discussed above. Balloon 2840 may include multiple channels 2841. For example, balloon 2840 may include two parallel channels 2841, and a filament 2802 may be passed through each channel 2841. The implantation site may include four anchors, e.g., anchors 803A'-D', as shown in FIGS. 21-24. A first filament 2802 may be coupled to anchor 803A', pass through a first channel 2841, and be secured to anchor 803B', and a second filament 2802 may be coupled to anchor 803C', pass through a second channel 2841 (not shown), and be secured to anchor 803D'. In another example, one channel 2841 may cross over another channel 2841 (not shown), such that two filaments 2802 passing through the channels 2841 may crisscross as shown in FIGS. 25A-25D. Channels 2841 may thus be an alternative to the tabs 812A-D and openings 838A-D for coupling an implant to tissue at an implantation site. As discussed below with respect to FIGS. 62C-62D, in some examples, channel 2841 may be formed in an augment instead of in a channel 2841. Thus, rather than using fixation elements (e.g., U-shaped bars, staples, pins) to pierce through the material of an augment (e.g., as shown in FIG. 33) or stitching filaments through the material of an augment, a filament channel 2841 may be passed through a channel in an augment in a manner similar to passing a filament 2802 through a channel 2841 as discussed above. While channel 2841 is shown crossing a top surface of balloon 2840 in FIGS. 62A-62B, in some examples, channel 2841 may cross a bottom surface of balloon 2840, such that channel 2841 is positioned between balloon 2840 and tissue to which implant 2810 is secured.

FIGS. 62C-62D illustrate an implant 2810' according to an example aspect. Implant 2810' may be substantially similar to implant 2810 as shown in FIGS 62A-62B, except that implant 2810' includes an augment with a channel (e.g., similar to channel 2841) rather than balloon with a channel. Implant 2810' includes a balloon 2840' with an augment 2830' coupled thereto (e.g., using adhesive or mechanical coupling methods as discussed above). Augment 2830' includes a channel 2841', similar to channel 2841, extending along a surface of the augment 2830' (and forming a peripheral portion of the implant 2810) through which a filament 2802' (e.g., a suture) may be passed. The ends of filament 2802' may be secured to an anchor coupled to tissue at an implantation site or may be directly coupled to the tissue, as discussed above. Augment 2830' may include multiple channels 2841'. For example, augment 2830' may include two parallel channels 2841, and a filament 2802 may be passed through each channel 2841. The implantation site may include four anchors, e.g., anchors 803A'-D', as shown in FIGS. 21-24. A first filament 2802' may be coupled to anchor 803A', pass through a first channel 2841, and be secured to anchor 803B', and a second filament 2802 may be coupled to anchor 803C', pass through a second channel 2841' (not shown), and be secured to anchor 803D'. In another example, one channel 2841' may cross over another channel 2841' (not shown), such that two filaments 2802' passing through the channels 2841' may crisscross as shown in FIGS. 25A-25D. Channels 2841' may thus be an alternative to the tabs 812A-D and openings 838A-D for coupling an implant to tissue at an implantation site. While augment 2830' is shown on a top surface of 2840' in FIGS. 62C-62D, in some examples, augment 2830' may be positioned below balloon 2840', such that channel 2841' is positioned between augment 2830' and tissue to which implant 2810' is secured.

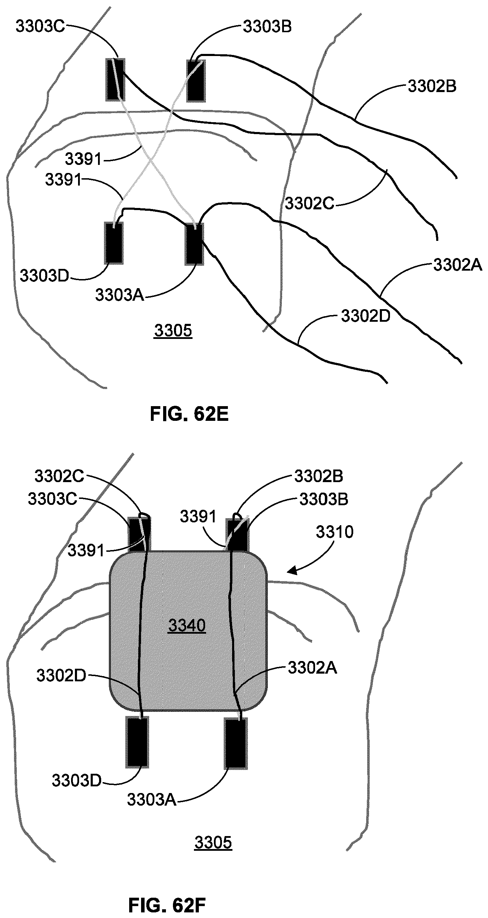

FIGS. 62E-62D illustrate a method of coupling and securing an implant 3310 to tissue 3305 at an implantation site. FIG. 62E illustrates the implantation site prior to delivery of implant 3310. Anchors 3303A-D are installed in tissue 3305. A first filament 3391 extends from anchor 3303A to anchor 2203C, and a second filament 3391 extends from anchor 3303B to anchor 3303D. Filaments 3391 may be sutures used to perform, for example, a double row repair of a rotator cuff. Four filaments 3302A-D each extend respectively from anchors 3303A-D. In some examples, any of filaments 3302A-D may be additional lengths of filaments 3391 or may be separate filaments. Implant 3310 may be advanced into the patient to the implantation site. For example, implant 3310 may be advanced into the patient to the implantation site in a rolled or folded configuration in sheath 872 of implant delivery instrument 850 (or in a sheath of another one of the implantation tools disclosed herein), and sheath 872 may be retracted at the implantation site to expose implant 3310, which may unroll or unfold into the flat, expanded configuration shown.

Filament 3302A may be drawn over balloon 3340 of implant 3310 and coupled to filament 3302B, for example, by tying a knot or using a knotless fixation feature such as a finger trap, which may be coupled to either of filaments 3302A-B. Filament 3302D may be drawn over balloon 3340 of implant 3310 and coupled to filament 3302C, for example, by tying a knot or using a knotless fixation feature such as a finger trap, which may be coupled to either of filaments 3302C-D. In other examples, the pairs of filaments may not be coupled together, and two filaments may each be respectively directly coupled to two anchors 3303A-D. For example, filament 3302A may directly couple anchor 3303A to anchor 3303B, and filament 3302D may directly couple anchor 3303D to anchor 3303D, while filaments 3302B and 3302C may not be used. In such examples, shuttles may be used to pull filaments through eyelets of the anchors, as discussed above. In other examples, filaments may crisscross over balloon 3340 rather than being arranged in parallel as shown in FIG. 62F. For example, filament 3302A may couple anchor 3303A to anchor 3303C, and filament 3302D may couple anchor 3303D to anchor 3303B. Notably, aside from extending over balloon 3340, the filaments 3302A-D may not otherwise interface with implant 3310. For example, filaments 3302A-D may not pass through openings, tabs, or other features of implant 3310, and may instead form a cage that secures implant 3310 using downward pressure on balloon 3340. In some examples, additional anchors and filaments may be used to form the cage. For example, in some examples, an additional filament may cross over balloon 3340 perpendicular to the filaments 3302A, 3302D shown in FIG. 62F to resist the movement of 3310 left or right (as shown).

As discussed in the various examples above, one or more filaments coupled to tissue in a patient (e.g., coupled to anchors or directly coupled to tissue) are slidably coupled to a balloon implant while the balloon implant is outside the patient, the balloon implant is advanced along the one or more filaments into the patient, and the implant is secured to the tissue, for example using the one or more filaments or using one or more other filaments or fixation elements. Each of the one or more filaments may be coupled to the tissue by one of a plurality of anchors, which may have been coupled together during a previously completed joint repair by the one or more filaments or by one or more other filaments. The balloon implant may be secured to the tissue at the location of the previously completed joint repair. Slidably coupling the one or more filaments to the balloon implant may include passing a first end of a first filament of the one or more filaments through a first opening in the balloon implant. The first opening may extend through a peripheral portion of the balloon implant, such as a tab, a channel, or a loop member formed at the end of a strap of the balloon implant. In some examples, the first end of the first filament may be passed through one or more additional openings in the balloon implant. In some examples, slidably coupling the one or more filaments to the balloon implant includes passing a first end of each of the one or more filaments directly through material of one or more tabs forming peripheral portions of the balloon implant. In some examples, a filament may be coupled to a first anchor coupled to the tissue and is slidably coupled to the balloon implant in a first two locations, and securing the balloon implant to the tissue includes securing the first filament to a second anchor coupled to the tissue. In some examples, a second filament of the one or more filaments is coupled to a third anchor coupled to the tissue and is slidably coupled to the balloon implant in a second two locations, and securing the balloon implant to the tissue includes securing the second filament to a fourth anchor coupled to the tissue.

The balloon implant may be advanced into the patient while in a collapsed configuration using an implant delivery instrument and transitions from the collapsed configuration to an expanded configuration in the patient. For example, the implant delivery instrument may include a sheath, and the balloon implant may be positioned in the sheath when the balloon implant is advanced into the patient. The sheath may be retracted to release the balloon implant from the sheath inside the patient, releasing the balloon implant causing the balloon implant to transition from the collapsed configuration to the expanded configuration. A balloon of the balloon implant may be inflated inside the patient using the implant delivery instrument (e.g., by providing fluid to the balloon via a conduit extending through a shaft of the instrument). The filaments may be tensioned while the balloon implant is advanced and when the implant is in position over the implantation site, prior to securing the balloon implant to the tissue

Securing the balloon implant to the tissue may include at least one of tying one of the one or more filaments to the balloon implant (e.g., tying a knot around a tab of the implant), tying one of the one or more filaments to an anchor coupled to the tissue (e.g., passing a filament through a tab of the implant and tying the filament to an anchor), tying a first filament of the one or more filaments to a second filament of the one or more filaments (e.g., tying a first filament coupled to a first anchor to a second filament coupled to a second anchor with one of the filaments passing through a tab of the implant), tying the first filament to another filament coupled to an anchor, where the other filament is not used to guide the implant to the implantation site, tying a first end of the first filament to a second end of the first filament, the first filament passing through the anchor, tying a first end of filament not used to guide the implant to the implantation site to at least one of an anchor, the balloon implant, a second end of the filament or to another filament, or pressing a fixation element through a portion of the implant and into tissue at the implantation site.

In other examples, knotless techniques may be used to secure the implant. In one example, a first filament, after being used to guide the implant to the implantation site may be secured to a second filament using a knotless fixation feature (e.g., a finger trap). For example, the first filament may be coupled to a first anchor, pass through the implant in one or more locations, and be coupled to a second filament coupled to a second anchor, the second filament including a finger trap used to secure the first filament. In another example, the first filament may be to an anchor coupled to the tissue using a knotless fixation feature. For example, the anchor may be a suture anchor with a self-locking feature that captures the filament without the use of a knot. In another example, a first portion of the first filament to a second portion of the first filament using a knotless fixation feature after passing the first filament passing through one or more anchors and the implant. In some examples, a filament not used to guide to the implant may be used to secure to the implant using one of the knotless fixation methods discussed above.

To the extent not explicitly described above, it should be appreciated that any one of implants 810, 810', 1810, 1910, 2010 may be modified to have a total of one or more attachment points for the attachment of suture or other fixation anchors. In some examples, an attachment point may be an opening defined through part of the implant. In these examples, locations of the attachment points may be on any side of the implant and on one or more sides. Depending on the arrangement of the specific implant, an opening may be through a tissue augment or a fixation member of a balloon. Further, any one of the aspects contemplated by the present disclosure may utilize one-way tensioning mechanisms and/or shuttles to secure filament and an accompanying implant. And, any one of the contemplated one-way tensioning mechanisms and/or shuttles may be used in any one of the contemplated aspects.

The foregoing method descriptions and the process flow diagrams are provided merely as illustrative examples and are not intended to require or imply that the steps of the various embodiments must be performed in the order presented. The steps in the foregoing embodiments may be performed in any order unless otherwise specified. Words such as "then," "next," etc., are not necessarily intended to limit the order of the steps; these words may be simply used to guide the reader through the description of the methods. Although process flow diagrams may describe the operations as a sequential process, many of the operations can be performed in parallel or concurrently. In addition, the order of the operations may be re-arranged. It should be appreciated that to the extent it is not explicitly mentioned for one or more of the contemplated method aspects for placement of an implant onto an internal tissue of a patient, some examples of the method may include providing or otherwise assembling an implant delivery instrument with a filament loaded therein prior to advancement of the implant delivery instrument into the patient. In such methods, the filament may be loaded into the implant delivery instrument prior to attachment of the filament to an internal tissue of a patient, e.g., via an anchor.

Further, it should be appreciated that any of the implants described above may be placed over an existing repair. For instance, an implant may be used where sutures and anchors were previously used to repair a torn ligament (e.g. a rotator cuff). It should also be appreciated that any of the implants described above may be placed on top of an augment which has been secured to the tissue.

It should be noted that any of the instruments, implants and methods disclosed herein may be used in conjunction with robotic technology. For example, any of the instruments described herein may be used with robotic surgical systems to place an implant, such as a balloon implant, in a patient. An instrument may be manipulated with a robotic system or a robotic arm to control placement and deployment, and/or to secure the implant within a patient during a surgical procedure. In other examples, a robotic surgical system may be used to place anchors into tissue of a patient, where such anchors are configured to receive filament attachable to an implant. Further, any or all of the steps described in the methods for performing an implantation procedure of the present disclosure may be performed using a robotic system.

Although the disclosure herein has been described with reference to particular aspects, it is to be understood that these aspects are merely illustrative of the principles and applications of the present disclosure. For example, various implants, balloons, augments, etc. discussed herein may be secured to tissue using a variety of methods and structures, including sutures coupled to suture anchors, tacks, staples, or a combination thereof. The various implants, balloons, augments, etc. discussed herein may include any number of securement locations, including tabs, apertures, flaps, excess material, protrusions, augment material legs, that may be in various shapes, sizes and locations. It is therefore to be understood that numerous modifications may be made to the illustrative aspects and that other arrangements may be devised without departing from the spirit and scope of the present disclosure as defined by the appended claims.

## Claims

1. An implant system comprising:
an implant comprising:
a balloon including a body and an inlet being in fluid communication with an internal cavity of the body;
a fitting attached to the inlet of the balloon and configured to receive a fluid for inflating the body of the balloon; and
a connection feature configured to receive a filament therethrough; and
an implant delivery instrument configured to receive the implant in a collapsed configuration.

2. The implant system of claim 1, wherein the implant delivery instrument comprises a sheath configured to receive the implant in a deflated and collapsed configuration and a shaft configured to be coupled to the fitting, the shaft comprising a conduit configured to provide the fluid for inflating the body of the balloon.

3. The implant system of claim 2, wherein the sheath is retractable relative to the conduit to release the implant from the sheath.

4. The implant system of claim 3, wherein the sheath comprises a slot configured to allow the filament coupled to the connection feature to extend out of a side of the sheath.

5. The implant system of claim 4, wherein the slot extends to a distal end of the sheath.

6. The implant system of claim 4 or 5, wherein the slot includes a widened portion configured to be positioned adjacent to the connection feature when the conduit interfaces with the fitting.

7. The implant system of any of claims 1-6, further comprising one or more filaments slidably coupled to the implant.

8. The implant system of claim 7, wherein a filament of the one or more filaments is coupled to the connection feature.

9. The implant system of any of claims 1-8, wherein the connection feature comprises an opening extending through a peripheral portion of the implant.

10. The implant system of any of claims 1-8, further comprising a strap coupled to the body of the balloon, wherein the connection feature comprises a structure defining an opening coupled to the strap, the opening configured to receive the filament.

11. The implant system of any of claims 1-8, wherein the connection feature comprises a tab extending peripherally from the body of the balloon.

12. The implant system of any of claims 1-8, wherein the connection feature comprises an opening extending through a portion of the fitting.

13. The implant system of any of claims 1-12, as far as dependent from claim 7, further comprising an anchor, wherein one of the one or more filaments is secured to the anchor.

14. The implant system of any of claims 1-13, as far as dependent from claim 7, wherein a first filament of the one or more filaments is secured to a second filament of the one or more filaments using a knotless fixation feature.

15. The implant system of any of claims 1-13, as far as dependent from claim 7, wherein a first end of a first filament of the one or more filaments extends through a first anchor coupled to the tissue and is secured to a second end of the first filament using a knotless fixation feature.
